# EUROPEAN PATENT APPLICATION

(11) **EP 2 409 703 A1**
(43) Date of publication of application: **25.01.2012**
(21) Application number: 11075209.4
(22) Date of filing: 01.08.2008
(51) Int. Cl.: A61K 31/4545, A61P 25/00, A61P 25/02, A61P 25/14, A61P 25/16, A61P 25/18, A61P 25/22, A61P 25/24, A61P 25/28, A61P 29/00, A61P 35/00, A61P 43/00, A61K 45/06, C07D 453/02

(54) **Treatment with alpha7-selective ligands**

(30) Priority: 02.08.2007 US 953610 P; 02.08.2007 US 953614 P; 02.08.2007 US 953613 P; 12.09.2007 US 971654 P
(62) Divisional of application: 08782589.9
(71) Applicant: Targacept, Inc., Winston-Salem, NC 27101 (US); Medical College Of Georgia Research Institute, Inc, Augusta, GA 30912 (US)
(72) Inventor: Bencherif, Merouane, Winston-Salem, North Carolina 27106 (US); Marrero, Mario B., Evans, Georgia 30809 (US)
(74) Representative: Crowhurst, Charlotte Waveney

(57) **Abstract**

The present invention relates to selective α7 nAChR agonist compounds for use in treating or preventing metabolic syndrome.

## Description

### Cross-Reference to Related Applications

The present application claims benefit to U.S. Provisional Application Serial Nos. 60/971,654, filed September 12, 2007; 60/953,610, filed August 2, 2007; 60/953,613, filed August 2, 2007; and 60/953,614 filed August 2, 2007, each of which is incorporated herein by reference in its entirety.

### Field of the Invention

The present invention includes methods, uses, and selective α7 nAChR agonist compounds for treating or preventing metabolic disorders.

### Background

Many patients who have insulin resistance or Type 2 diabetes mellitus (T2DM) often have several symptoms that together are referred to as syndrome X, or metabolic syndrome. Metabolic syndrome is a combination of medical disorders that increase the risk for cardiovascular disease and diabetes. Metabolic syndrome affects as much as 25% of the US population and is known by various other names such as (metabolic) syndrome X, insulin resistance syndrome, or Reaven's syndrome. A patient diagnosed with metabolic syndrome typical exhibits three or more symptoms selected from the following group of five symptoms: (1) abdominal obesity; (2) hypertriglyceridemia; (3) low high-density lipoprotein cholesterol (low HDL); (4) high blood pressure; and (5) elevated fasting glucose, which may be in the range characteristic of Type 2 diabetes. Each of these symptoms is defined in the recently released Third Report of the National Cholesterol Education Program Expert Panel on Detection, Evaluation and Treatment of High Blood Cholesterol in Adults (Adult Treatment Panel III, or ATP III), National Institutes of Health, 2001, NIH Publication No. 01-3670, herein incorporated by reference with regard to the definition of metabolic syndrome and its symptoms. Symptoms and features include diabetes mellitus type 2, insulin resistance, high blood pressure, fat deposits mainly around the waist, decreased HDL, elevated triglycerides, and elevated uric acid levels. Primary clinical problems are obesity and the high incidence of diabetes, a condition secondary to the insulin resistant state caused by excess adiposity. Insulin resistance in skeletal muscle, liver and adipose tissue impedes glucose disposal and results in the release of free fatty acids and the characteristic triglyceride dyslipidemia associated with the metabolic syndrome. Elevations in post-prandial and ultimately fasting glucose levels result in compensatory hyperinsulinemia, a condition which causes β-cell hypertrophy and eventual failure of the Islets and frank type 2 diabetes. Different quantitative inclusion criteria for metabolic syndrome have been proposed by the National Diabetes Federation, the World Health Organization, the European Group for the Study of Insulin Resistance (1999) and the National Cholesterol Education Program Adult Treatment Panel III (2001). Patients with metabolic syndrome, whether or not they have or develop overt diabetes mellitus, have an increased risk of developing the macrovascular and microvascular complications that occur with type 2 diabetics, such as atherosclerosis and coronary heart disease.

In addition to the hyperglycemia experienced with diabetes mellitus and metabolic syndrome, certain drug therapies can cause similar symptomatic effects. Statins, otherwise referred to as 3-hydroxy-3-methylglutaryl-coenzyme A (HMG-CoA) reductase inhibitors, are potent inhibitors of cholesterol synthesis that are extensively used in the treatment of hypercholesterolemia. Several studies have demonstrated the beneficial effects of statins in reducing cardiovascular morbidity and mortality. There is recent evidence however, from clinical and preclinical studies, that treatment with statins has unwanted effects, including increased glycemia. See, for example, Ohmura et al., Acute Onset and Worsening of Diabetes Concurrent with Administration of Statins, Endocrine Journal, 52: 369-372, 2005; Sasaki et al., Statins: Beneficial or Adverse For Glucose metabolism (review), Journal of Atherosclerosis and Thrombosis 13: 123-129, 2006; Takano et al., Influence of Statins on Glucose tolerance in Patients with Type II Diabetes mellitus, Journal of Atherosclerosis and Thrombosis 13: 95-100, 2006; and Nakata et al., Effects of Statins On the Adipocyte Maturation and Expression of Glucose Transporter 4; Implications in Glycemic Control, Diabetologia 49: 1881-1892, 2006.

In addition to hyperglycemia, there is evidence from epidemiological studies that long-term treatment with statins has unwanted effects like increasing the risk for Alzheimer Disease. Nicotine has been found to inhibit death of PC12 cells cultured in serum-free medium. Furthermore, the selective α7 receptor agonist, 3-(4)-dimethylaminocinnamylidine anabaseine (DMAC), and the nAChR (including the α7 subtype) activator, ABT-418, have also been reported to exert cytoprotective effects. It was also recently shown that nicotine activates the growth promoting enzyme janus kinase 2 (JAK2) in PC12 cells, and that preincubation of these cells with the JAK2 specific inhibitor AG-490 blocks the nicotine-induced activation of neuroprotective signaling cascades.

The following references are incorporated by reference with regard to a background understanding of relevant disease, as well as management and prevention: Banes AK, Shaw S, Jenkins J, Redd H, Amiri F, Pollock DM and Marrero MB, Angiotensin II blockade prevents hyperglycemia-induced activation of JAK and STAT proteins in diabetic rat kidney glomerul, Am J Physiol Renal Physiol 286: F653-F659, 2004; Bartholomeusz C, Itamochi H, Yuan LX, Esteva FJ, Wood CG, Terakawa N, Hung MC and Ueno NT, Bcl-2 antisense oligonucleotide overcomes resistance to E1A gene therapy in a low HER2-expressing ovarian cancer xenograft model, Cancer Res 65: 8406-8413, 2005; Cho-Chung YS, Park YG and Lee YN, Oligonucleotides as transcription factor decoys, Curr Opin Mol Ther 1: 386-392, 1999; Danesh FR, Sadeghi MM, Amro N, Philips C, Zeng L, Lin S, Sahai A and Kanwar YS. 3-Hydroxy-3-methylglutaryl CoA reductase inhibitors prevent high glucose-induced proliferation of mesangial cells via modulation of Rho GTPase/ p21 signaling pathway: Implications for diabetic nephropathy, Proc Natl Acad Sci U S A 99: 8301-8305, 2002; de Fiebre CM, Meyer EM, Henry JC, Muraskin SI, Kem WR and Papke RL. Characterization of a series of anabaseine-derived compounds reveals that the 3-(4)-dimethylaminocinnamylidine derivative is a selective agonist at neuronal nicotinic α7/125l-α-bungarotoxin receptor subtypes, Mol. Pharmacol. 47: 164-171, 1995; Deigner HP, Haberkom U and Kinscherf R, Apoptosis modulators in the therapy of neurodegenerative diseases, Expert Opin. lnvestig. Drugs 9: 747-764, 2000; Donnelly-Roberts DL, Xue IC, Arneric SP and Sullivan JP, In vitro neuroprotective properties of the novel cholinergic channel activator (ChCA), ABT-418, Brain Res 719: 36-44, 1996; Epstein M and Campese VM, Pleiotropic effects of 3-hydroxy-3-methylglutaryl coenzyme a reductase inhibitors on renal function; Am J Kidney Dis 45: 2-14, 2005; Garcia-Roman N, Alvarez AM, Toro MJ, Montes A and Lorenzo MJ, Lovastatin Induces Apoptosis of Spontaneously Immortalized Rat Brain Neuroblasts: Involvement of Nonsterol Isoprenoid Biosynthesis Inhibition, Molecular and Cellular Neuroscience 17: 329-341, 2001; Kastelein JJ, The future of lipid-lowering therapy: the big picture, Neth J Med 61: 35-39, 2003; Kirito K, Watanabe T, Sawada K, Endo H, Ozawa K and Komatsu N, Thrombopoietin regulates Bcl-xL gene expression through Stat5 and phosphatidylinositol 3-kinase activation pathways, J Biol Chem 277: 8329-8337, 2002; Li P, Nijhawan D, Budihardjo I, Srinivasula SM, Ahmad M, Alnemri ES and Wang X, Cytochrome c and dATP-dependent formation of Apaf-1/caspase-9 complex initiates an apoptotic protease cascade, Cell 91: 479-489, 1997; Li Y, Higashi Y, Itabe H, Song YH, Du J and Delafontaine P, Insulin-Like Growth Factor-1 Receptor Activation Inhibits Oxidized LDL-Induced Cytochrome C Release and Apoptosis via the Phosphatidylinositol 3 Kinase/Akt Signaling Pathway, Arterioscler Thromb Vasc Biol 23: 2178-2184, 2003; Mata P, Alonso R and Badimon J, Benefits and risks of simvastatin in patients with familial hypercholesterolaemia, Drug Saf 26: 769-786, 2003; McKay DM, Botelho F, Ceponis PJ and Richards CD, Superantigen immune stimulation activates epithelial STAT-1 and PI 3-K: PI 3-K regulation of permeability, Am J Physiol Gastrointest Liver Physiol 279: G1094-G1103, 2000; Meske V, Albert F, Richter D, Schwarze J and Ohm TG, Blockade of HMG-CoA reductase activity causes changes in microtubule-stabilizing protein tau via suppression of geranylgeranylpyrophosphate formation: implications for Alzheimer's disease, European Journal of Neuroscience 17: 93-102, 2003; Newman MB, Arendash GW, Shytle RD, Bickford PC, Tighe T and Sanberg PR, Nicotine's oxidative and antioxidant properties in CNS, Life Sciences 71: 2807-2820, 2002; Marrero MB, Papke RL, Bhatti BS, Shaw S, Bencherif M. The neuroprotective effect of 2-(3-pyridyl)-1-azabicyclo[3.2.2]nonane (TC-1698), a novel alpha 7 ligand, is prevented through angiotensin II activation of a tyrosine phosphatase. J. Pharmacol Exp Ther. 2004 Apr;309(1):16-27; Yamashita H, Nakamura S. Nicotine rescues PC12 cells from death induced by nerve growth factor deprivation. Neurosci. Lett. 1996 Aug 2;213(2):145-7; Isomaa, B. A major health hazard: the metabolic syndrome. Life Sci. 73, 2395-2411 (2003); Sykiotis,G.P. & Papavassiliou, A.G. Serine phosphorylation of Insulin Receptor Substrate-1: A novel target for the reversal of insulin resistance. Mol. Endocrinol. 15, 1864-1869 (2001); Dandona, P., Aljada, A. & Bandyopadhyay, A. Inflammation: the link between insulin resistance, obesity and diabetes, Trends Immunol., 25, 4-7 (2004); Miao, F.J.P., Green, P., Benowitz, N. & Levine, J. D., Vagal modulation of spinal nicotine-induced inhibition of the inflammatory response mediated by descending antinociceptive controls, Neuropharmacology, 45, 605-611 (2003); Borovikova, L.V. et al., Role of vagus nerve signaling in CNI-1493-mediated suppression of acute inflammation, Autonomic Neurosci.: Basic and Clinical, 85, 141-147 (2000); Borovikova, L.V. et al., Vagus nerve stimulation attenuates the systemic inflammatory response to endotoxin, Nature, 405, 458-462 (2000); Wang, H. et al., Nicotinic acetylcholine receptor α7 subunit is an essential regulator of inflammation, Nature, 421, 384-387 (2003); de Jonge, W.J. & Ulloa, L., The alpha 7 nicotinic acetylcholine receptor as a pharmacological target for inflammation, British J. Pharmacol., 151, 915-929 (2007); Fornari, A. et al., Nicotine withdrawal increases body weight, neuropeptide Y and Agouti-related protein expression in the hypothalamus and decreases uncoupling protein-3 expression in the brown adipose tissue in high-fat fed mice, Neurosci. Lett., 411, 72-76 (2007); Asante-Appiah, E. & Kennedy, B.P., Protein tyrosine phosphatases: the quest for negative regulators of insulin action, Am. J. Physiol. Endocrinol. Metabol. 284, E663-E670 (2003); Elchebly, M. et al., Increased insulin sensitivity and obesity resistance in mice lacking the protein tyrosine phosphatase-1B gene. Science, 283, 1544-1548 (1999); Dube, N. & Tremblay, M.L., Beyond the metabolic function of PTP1B, Cell Cycle, 3, 550-553 (2004); Uysal, K.T., Wiesbrock, S.M., Marino, M.W. & Hotamisligil, G.S., Profection from obesity-induced insulin resistance in mice lacking TNF-α function, Nature, 389, 610-614 (1997); Gallowitsch-Puerta, M. & Tracey, K.J., Immunologic role of the cholinergic anti-inflammatory pathway and the nicotinic acetylcholine alpha 7 receptor, Ann. N.Y. Acad. Sci. 1062, 209-19 (2005); Shaw, S., Bencherif, M. & Marrero, M.B., Janus kinase 2, an early target of α7 nicotinic acetylcholine receptor-mediated neuroprotection against Aβ-(1-42) amyloid, J. Biol. Chem., 277, 44920-44924 (2002); de Jonge, W.J. et al., Stimulation of the vagus nerve attenuates macrophage activation by activating the Jak2-STAT3 signaling pathway, Nat. Immunol., 6, 844-51 (2005); Ahima, R.S., Qi, Y. & Singhal, N.S., Adipokines that link obesity and diabetes to the hypothalamus, Prog. Brain Res., 153, 155-174 (2006); Kenner, K.A., Ezenta, A., Olefsky, J.M. & Kusari, J., Protein-tyrosine phosphatase 1B is a negative regulator of Insulin- and Insulin-like Growth Factor-I-stimulated signaling, J. Biol. Chem. 271, 19810-19816 (1996); Klaman, L.D. et al., Increased energy expenditure, decreased adiposity, and tissue-specific insulin sensitivity in Protein-tyrosine Phosphatase 1 B-deficient mice, Mol. Cell. Biol., 20, 5479-5489 (2000); Dadke, S., Kusari, J. & and Chernoff, J., Down-regulation of insulin signaling by Protein-tyrosine Phosphatase 1B is mediated by an N-terminal binding region, J. Biol. Chem., 275, 23642-23647 (2000); Cheng, A. et al., Attenuation of leptin action and regulation of obesity by Protein- tyrosine Phosphatase 1B, Dev.Cell, 2, 497-503 (2002); Myers, M.P. et al. TYK2 and JAK2 are substrates of Protein-tyrosine Phosphatase 1B, J. Biol. Chem., 276, 47771-47774 (2001); and Bence, K.K. et al., Neuronal PTP1B regulates body weight, adiposity and leptin action, Nat. Med., 12, 917-24 (2006).

### Summary of the Invention

One aspect of the present invention includes a method for treating or preventing metabolic disorders comprising the administration of a selective α7 nAChR agonist.

Another aspect of the present invention includes a method for treating or preventing drug-induced central nervous system disorders comprising the administration of a selective α7 nAChR agonist.

In one embodiment, the α7 nAChR agonist is Compound A, Compound B, or Compound C, or a pharmaceutically acceptable salt thereof. In one embodiment, the α7 nAChR agonist is Compound C or a pharmaceutically acceptable salt thereof.

In one embodiment, the metabolic disorder is one or more of type I diabetes mellitus, type II diabetes mellitus, metabolic syndrome, atherosclerosis, obesity, and hyperglycemia. In a further embodiment, the hyperglycemia is a result of statin therapy.

In one embodiment, the drug-induced central nervous system disorder is a result of statin therapy.

One aspect of the present invention is a method for treating or preventing a metabolic disorder comprising the administration of 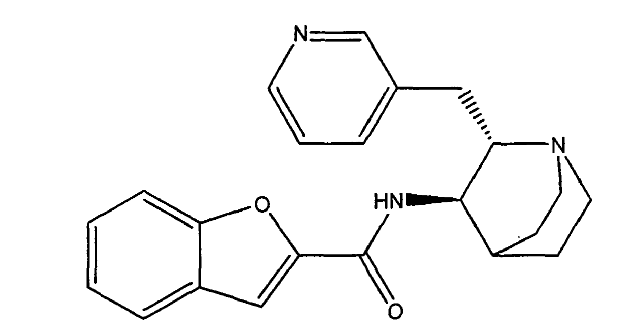 or a pharmaceutically acceptable salt thereof. In one embodiment, the metabolic disorder is one or more of type I diabetes mellitus, type II diabetes mellitus, metabolic syndrome, atherosclerosis, obesity, and hyperglycemia. In one embodiment, a daily dose is from about 0.001 mg/kg to about 3.0 mg/kg.

One aspect of the present invention is use of a selective α7 nAChR agonist in the manufacture of a medicament for treating or preventing metabolic disorders.

Another aspect is use of a selective α7 nAChR agonist in the manufacture of a medicament for treating or preventing drug-induced central nervous system disorders.

In one embodiment, the α7 nAChR agonist is Compound A, Compound B, or Compound C, or a pharmaceutically acceptable salt thereof. In one embodiment, the α7 nAChR agonist is Compound C or a pharmaceutically acceptable salt thereof.

In one embodiment, the metabolic disorder is one or more of type I diabetes mellitus, type II diabetes mellitus, metabolic syndrome, atherosclerosis, obesity, and hyperglycemia.

In one embodiment, the hyperglycemia is a result of statin therapy. In one embodiment, the drug-induced central nervous system disorder is a result of statin therapy.

Another aspect of the present invention is use of Compound C: 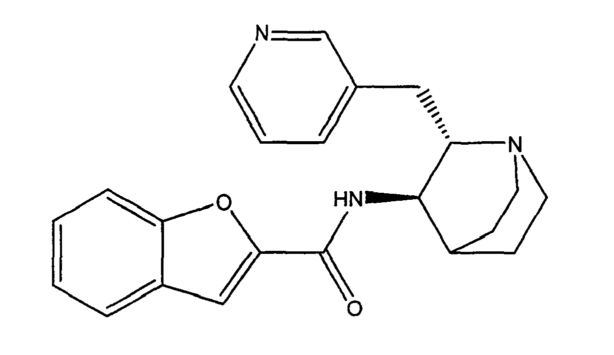 or a pharmaceutically acceptable salt thereof in the manufacture of a medicament for treating or preventing a metabolic disorder. In one embodiment, the metabolic disorder is one or more of type I diabetes mellitus, type II diabetes mellitus, metabolic syndrome, atherosclerosis, obesity, and hyperglycemia. In one embodiment, a daily dose is from about 0.001 mg/kg to about 3.0 mg/kg.

Another aspect of the present invention is a selective α7 nAChR agonist compound for use in treating or preventing metabolic disorders.

Another aspect of the present invention is a selective α7 nAChR agonist compound for use in treating or preventing drug-induced central nervous system disorders.

In one embodiment, the α7 nAChR agonist is Compound A, Compound B, or Compound C, or a pharmaceutically acceptable salt thereof. In one embodiment, the α7 nAChR agonist is Compound C or a pharmaceutically acceptable salt thereof.

In one embodiment, the metabolic disorder is one or more of type I diabetes mellitus, type II diabetes mellitus, metabolic syndrome, atherosclerosis, obesity, and hyperglycemia.

In one embodiment, the hyperglycemia is a result of statin therapy. In one embodiment, the drug-induced central nervous system disorder is a result of statin therapy.

Another aspect of the present invention is a selective α7 nAChR agonist compound 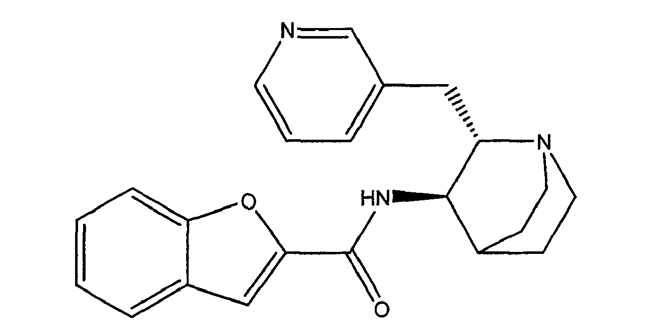 or a pharmaceutically acceptable salt thereof for use in treating or preventing a metabolic disorder. In one embodiment, the metabolic disorder is one or more of type I diabetes mellitus, type II diabetes mellitus, metabolic syndrome, atherosclerosis, obesity, and hyperglycemia. In one embodiment, a daily dose is from about 0.001 mg/kg to about 3.0 mg/kg.

The scope of the present invention includes combinations of aspects, embodiments, and preferences herein described.

### Brief Description of the Figures

The Figures describe results obtained according to particular embodiments of the invention and exemplify aspects of the invention but should not be construed to be limiting.

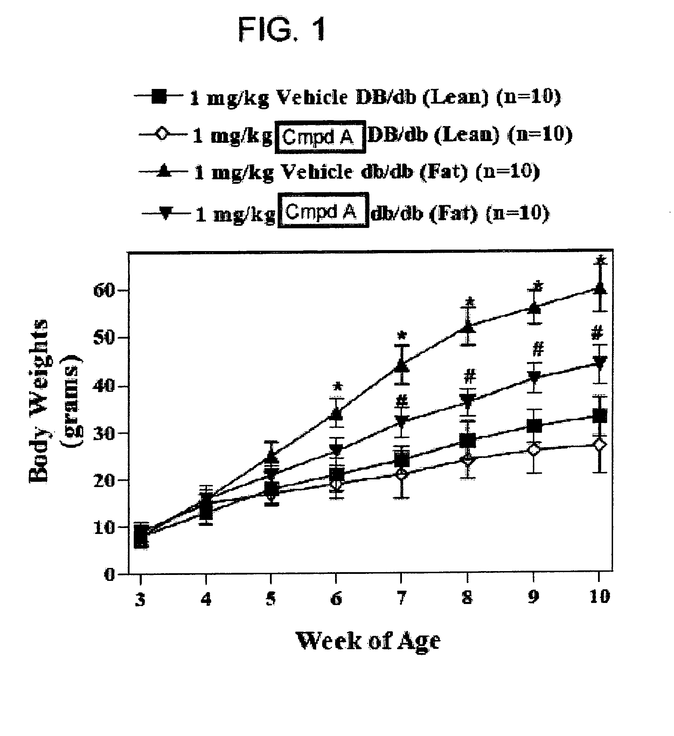

Figure 1 is a graphic representation showing the effects of Compound A on body weight in obese db/db mice.

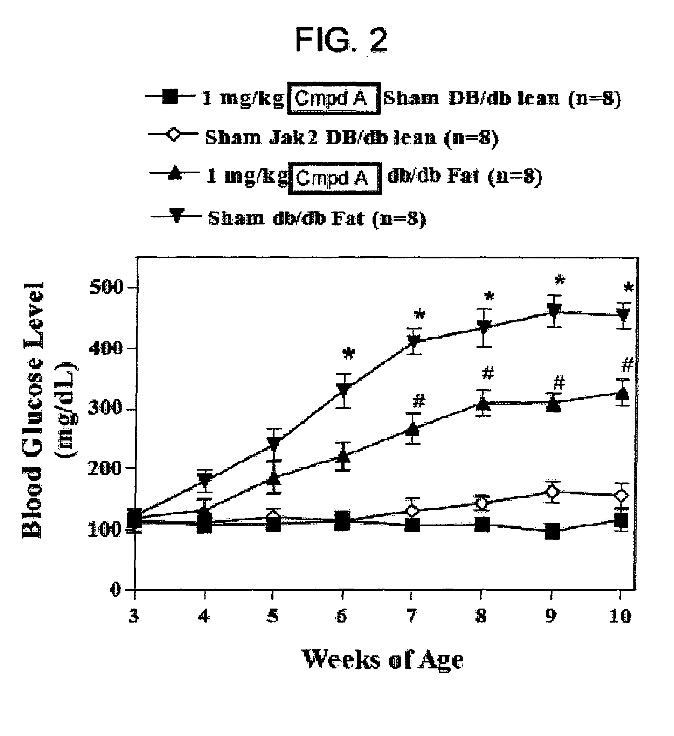

Figure 2 is a graphic representation showing the effects of Compound A on plasma glucose in obese db/db mice.

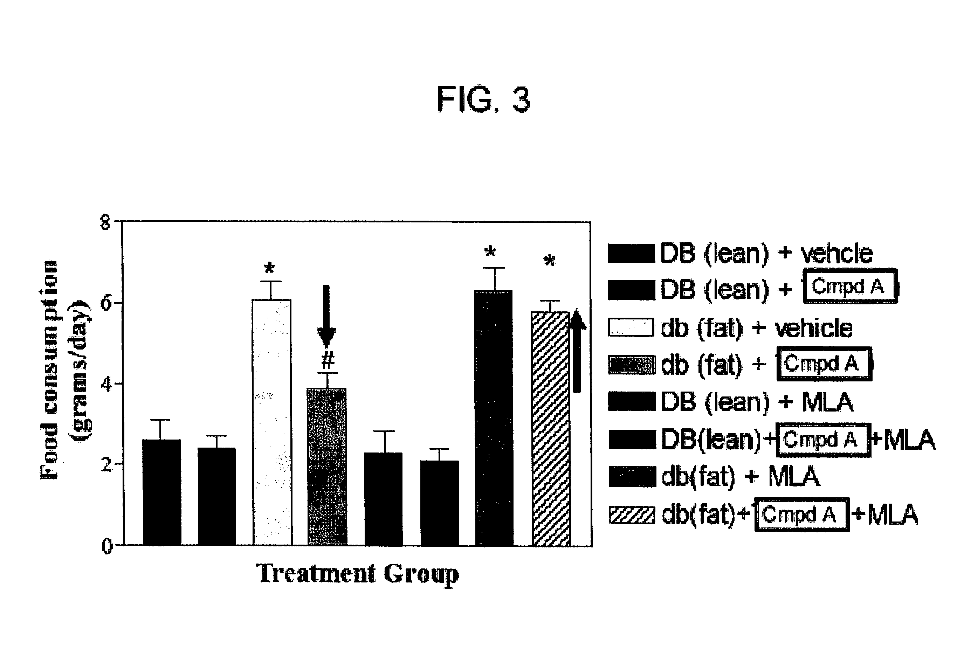

Figure 3 is a graphic representation showing the effects of Compound A on food consumption in obese db/db mice.

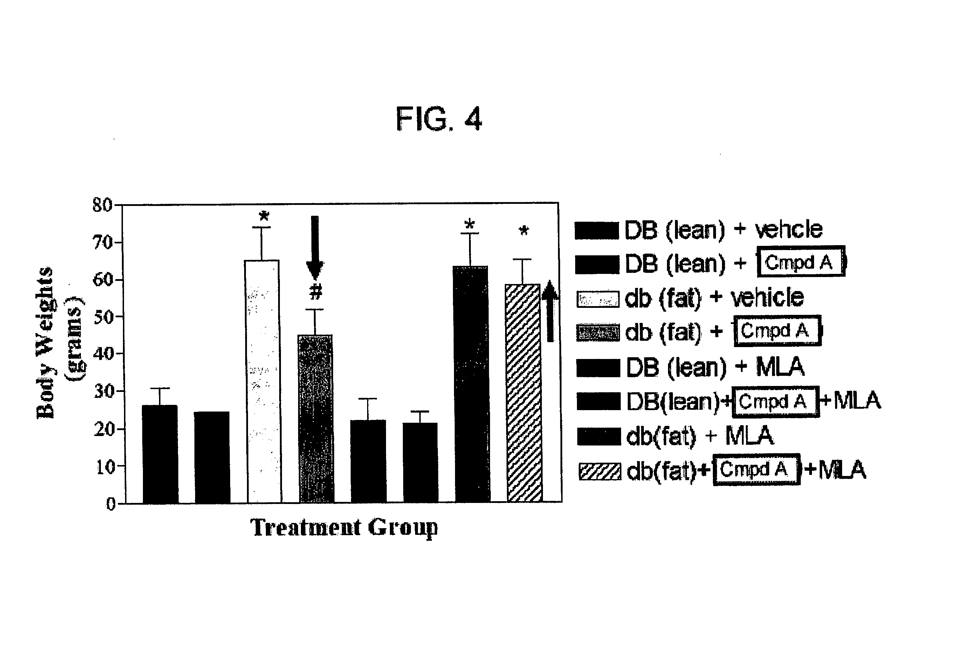

Figure 4 is a graphic representation showing the effects of Compound A on body weight in obese db/db mice.

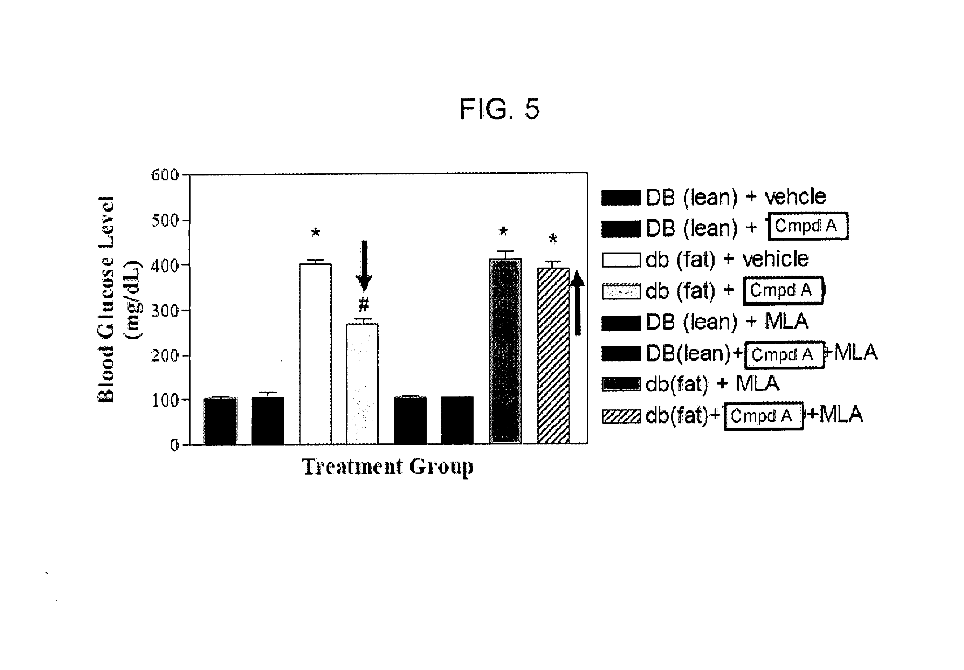

Figure 5 is a graphic representation showing the effects of Compound A on glucose levels in obese db/db mice.

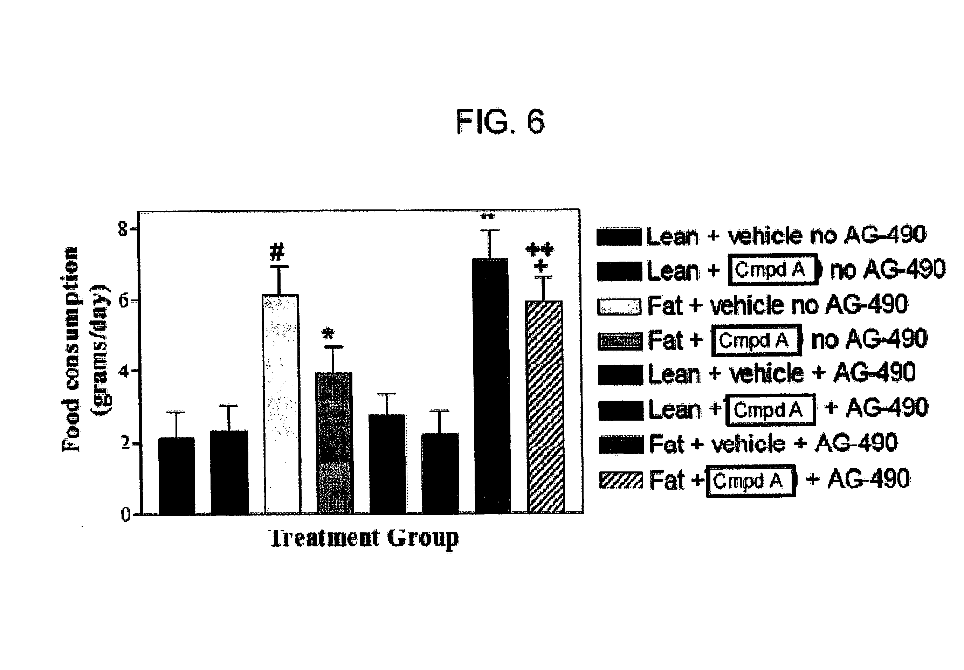

Figure 6 is a graphic representation showing the partial inhibition of the effects of Compound A on food consumption in obese db/db mice of the JAK2 tyrosine phosphorylation inhibitor AG-490. AG-490, a known inhibitor of JAK2 tyrosine phosphorylation, partially inhibits effects of Compound A.

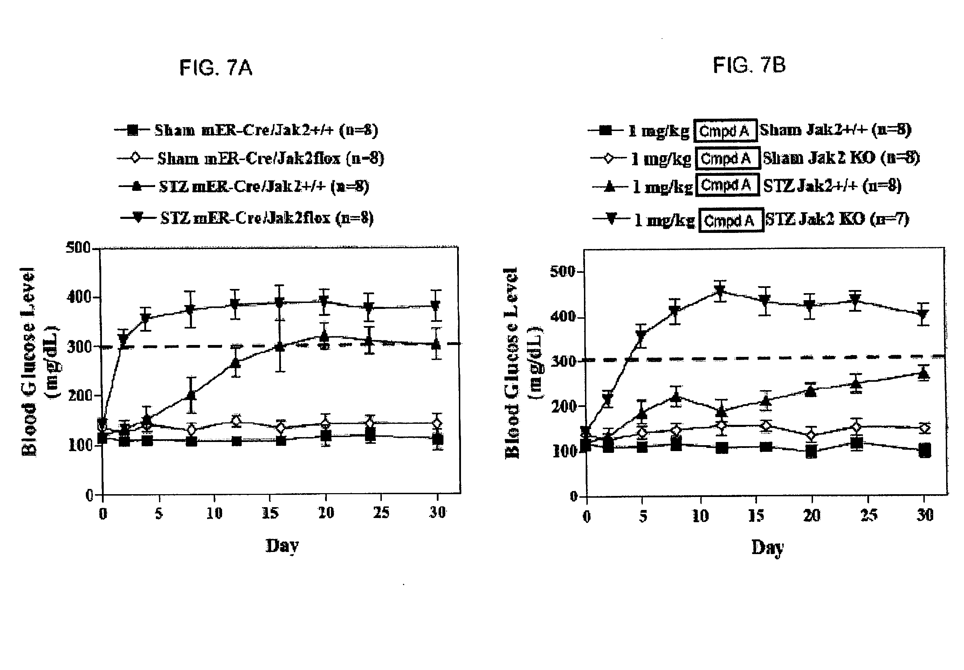

Figures 7A and 7B are graphic representations showing the effects of JAK2 loss-of-function on multiple low dose (MLDS) STZ-induced diabetes (Fasting Blood Glucose) in mice in the presence or absence of Compound A.

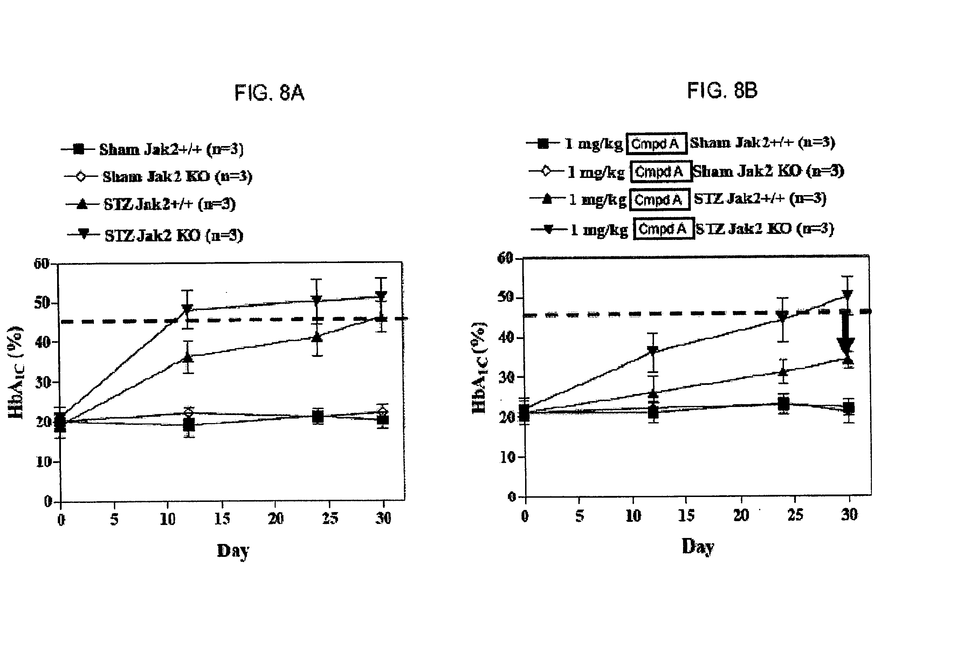

Figures 8A and 8B are graphic representations showing the effects of JAK2 loss-of-function on multiple low dose (MLDS) STZ-induced increase in HbA1c in mice in the presence or absence of Compound A.

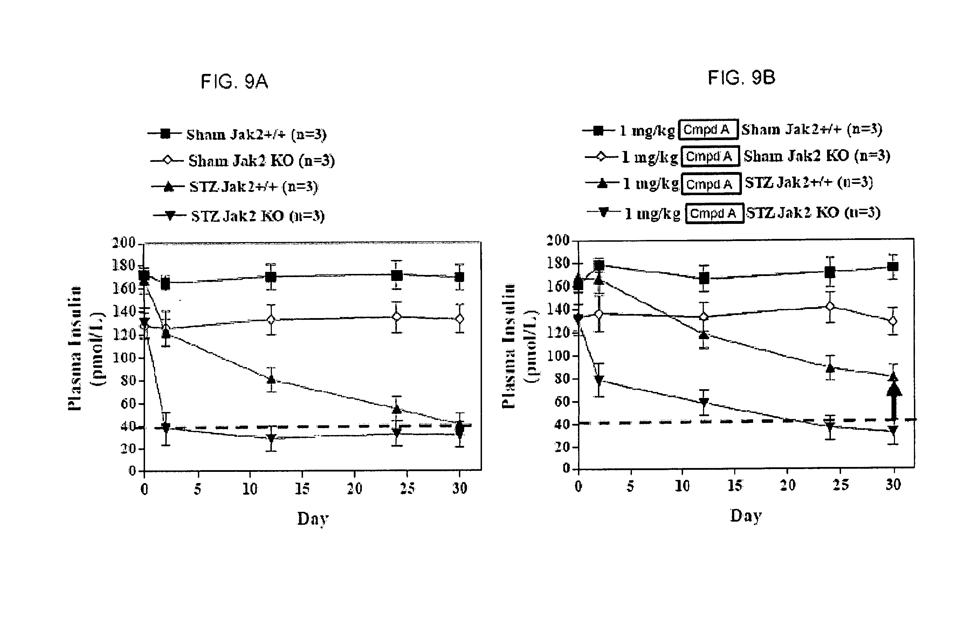

Figures 9A and 9B are graphic representations showing the effects of JAK2 loss-of-function on multiple low dose (MLDS) STZ-induced decrease in plasma insulin in mice in the presence or absence of Compound A.

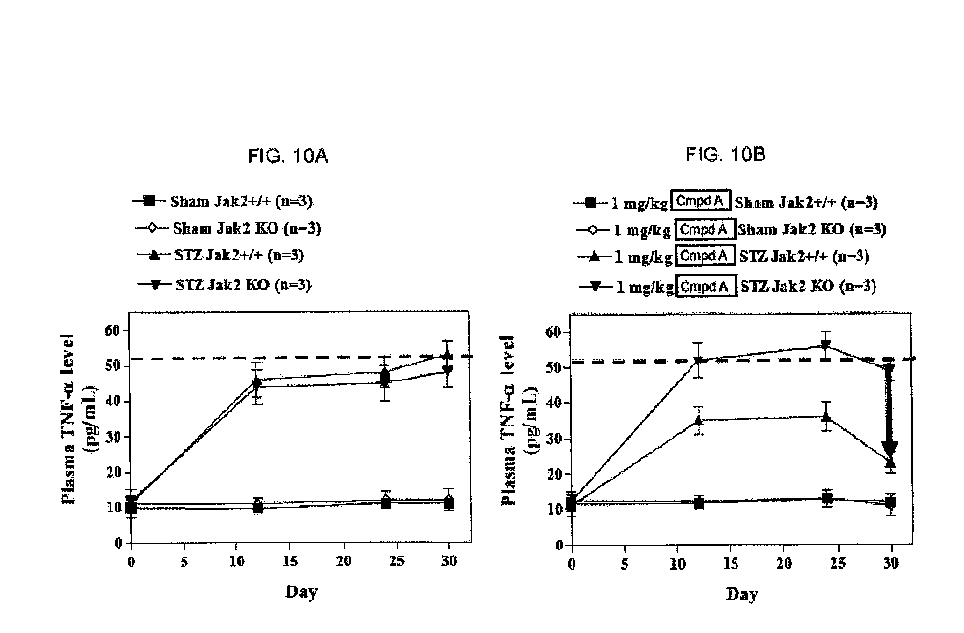

Figures 10A and 10B are graphic representations showing the effects of JAK2 loss-of-function on multiple low dose (MLDS) STZ-induced increase in plasma TNFα in mice in the presence or absence of Compound A.

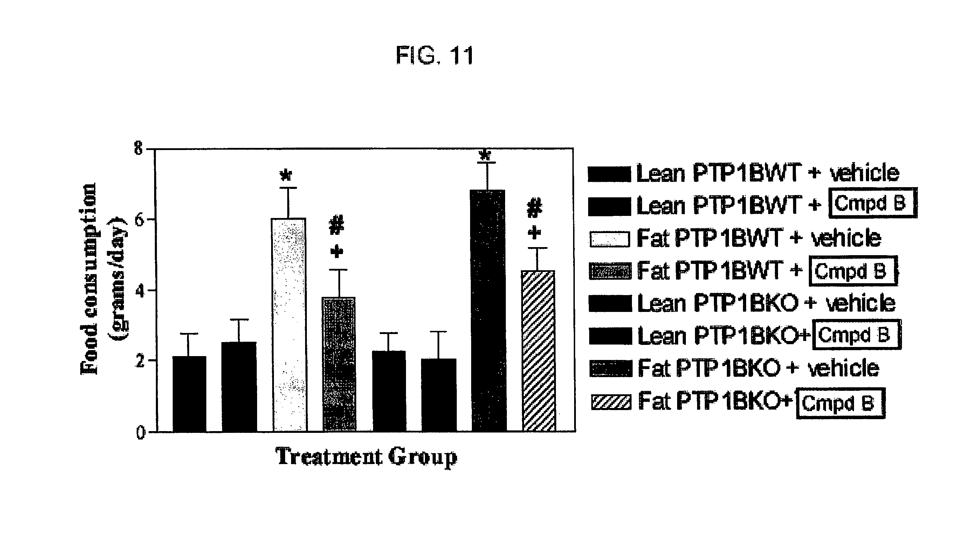

Figure 11 is a graphic representation showing the effects of Compound B on food consumption in db/db mice. Results represent the mean +/- SEM of eight treated mice and are expressed as food consumed in grams/day. Fat mice show a significant increase in food consumption (*P < 0.01) which was significantly inhibited by Compound B treatment (+P < 0.01).

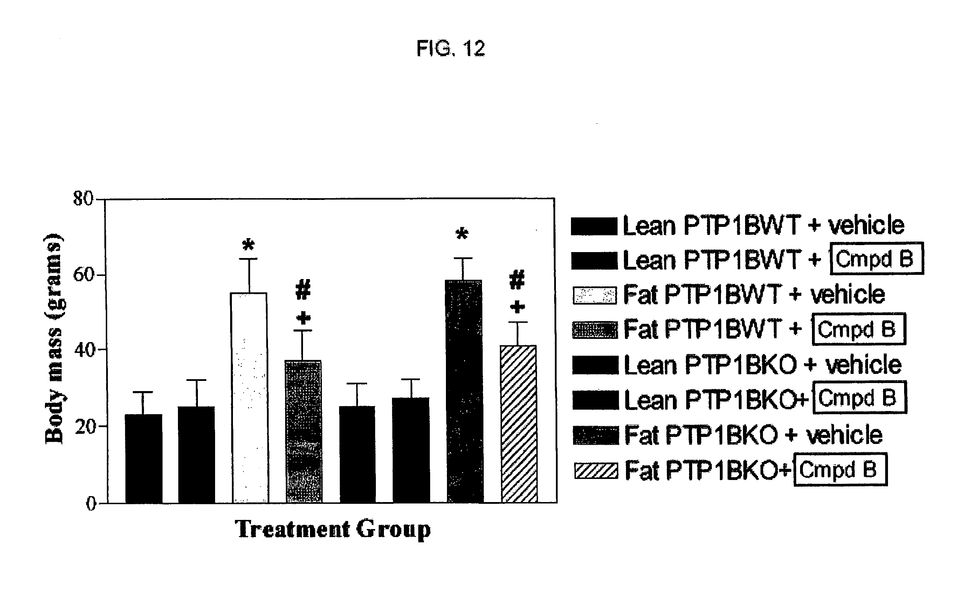

Figure 12 is a graphic representation showing the effects of Compound B on body mass in db/db mice. Results represent the mean +/- SEM of eight treated mice and are expressed as their body mass in grams. Fat mice show a significant increase in body mass (*P < 0.01) which was significantly inhibited by Compound B (+P < 0.01).

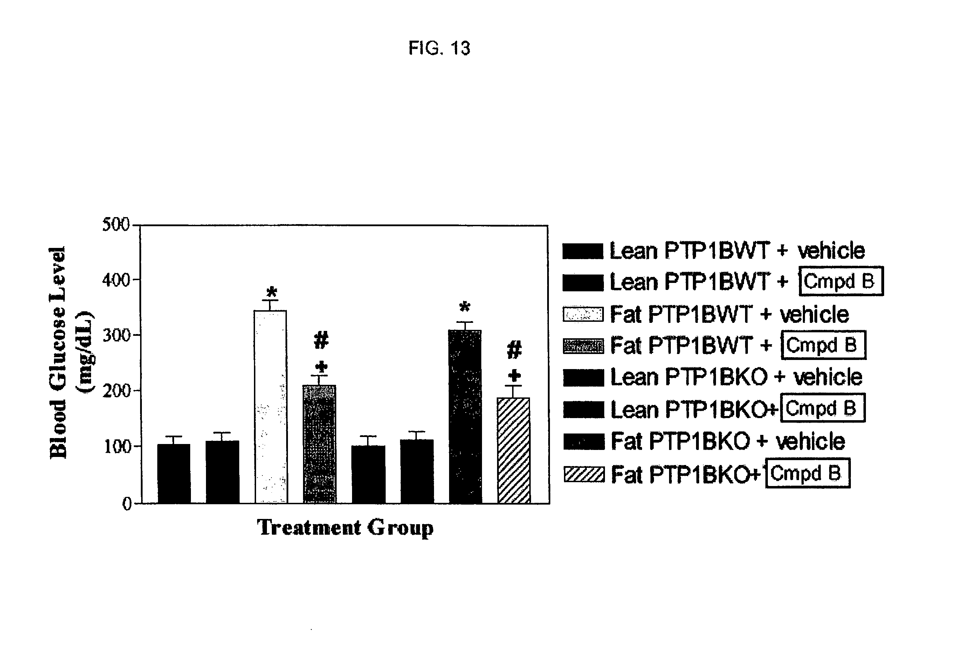

Figure 13 is a graphic representation showing the effects of Compound B on plasma blood glucose (BG) in db/db mice. Results represent the mean +/- SEM of eight treated mice and are expressed as mg/dL. Fat mice show a significant increase in BG (*P < 0.01) which was significantly inhibited by Compound B treatment (+P < 0.01).

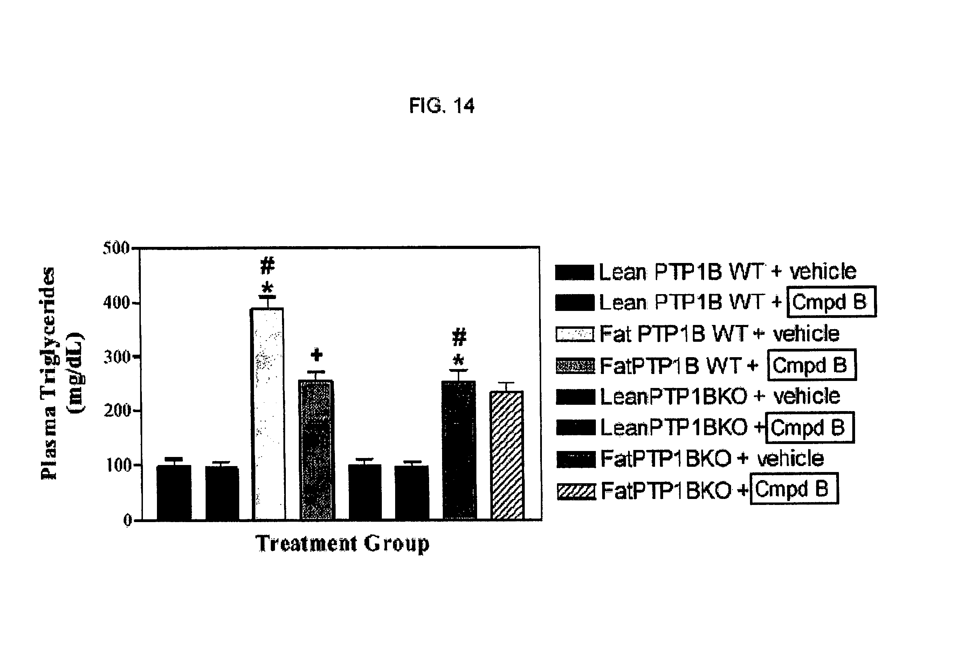

Figure 14 is a graphic representation showing the effects of Compound B on plasma triglycerides (TG) in db/db mice. Results represent the mean +/- SEM of eight treated mice and are expressed as mg/dL. Fat mice show a significant increase in plasma TG (*P < 0.01) which was significantly inhibited by Compound B treatment (+P < 0.01).

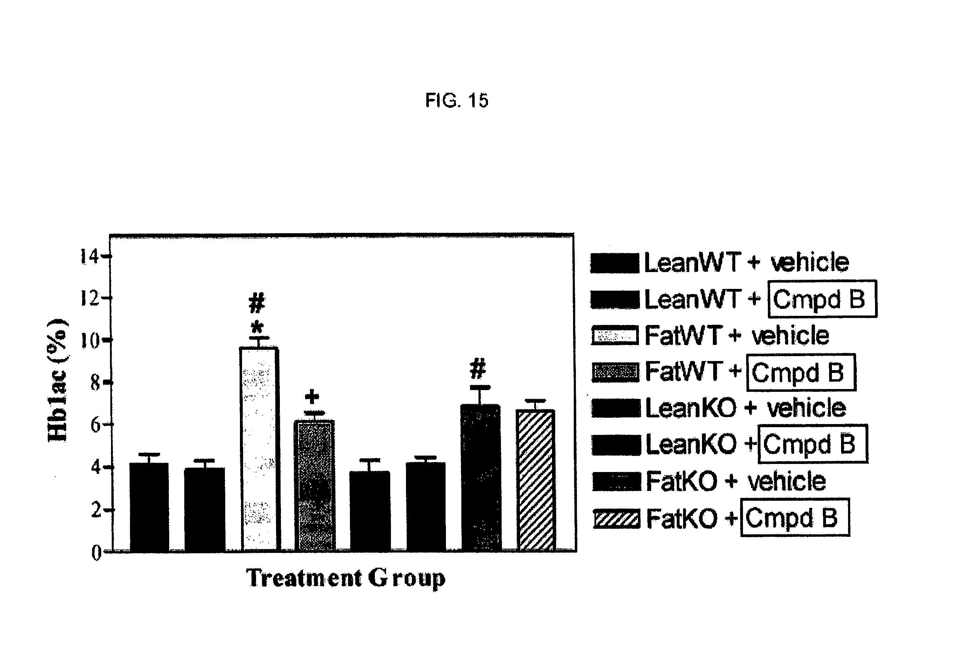

Figure 15 is a graphic representation showing the effects of Compound B on plasma glycosylated hemoglobin (Hb1ac) in db/db mice. Results represent the mean +/- SEM of five treated mice and are expressed as %. Fat mice show a significant increase in plasma HbA1c (*P < 0.01) which was significantly inhibited by Compound B treatment (+P < 0.01).

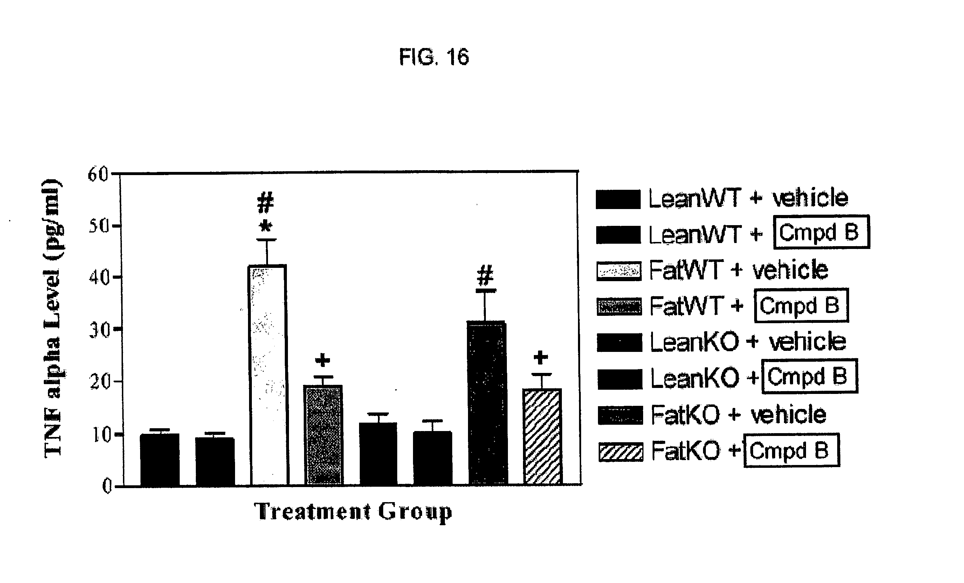

Figure 16 is a graphic representation showing the effects of Compound B on plasma TNFα in db/db mice. Results represent the mean +/- SEM of five treated mice and are expressed as pg/ml. Fat mice show a significant increase in TNFα (*P < 0.01) which was significantly inhibited by Compound B treatment (+P < 0.01).

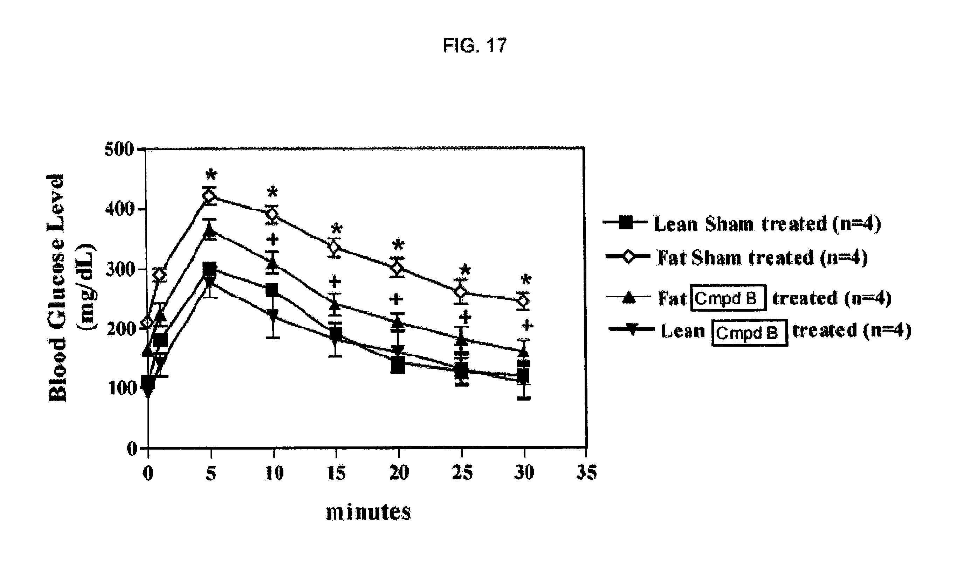

Figure 17 is a graphic representation showing the effects of Compound B on the Glucose Tolerance Test (GTT) in db/db mice PTP-1 B WT mice. Results represent the mean +/- SEM of four treated mice and are expressed as mg/dL. Fat mice show a significant increase in glucose levels(*P < 0.01) and a significant effect with Compound B treatment (+P < 0.01).

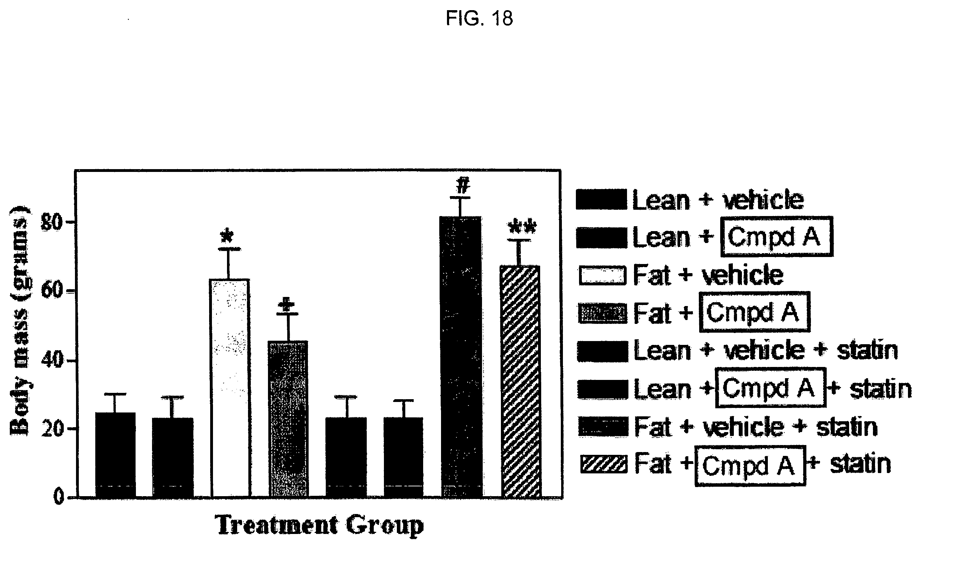

Figure 18 is a graphic representation of the effects of simvastatin, referred generally as "statin," and Compound A on body mass in db/db mice. Results represent the mean +/-SEM of eight treated mice and are expressed as their body mass in grams. Fat mice show a significant increase in body mass (*P < 0.01) which was significantly inhibited by Compound A (+P < 0.01). Simvastatin alone did not significantly inhibit the increase in body mass (#P > 0.05). However, the combination of simvastatin and Compound A had a significant effect in lowering the body mass compared to simvastatin alone (**P < 0.01).

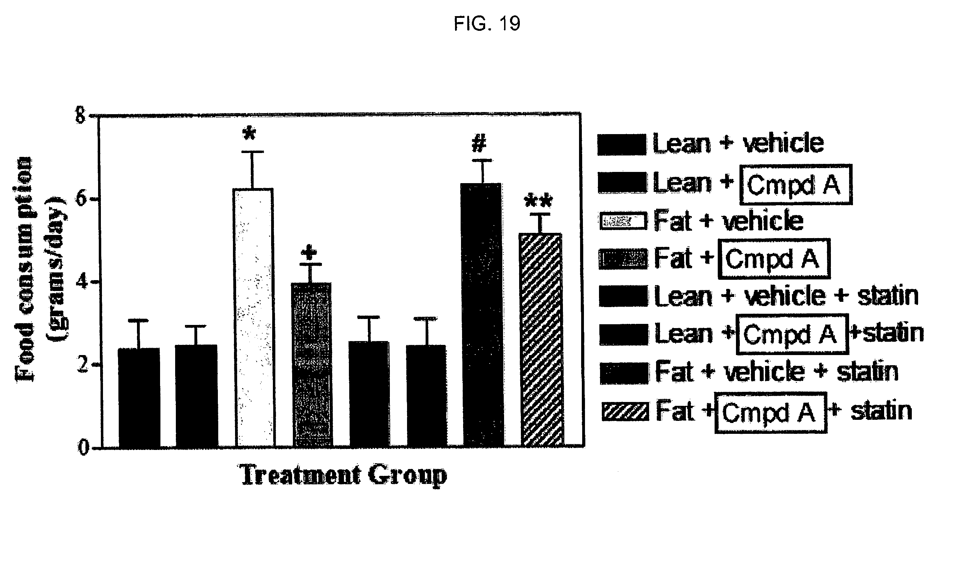

Figure 19 is a graphic representation of the effects of simvastatin, referred generally as "statin," and Compound A on food consumption in db/db mice. Results represent the mean +/- SEM of eight treated mice and are expressed as food consumed in grams/day. Fat mice show a significant increase in food consumption (*P < 0.01) which was significantly inhibited by Compound A (+P < 0.01). Simvastatin alone did not significantly inhibited the increased in food consumption (#P > 0.05). However, the combination of simvastatin and Compound A had a significant effect (**P < 0.01).

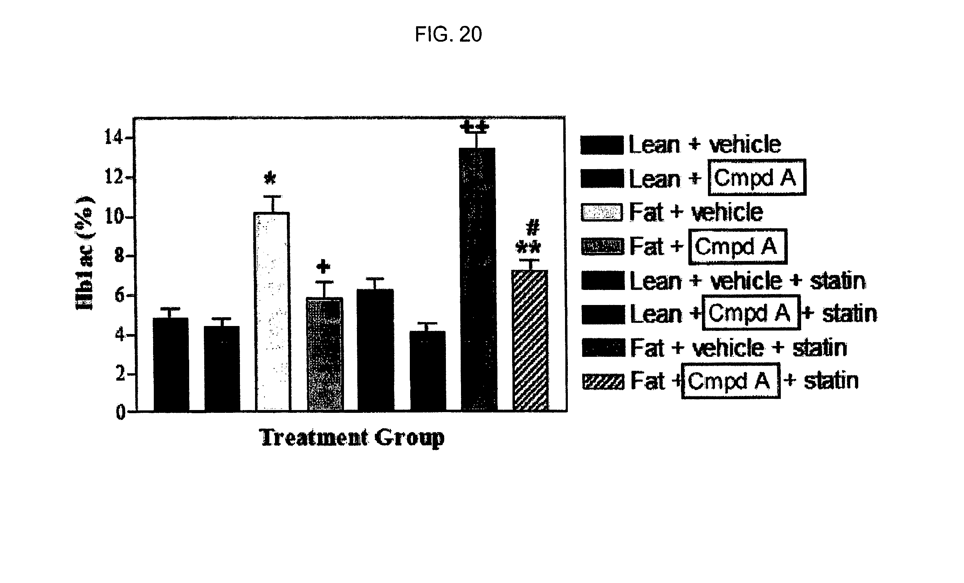

Figure 20 is a graphic representation of the effects of simvastatin, referred generally as "statin," and Compound A on Hb1ac in db/db mice. Results represent the mean +/- SEM of eight treated mice and are expressed as % glycated hemoglobin (%Hb1ac). Fat mice show a significant increase in %Hb1ac (*P < 0.01) which was significantly inhibited by Compound A (+P < 0.01). Simvastatin alone did not lower the levels %Hb1ac. On the other hand, it significantly increased the levels of %Hb1ac (++P < 0.01) above the fat mice treated with vehicle alone. The combination of simvastatin and Compound A significantly decreased the levels of %Hb1ac when compared to both the fat (**P < 0.01) and the fat plus simvastatin (#P < 0.01).

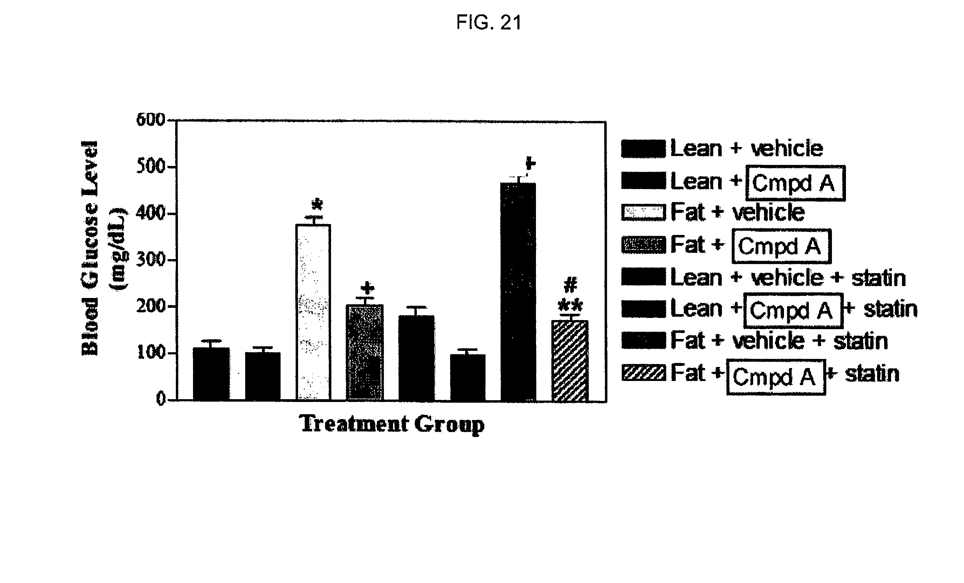

Figure 21 is a graphic representation of the effects of simvastatin, referred generally as "statin," and Compound A on plasma blood glucose (BG) in db/db mice. Results represent the mean +/- SEM of eight treated mice and are expressed as mg/dL. Fat mice show a significant increase in BG (*P < 0.01) which was significantly inhibited by Compound A (+P < 0.01). Simvastatin alone significantly increased the levels of BG (++P < 0.01) above the fat mice treated with vehicle alone. However, the combination of simvastatin and Compound A significantly decreased the levels of BG when compared to both the fat (**P < 0.01) and the fat plus simvastatin (#P < 0.01).

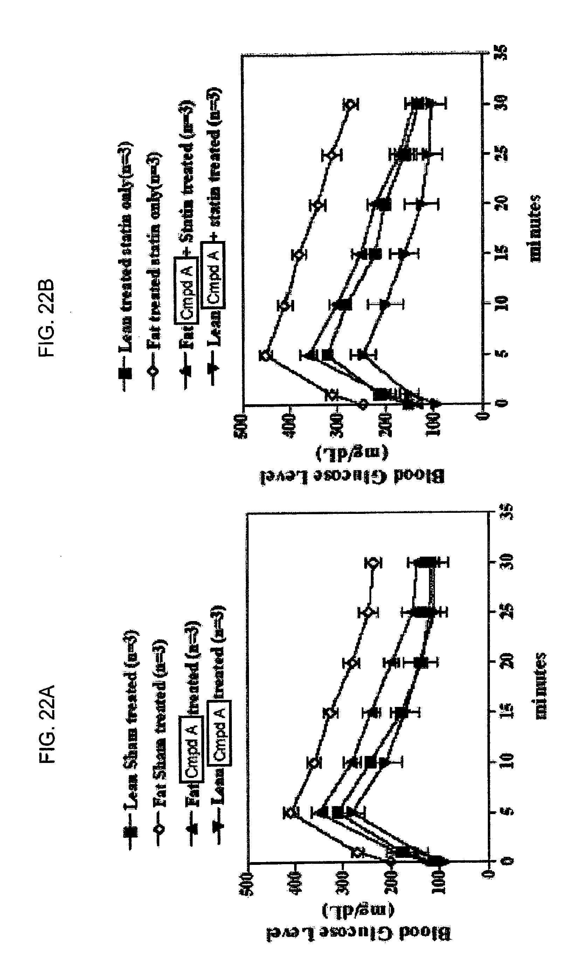

Figures 22A and 22B are graphic representations of the effects of simvastatin, referred generally as "statin," and Compound A on insulin resistance glucose tolerance test in db/db mice.

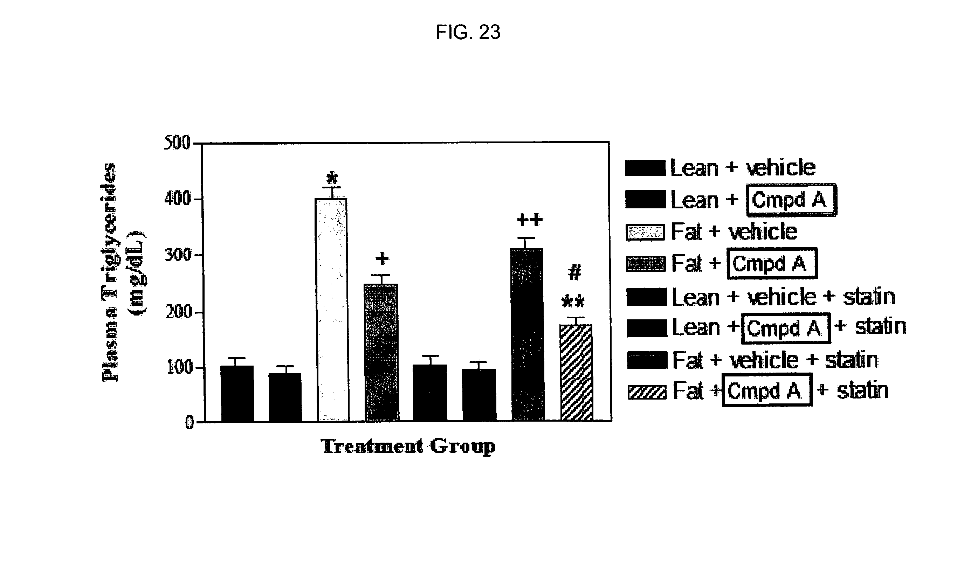

Figure 23 is a graphic representation of the effects of simvastatin, referred generally as "statin," and Compound A on plasma triglycerides (TG) in db/db mice. Results represent the mean +/- SEM of eight treated mice and are expressed as mg/dL. Fat mice show a significant increase in TG (*P < 0.01) which was significantly inhibited by Compound A (+P < 0.01). Simvastatin alone did not significantly decreased the levels of TG (++P > 0.01) above the fat mice treated with vehicle alone. However, the combination of simvastatin and Compound A significantly decreased the levels of TG when compared to both the fat and the fat (**P < 0.01) and the fat plus simvastatin (#P < 0.01).

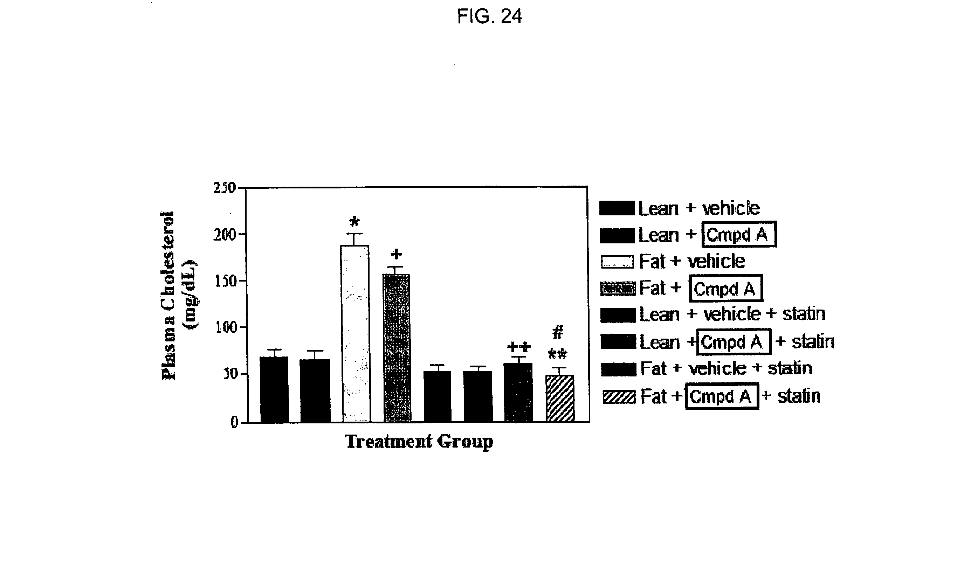

Figure 24 is a graphic representation of the effects of simvastatin, referred generally as "statin," and Compound A on plasma cholesterol (Chol) in db/db mice. Results represent the mean +/- SEM of eight treated mice and are expressed as mg/dL. Fat mice show a significant increase in Chol (*P < 0.01) which was significantly inhibited by Compound A (+P < 0.01). Simvastatin alone also significantly decreased the levels of Chol (++P > 0.01) above the fat mice treated with vehicle alone. The combination of simvastatin and Compound A also significantly decreased the levels of Chol when compared to the fat (**P < 0.01). However, there was no significant difference between the Compound A plus simvastatin and the simvastatin alone (#P > 0.05).

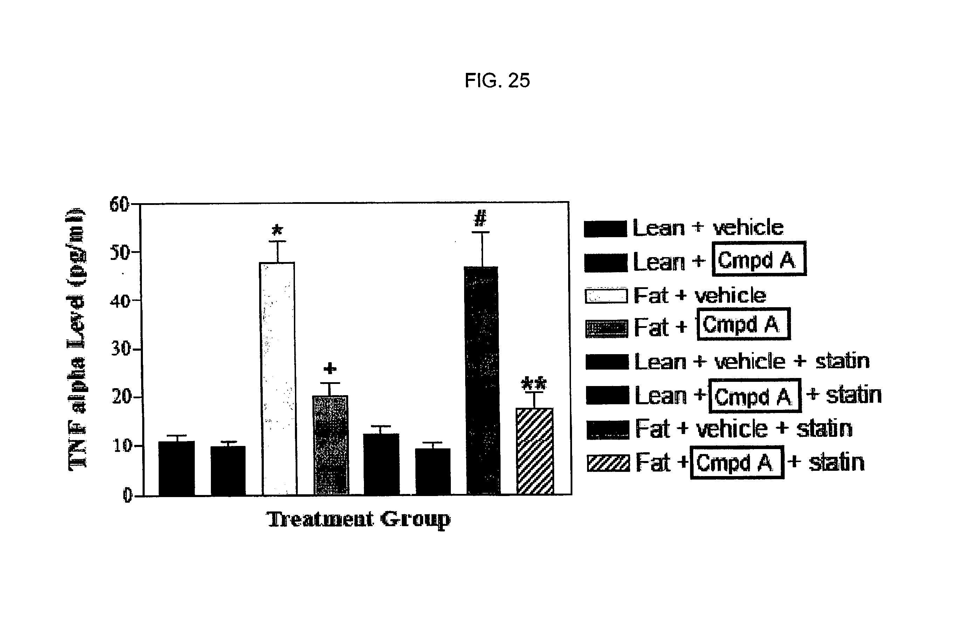

Figure 25 is a graphic representation of the effects of simvastatin, referred generally as "statin," and Compound A on plasma TNFα in db/db mice. Results represent the mean +/- SEM of eight treated mice and are expressed as pg/ml. Fat mice show a significant increase in TNFα (*P < 0.01) which was significantly inhibited by Compound A (+P < 0.01). Simvastatin alone did not significantly inhibit the increased in TNFα (#P > 0.05). However, the combination of simvastatin and Compound A had a significant effect in lowering the levels of TNFa (**P < 0.01).

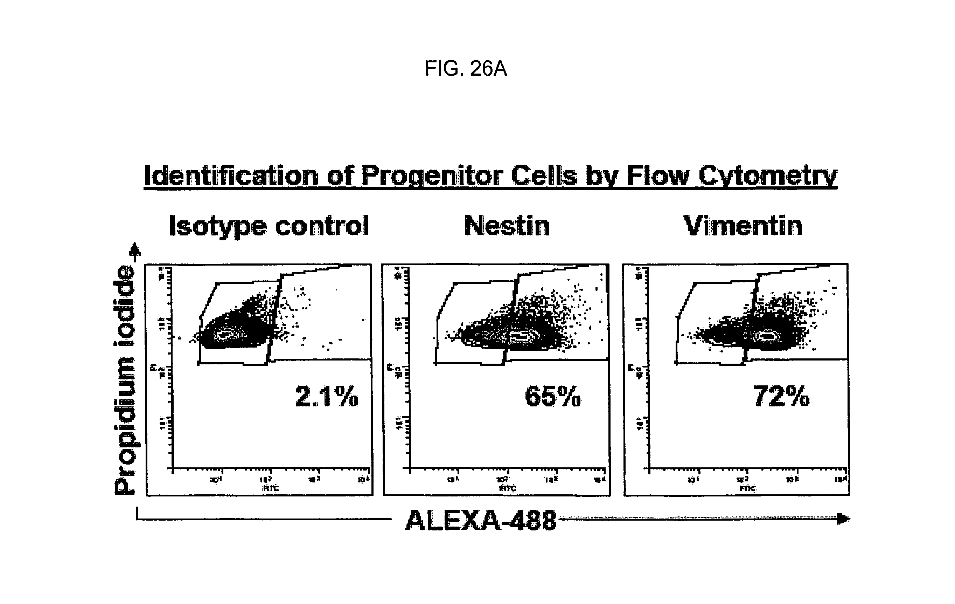

Figure 26A is an illustration of the identification of hippocampal progenitor cells using flow cytometry.

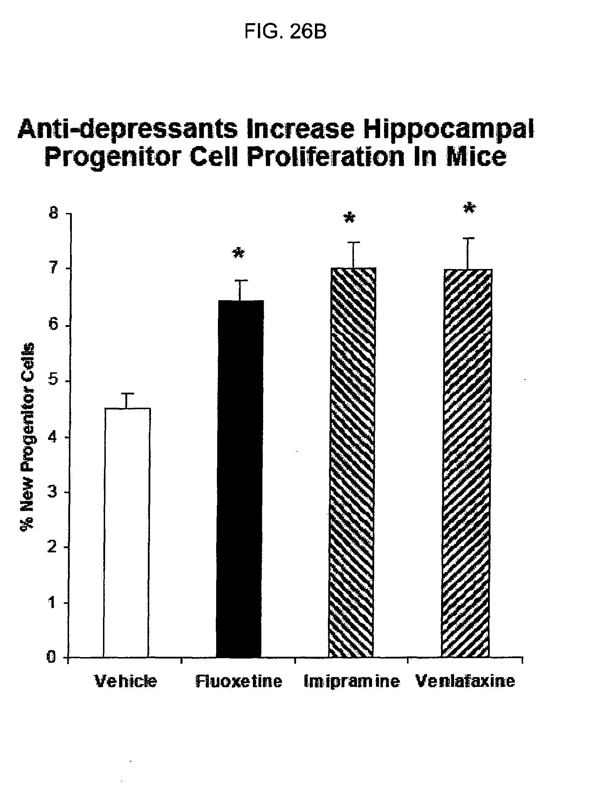

Figure 26B is a graphic representation of the effect of anti-depressants on hippocampal progenitor proliferation in mice.

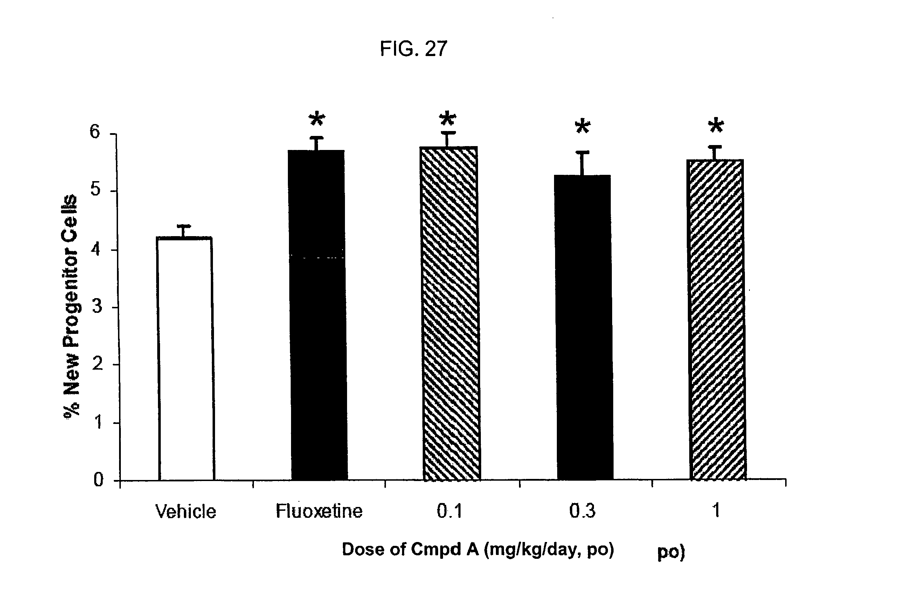

Figure 27 is a graphic representation of the effect of Compound A on hippocampal progenitor cell proliferation.

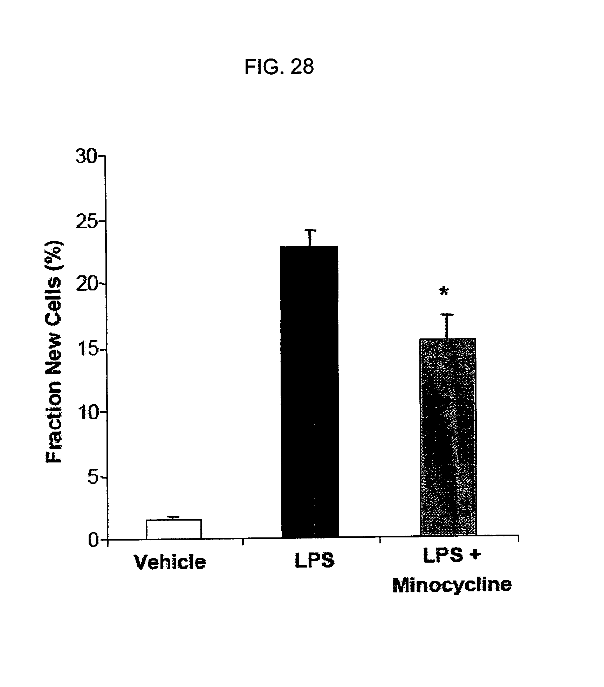

Figure 28 is a graphic representation illustrating a microglial cell proliferation assay.

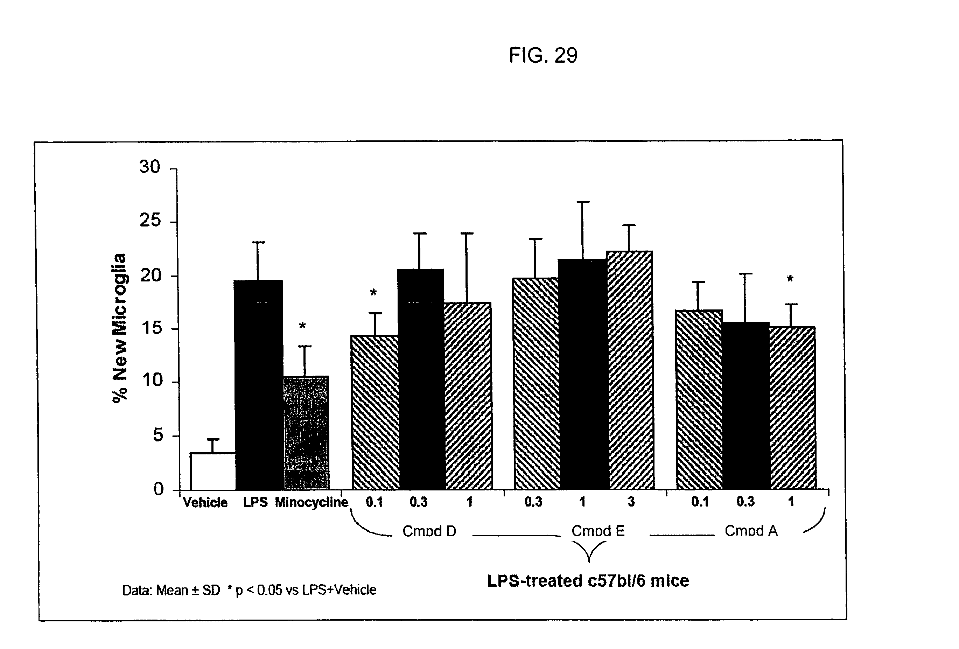

Figure 29 is a graphic representation of the effects of nicotine, Compound D, Compound E, and Compound A on microglial cell proliferation in an LPS-induced model of neuroinflammation.

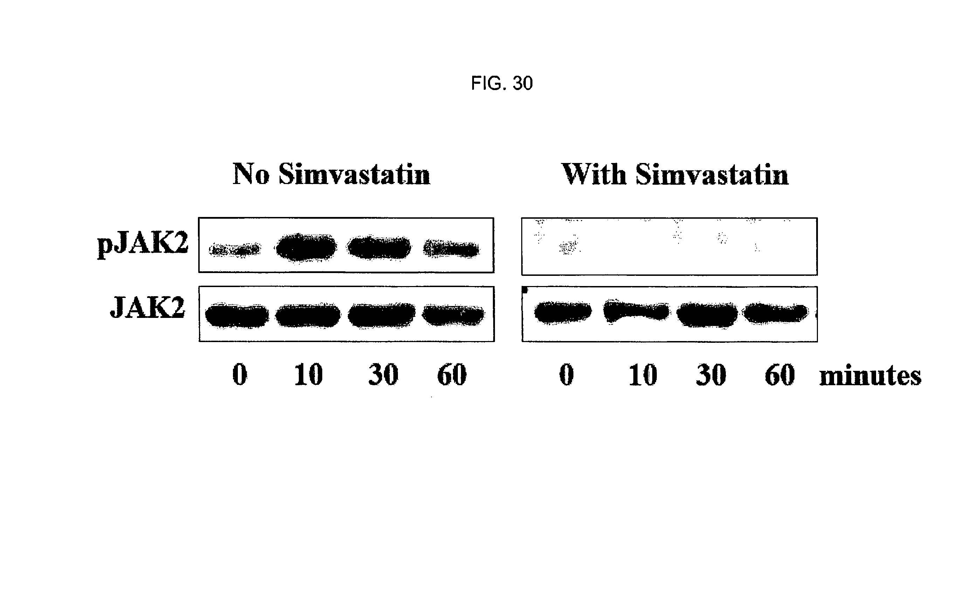

Figure 30 is a is a Western blot showing the effects of simvastatin on the nicotine-induced JAK2 activation in PC12 cells. Cells were pretreated with simvastatin (5 uM) for 24 hours and with nicotine at the time indicated. The methods for blotting are as described (see, Shaw S. et al, J. Biol. Chem., 2002, herein incorporated by reference). Pretreatment of cells with simvastatin significantly inhibited JAK2 activation induced by nicotine for the times indicated.

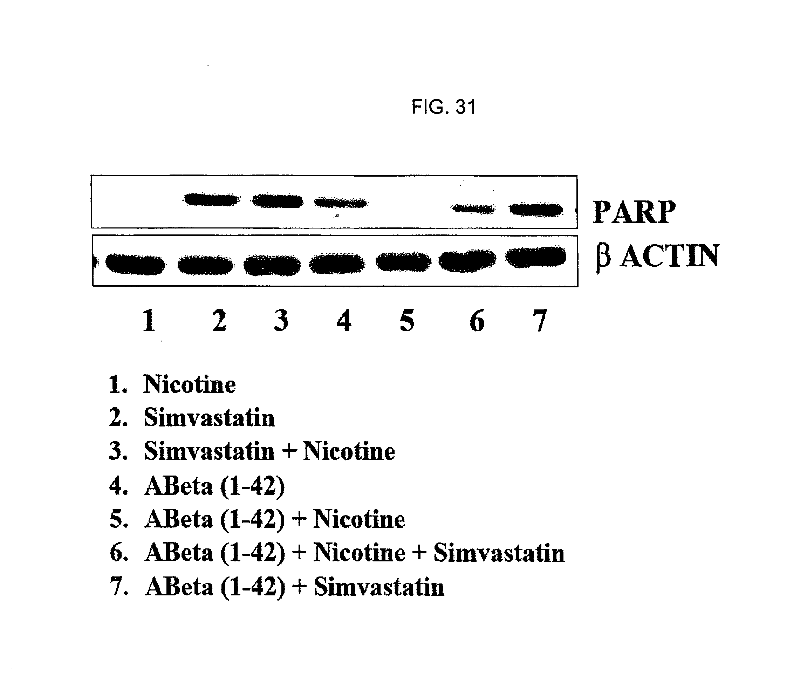

Figure 31 is a Western blot showing the effects of simvastatin on the nicotine-induced neuroprotection against Aβ-induced apoptosis in PC12 cells. The methods are as described. Poly-(ADP-ribose) polymerase (PARP) is marker of cells undergoing apoptosis. PARP expression was determined by Western analysis of PC12 cells nuclear extract.

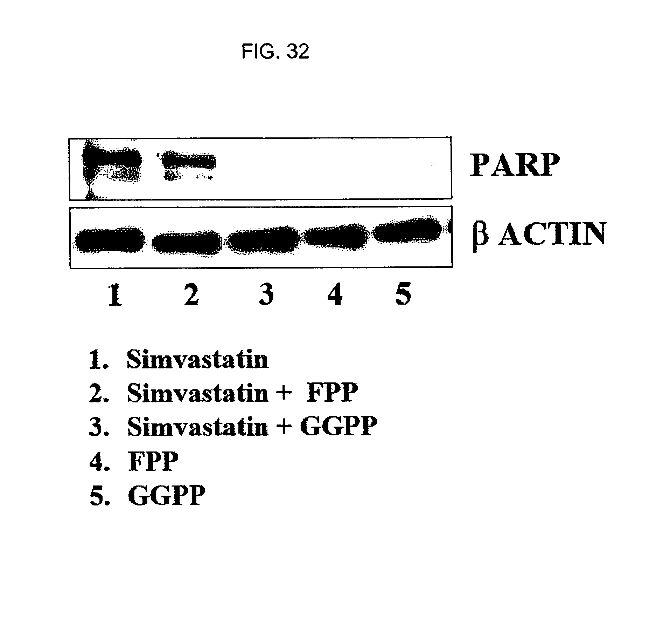

Figure 32 is a Western blot showing the effects of 10 µM farnesyl pyrophosphate (FPP) and geranylgeranyl pyrophosphate (GGPP) on the simvastatin-induced apoptosis in PC12 cells. The methods are as described.

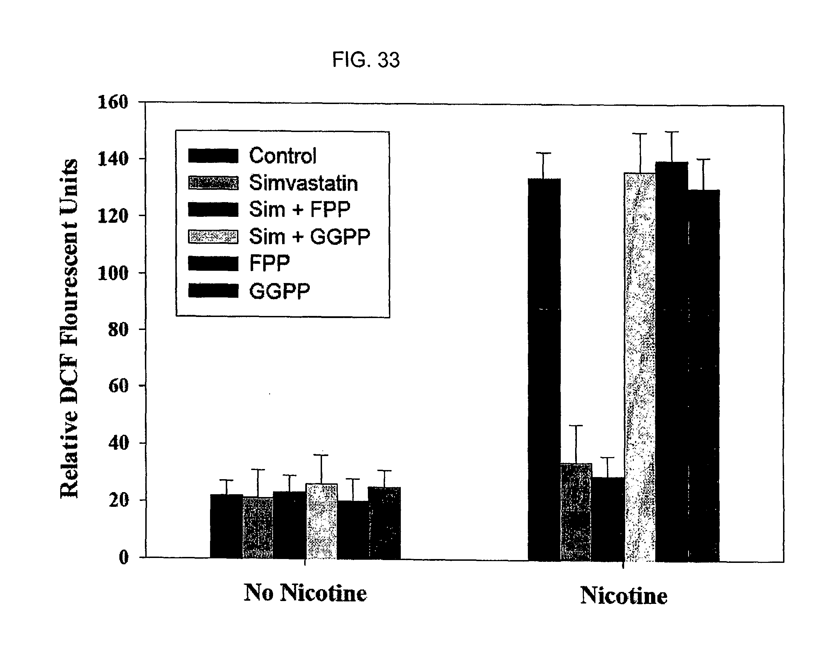

Figure 33 is graphic representation showing the effects of 10 µM farnesyl pyrophosphate (FPP) and geranylgeranyl pyrophosphate (GGPP) on the simvastatin blockade of nicotine-induced ROS production in PC12 cells.

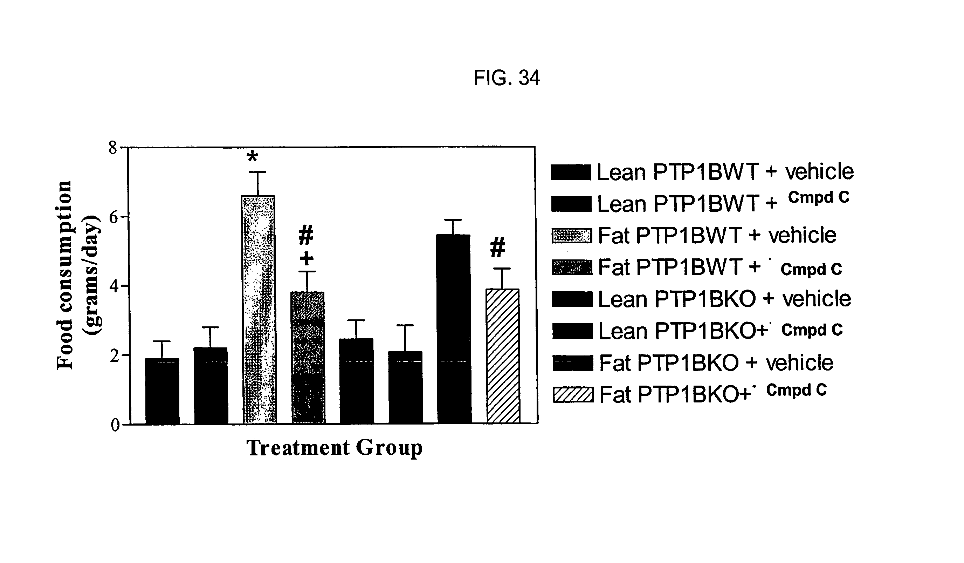

Figure 34 is a graphic representation of the effects of Compound C on food consumption in db/db mice. The illustrated results represent the mean ± SEM of five treated mice/group and are expressed as food consumed in grams/day. Fat mice show a significant increase in food consumption above lean mice (*P < 0.01) which was significantly inhibited by Compound C treatment (+P < 0.01). There is no significant difference between fat wild type and fat PTP-1 B KO mice in decreased food consumption due to Compound C treatment (#P > 0.05).

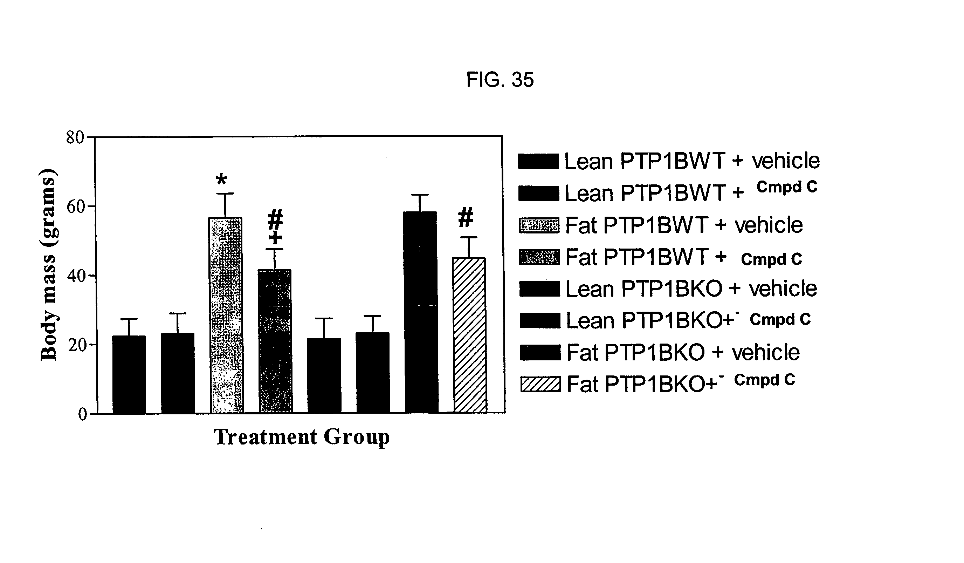

Figure 35 is a graphic representation of the effects of Compound C on body mass in db/db mice. The illustrated results represent the mean ± SEM of five treated mice/group and are expressed as body mass in grams. Fat mice show a significant increase in body mass (*P < 0.01) which was significantly inhibited by Compound C treatment (+P < 0.01). There is no significant difference between fat wild type and fat PTP-1 B KO mice due to Compound C treatment (#P > 0.05).

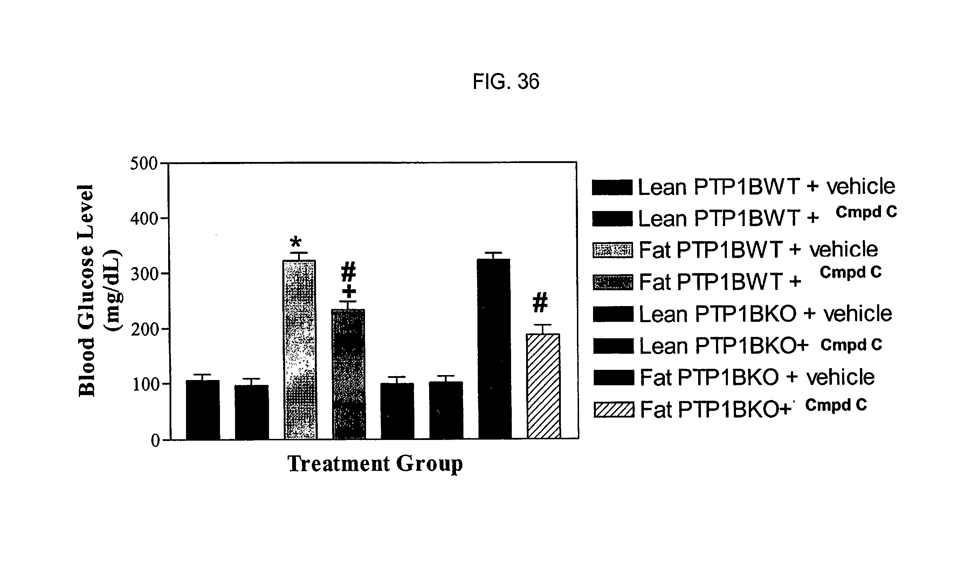

Figure 36 is a graphic representation of the effects of Compound C on plasma blood glucose (BG) in db/db mice. The illustrated results represent the mean ± SEM of five treated mice/group and are expressed as mg/dL of BG. Fat mice show a significant increase in BG (*P < 0.01) which was significantly inhibited by Compound C treatment (+P < 0.01). There is no significant difference between fat wild type and fat PTP-1 B KO mice due to Compound C treatment (#P > 0.05).

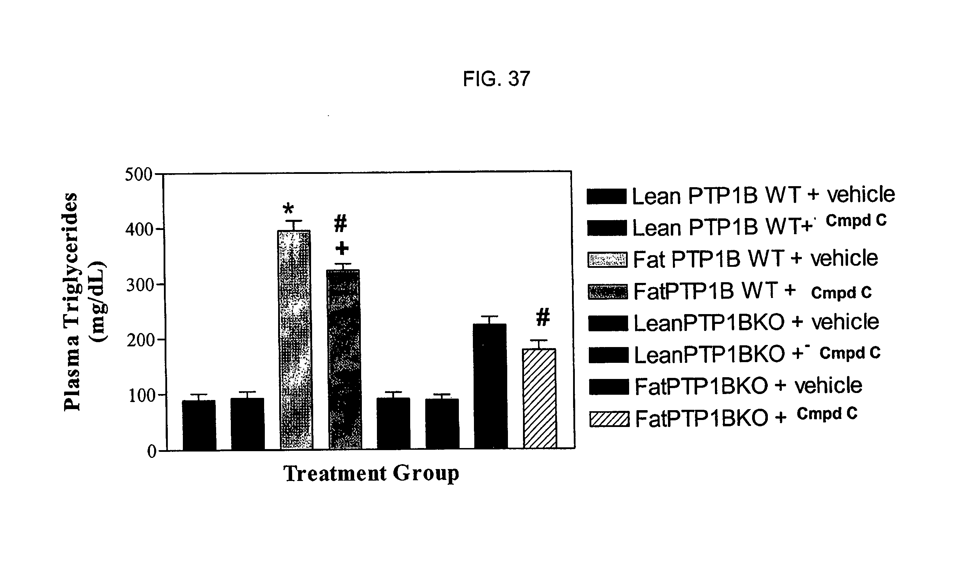

Figure 37 is a graphic representation of the effects of Compound C on plasma triglycerides in db/db mice. The illustrated results represent the mean ± SEM of five treated mice/group and are expressed as mg/dL. Fat mice show a significant increase in plasma TG (*P < 0.01) which was significantly inhibited by Compound C treatment (+P < 0.01) in the fat PTP-1 B wild type but not in the fat PTP-1B KO. There is also a significant difference in plasma TG levels between the treated fat wild type and treated fat PTP-1B KO (#P < 0.01).

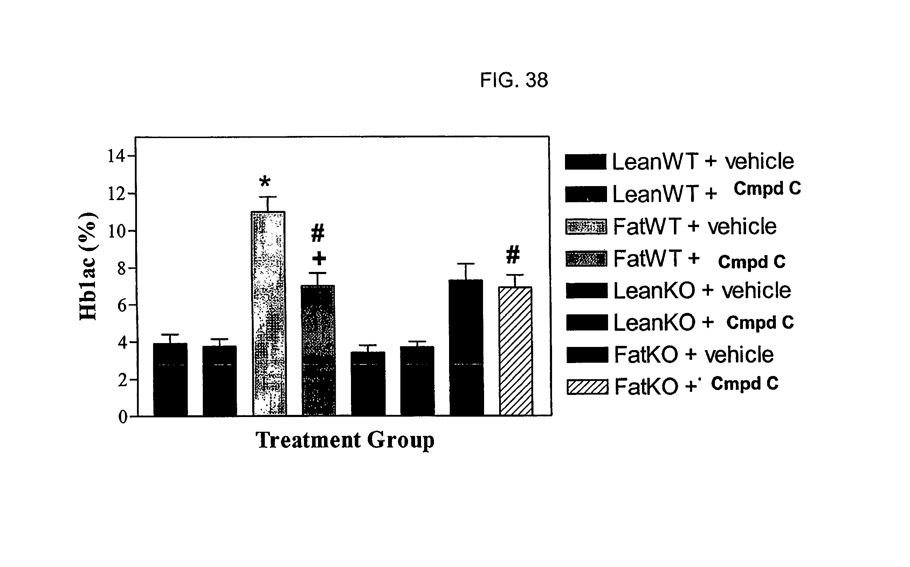

Figure 38 is a graphic representation of the effects of Compound C on plasma glycosylated hemoglobin (Hb1ac) in db/db mice. The illustrated results represent the mean ± SEM of five treated mice/group and are expressed as %. Fat mice show a significant increase in plasma Hb1ac (*P < 0.01) which was significantly inhibited by Compound C treatment (+P < 0.01). There is no significant difference in plasma Hb1ac between fat PTP-1 B wild type and fat PTP-1 B KO (#P > 0.05).

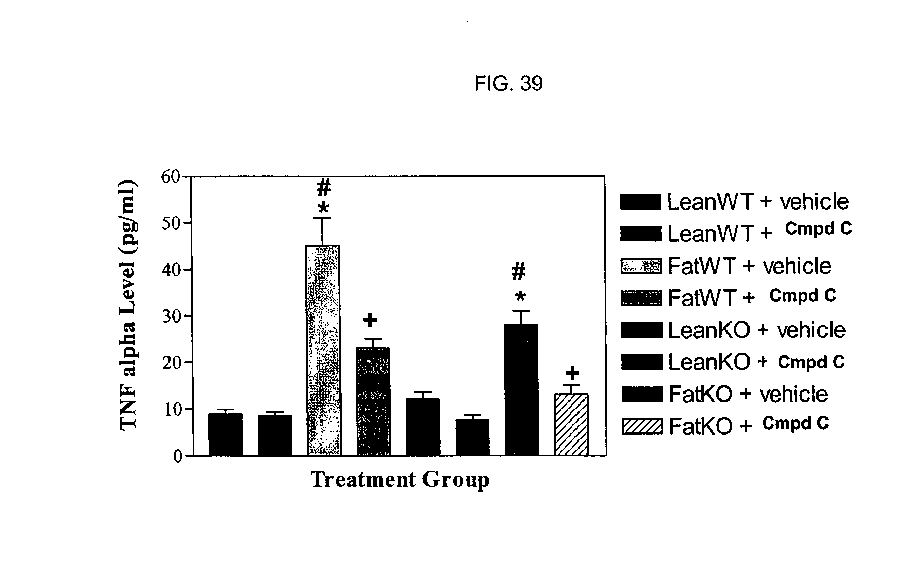

Figure 39 is a graphic representation of the effects of Compound C on TNFα in db/db mice. The illustrated results represent the mean ± SEM of five treated mice/group and are expressed as pg/mL. Fat mice show a significant increase in TNFα (*P < 0.01) which was significantly inhibited by Compound C treatment (+P < 0.01). There is a significant difference between TNFα plasma levels between the fat wild type and fat PTP-1 B KO (#P < 0.01).

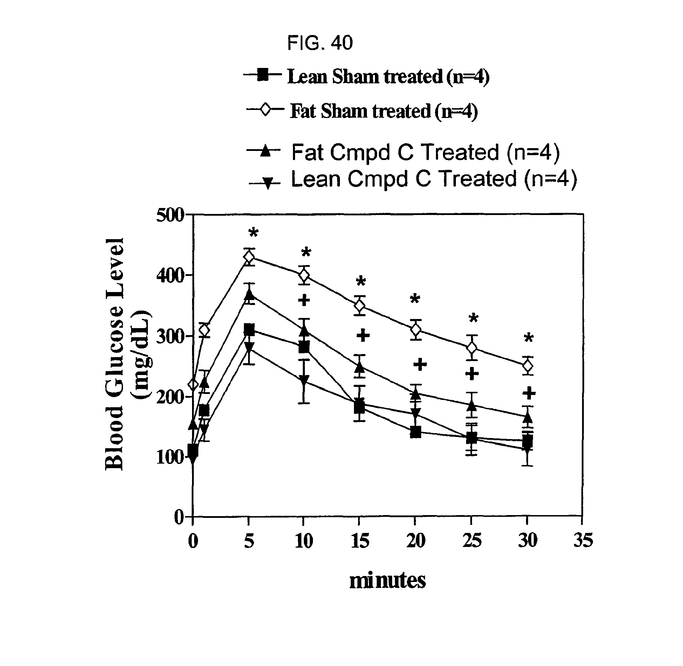

Figure 40 is a graphic representation of the effects of Compound C in the glucose tolerance test (GTT) in db/db mice PTP-1B wild type mice. The illustrated results represent the mean ± SEM of four treated mice/group and are expressed as mg/dL. Fat mice show a significant decrease in glucose deposition (*P < 0.01) with Compound C treatment. Fat mice show a significant increase in deposition (+P < 0.01).

### Detailed Description of the Preferred Embodiments

One aspect of the present invention includes the role of α7 nAChRs in regulating key biological pathways involved in the metabolic syndrome and the potential of selective α7 nAChR agonists as a novel therapeutic approach to treat this condition. Although α7 has been implicated in the cholinergic inflammatory pathway, the evidence is based exclusively on the use of non-selective agonists in the presence of putative selective antagonists, some with rather poor pharmacokinetics or brain penetration properties. Thus, another aspect of the present invention includes compounds (hereinafter defined and referred to as Compounds A, B, or C) with high selectivity for the α7 nAChR.

Compound A is (5-methyl-N-[(2S,3R)-2-(pyridin-3-ylmethyl)-1-azabicyclo[2.2.2]oct-3-yl]thiophene-2-carboxamide), illustrated below. 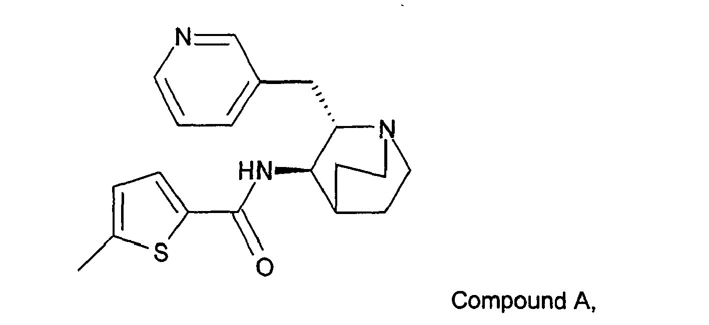 or a pharmaceutically acceptable salt thereof. As will be appreciated, alternate naming conventions provide alternative names. Thus, Compound A may also be referred to as (2S,3R)-N-(2-((3-pyridinyl)methyl)-1-azabicyclo[2.2.2]octan-3-yl)-5-methylthiophene-2-carboxamide. Such naming conventions should not impact the clarity of the present invention.

Compound B is (2S,3R)-N-(2-((3-pyridinyl)methyl)-1-azabicyclo[2.2.2]oct-3-yl)-5-(2-pyridinyl)thiophene-2-carboxamide, illustrated below: 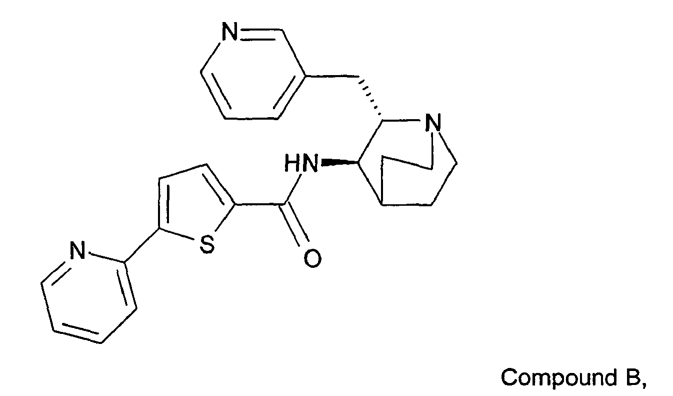 or a pharmaceutically acceptable salt thereof.

Compound C is (2S,3R)-N-(2-((3-pyridinyl)methyl)-1-azabicyclo[2.2.2]oct-3-yl)benzofuran-2-carboxamide, illustrated below: 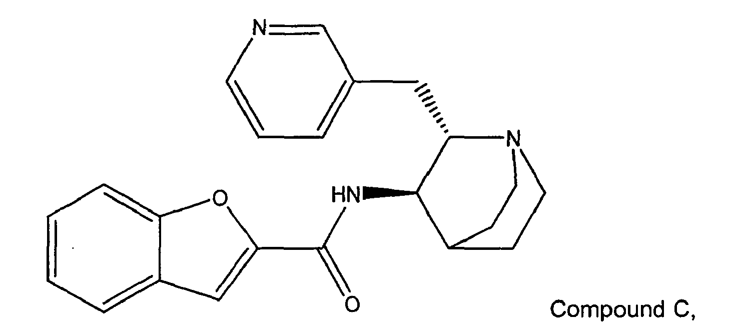 or a pharmaceutically acceptable salt thereof.

In the studies of this application, evidence is presented showing that α7-selective ligands inhibit the metabolic syndrome observed in db/db mice by reducing weight gain, normalizing glucose levels, increasing insulin secretion, decreasing glycated hemoglobin, reducing pro-inflammatory cytokines, reducing triglycerides, and normalizing insulin resistance glucose tolerance test. These data indicate that a7-selective ligands are useful for the management of the metabolic syndrome (diabetes I and II, atherosclerosis, obesity).

Another aspect of the present invention provides methods and compositions relating to co-administration of α7 selective ligands with statins, in order to decrease unwanted side effects of statins, including increased glycemia. Relevant statins include atorvastatin, cerivastatin, fluvastatin, lovastatin, mevastatin, pitavastatin, pravastatin, rosuvastatin, simvastatin, and additional statins, defined based on their inhibition of HMG CoA reductase. Although trade names or generic names may be used herein, reference is had to the underlying active ingredient(s) in such drug products.

### Compounds

Compounds useful according to the present invention are α7 NNR selective ligands, as exemplified by Compounds A, B, and C, herein.

Compounds A, B and C are members of a genus of compounds described in US Patent 6,953,855 (incorporated herein by reference in its entirety). US Patent 6,953,855 includes compounds represented by Formula 1.

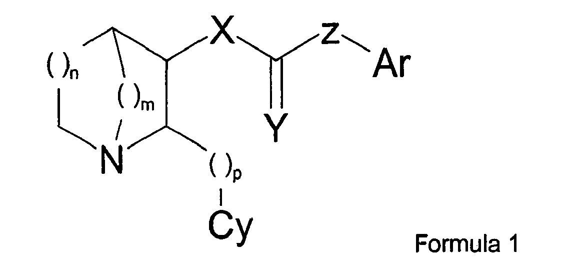

In Formula 1, m and n individually can have a value of 1 or 2, and p can have a value of 1, 2, 3 or 4. In the Formula, X is either oxygen or nitrogen (i.e., NR'), Y is either oxygen or sulfur, and Z is either nitrogen (i.e., NR'), a covalent bond or a linker species, A. A is selected from the group -CR' R"-, -CR' R"- CR' R"-, -CR'= CR'-, and -C₂-, wherein R' and R" are as hereinafter defined. When Z is a covalent bond or A, X must be nitrogen. Ar is an aryl group, either carbocyclic or heterocyclic, either monocyclic or fused polycyclic, unsubstituted or substituted; and Cy is a 5- or 6-membered heteroaromatic ring, unsubstituted or substituted. Thus, the invention includes compounds in which Ar is linked to the azabicycle by a carbonyl group-containing functionality, such as an amide, carbamate, urea, thioamide, thiocarbamate or thiourea functionality. In addition, in the case of the amide and thioamide functionalities, Ar may be bonded directly to the carbonyl (or thiocarbonyl) group or may be linked to the carbonyl (or thiocarbonyl) group through linker A. Furthermore, the invention includes compounds that contain a 1-azabicycle, containing either a 5-, 6-, or 7-membered ring and having a total of 7, 8 or 9 ring atoms (e.g., 1-azabicyclo[2.2.1]heptane, 1-azabicyclo[3.2.1]octane, 1-azabicyclo[2.2.2]octane, and 1-azabicyclo[3.2.2]nonane).

In one embodiment, the value of p is 1, Cy is 3-pyridinyl or 5-pyrimidinyl, X and Y are oxygen, and Z is nitrogen. In another embodiment, the value of p is 1, Cy is 3-pyridinyl or 5-pyrimidinyl, X and Z are nitrogen, and Y is oxygen. In a third embodiment, the value of p is 1, Cy is 3-pyridinyl or 5-pyrimidinyl, X is nitrogen, Y is oxygen, and Z is a covalent bond (between the carbonyl and Ar). In a fourth embodiment, the value of p is 1, Cy is 3-pyridinyl or 5-pyrimidinyl, X is nitrogen, Y is oxygen, Z is A (a linker species between the carbonyl and Ar).
The compounds of Formula 1 have one or more asymmetric carbons and can therefore exist in the form of racemic mixtures, enantiomers and diastereomers. Both relative and absolute stereochemistry at asymmetric carbons are variable (e.g., *cis* or *trans,* R or S). In addition, some of the compounds exist as E and Z isomers about a carbon-carbon double bond. All these individual isomeric compounds and their mixtures are also intended to be within the scope of Formula 1.

As used in Formula 1, Ar ("aryl") includes both carbocyclic and heterocyclic aromatic rings, both monocyclic and fused polycyclic, where the aromatic rings can be 5- or 6-membered rings. Representative monocyclic aryl groups include, but are not limited to, phenyl, furanyl, pyrrolyl, thienyl, pyridinyl, pyrimidinyl, oxazolyl, isoxazolyl, pyrazolyl, imidazolyl, thiazolyl, isothiazolyl and the like. Fused polycyclic aryl groups are those aromatic groups that include a 5- or 6-membered aromatic or heteroaromatic ring as one or more rings in a fused ring system. Representative fused polycyclic aryl groups include naphthalene, anthracene, indolizine, indole, isoindole, benzofuran, benzothiophene, indazole, benzimidazole, benzthiazole, purine, quinoline, isoquinoline, cinnoline, phthalazine, quinazoline, quinoxaline, 1,8-naphthyridine, pteridine, carbazole, acridine, phenazine, phenothiazine, phenoxazine, and azulene.
As used in Formula 1, "Cy" groups are 5- and 6-membered ring heteroaromatic groups. Representative Cy groups include pyridinyl, pyrimidinyl, furanyl, pyrrolyl, thienyl, oxazolyl, isoxazolyl, pyrazolyl, imidazolyl, thiazolyl, isothiazolyl and the like, where pyridinyl is preferred.
Individually, Ar and Cy can be unsubstituted or can be substituted with 1, 2 or 3 substituents, such as alkyl, alkenyl, heterocyclyl, cycloalkyl, aryl, substituted aryl, arylalkyl, substituted arylalkyl, halo (e.g., F, Cl, Br, or I), -OR', -NR'R", -CF₃, -CN, -NO₂, -C₂R', -SR', -N₃, -C(=O)NR'R", -NR'C(=O) R", -C(=O)R', -C(=O)OR', -OC(=O)R', -O(CR'R")ᵣC(=O)R', -O(CR'R")ᵣNR"C(=O)R', -O(CR'R")ᵣNR"SO₂R', -OC(=O)NR'R", -NR'C(=O)O R", -SO₂R', -SO₂NR'R", and -NR'SO₂R", where R' and R" are individually hydrogen, C₁-C₈ alkyl (e.g., straight chain or branched alkyl, preferably C₁-C₅, such as methyl, ethyl, or isopropyl), cycloalkyl (e.g., C₃-₈ cyclic alkyl), heterocyclyl, aryl, or arylalkyl (such as benzyl), and r is an integer from 1 to 6. R' and R" can also combine to form a cyclic functionality.

Compounds of Formula 1 form acid addition salts which are useful according to the present invention. Examples of suitable pharmaceutically acceptable salts include inorganic acid addition salts such as chloride, bromide, sulfate, phosphate, and nitrate; organic acid addition salts such as acetate, galactarate, propionate, succinate, lactate, glycolate, malate, tartrate, citrate, maleate, fumarate, methanesulfonate, p-toluenesulfonate, and ascorbate; salts with acidic amino acid such as aspartate and glutamate. The salts may be in some cases hydrates or ethanol solvates.

Representative compounds of Formula 1 include:
2-((3-pyridinyl)methyl)-1-azabicyclo[2.2.2]oct-3-yl N-phenylcarbamate,
2-((3-pyridinyl)methyl)-1-azabicyclo[2.2.2]oct-3-yl N-(4-fluorophenyl)carbamate,
2-((3-pyridinyl)methyl)-1-azabicyclo[2.2.2]oct-3-yl N-(4-chlorophenyl)carbamate,
2-((3-pyridinyl)methyl)-1-azabicyclo[2.2.2]oct-3-yl N-(4-bromophenyl)carbamate,
2-((3-pyridinyl)methyl)-1-azabicyclo[2.2.2]oct-3-yl N-(3-fluorophenyl)carbamate,
2-((3-pyridinyl)methyl)-1-azabicyclo[2.2.2]oct-3-yl N-(3-chlorophenyl)carbamate,
2-((3-pyridinyl)methyl)-1-azabicylo[2.2.2]oct-3-yl N-(3-bromophenyl)carbamate,
2-((3-pyridinyl)methyl)-1-azabicyclo[2.2.2]oct-3-yl N-(2-fluorophenyl)carbamate,
2-((3-pyridinyl)methyl)-1-azabicyclo[2.2.2]oct-3-yl N-(2-chlorophenyl)carbamate,
2-((3-pyridinyl)methyl)-1-azabicyclo[2.2.2]oct-3-yl N-(2-bromophenyl)carbamate,
2-((3-pyridinyl)methyl)-1-azabicyclo[2.2.2]oct-3-yl N-(3,4-dichlorophenyl)carbamate,
2-((3-pyridinyl)methyl)-1-azabicyclo[2.2.2]oct-3-yl N-(2-methylphenyl)carbamate,
2-((3-pyridinyl)methyl)-1-azabicyclo[2.2.2]oct-3-yl N-(2-biphenyl)carbamate,
2-((3-pyridinyl)methyl)-1-azabicyclo[2.2.2]oct-3-yl N-(3-methylphenyl)carbamate,
2-((3-pyridinyl)methyl)-1-azabicyclo[2.2.2]oct-3-yl N-(3-biphenyl)carbamate,
2-((3-pyridinyl)methyl)-1-azabicyclo[2.2.2]oct-3-yl N-(4-methylphenyl)carbamate,
2-((3-pyridinyl)methyl)-1-azabicyclo[2.2.2]oct-3-yl N-(4-biphenyl)carbamate,
2-((3-pyridinyl)methyl)-1-azabicyclo[2.2.2]oct-3-yl N-(2-cyanophenyl)carbamate,
2-((3-pyridinyl)methyl)-1-azabicyclo[2.2.2]oct-3-yl N-(3-cyanophenyl)carbamate,
2-((3-pyridinyl)methyl)-1-azabicyclo[2.2.2]oct-3-yl N-(4-cyanophenyl)carbamate,
2-((3-pyridinyl)methyl)-1-azabicyclo[2.2.2]oct-3-yl N-(3-trifluoromethylphenyl)carbamate,
2-((3-pyridinyl)methyl)-1-azabicyclo[2.2.2]oct-3-yl N-(4-dimethylaminophenyl)carbamate,
2-((3-pyridinyl)methyl)-1-azabicyclo[2.2.2]oct-3-yl N-(2-methoxyphenyl)carbamate,
2-((3-pyridinyl)methyl)-1-azabicyclo[2.2.2]oct-3-yl N-(2-phenoxyphenyl)carbamate,
2-((3-pyridinyl)methyl)-1-azabicyclo[2.2.2]oct-3-yl N-(2-methylthiophenyl)carbamate,
2-((3-pyridinyl)methyl)-1-azabicyclo[2.2.2]oct-3-yl N-(2-phenylthiophenyl)carbamate,
2-((3-pyridinyl)methyl)-1-azabicyclo[2.2.2]oct-3-yl N-(3-methoxyphenyl)carbamate,
2-((3-pyridinyl)methyl)-1-azabicyclo[2.2.2]oct-3-yl N-(3-phenoxyphenyl)carbamate,
2-((3-pyridinyl)methyl)-1-azabicyclo[2.2.2]oct-3-yl N-(3-methylthiophenyl)carbamate,
2-((3-pyridinyl)methyl)-1-azabicyclo[2.2.2]oct-3-yl N-(3-phenylthiophenyl)carbamate,
2-((3-pyridinyl)methyl)-1-azabicyclo[2.2.2]oct-3-yl N-(4-methoxyphenyl)carbamate,
2-((3-pyridinyl)methyl)-1-azabicyclo[2.2.2]oct-3-yl N-(4-phenoxyphenyl)carbamate,
2-((3-pyridinyl)methyl)-1-azabicyclo[2.2.2]oct-3-yl N-(4-methylthiophenyl)carbamate,
2-((3-pyridinyl)methyl)-1-azabicyclo[2.2.2]oct-3-yl N-(4-phenylthiophenyl)carbamate,
2-((3-pyridinyl)methyl)-1-azabicyclo[2.2.2]oct-3-yl N-(2,4-dimethoxyphenyl)carbamate,
2-((3-pyridinyl)methyl)-1-azabicyclo[2.2.2]oct-3-yl N-(2-thienyl)carbamate,
2-((3-pyridinyl)methyl)-1-azabicyclo[2.2.2]oct-3-yl N-(3-thienyl)carbamate,
2-((3-pyridinyl)methyl)-1-azabicyclo[2.2.2]oct-3-yl N-(3-benzothienyl)carbamate,
2-((3-pyridinyl)methyl)-1-azabicyclo[2.2.2]oct-3-yl N-(1-naphthyl)carbamate, and
2-((3-pyridinyl)methyl)-1-azabicyclo[2.2.2]oct-3-yl N-(2-naphthyl)carbamate.

Other compounds representative of Formula 1 include:
N-phenyl-N'-(2-((3-pyridinyl)methyl)-1-azabicyclo[2.2.2]oct-3-yl)urea,
N-(4-fluorophenyl)-N'-(2-((3-pyridinyl)methyl)-1-azabicyclo[2.2.2]oct-3-yl)urea,
N-(4-chlorophenyl)-N'-(2-((3-pyridinyl)methyl)-1-azabicyclo[2.2.2]oct-3-yl)urea,
N-(4-bromophenyl)-N'-(2-((3-pyridinyl)methyl)-1-azabicyclo[2.2.2]oct-3-yl)urea,
N-(3-fluorophenyl)-N'-(2-((3-pyridinyl)methyl)-1-azabicyclo[2.2.2]oct-3-yl)urea,
N-(3-chlorophenyl)-N'-(2-((3-pyridinyl)methyl)-1-azabicyclo[2.2.2]oct-3-yl)urea,
N-(3-bromophenyl)-N'-(2-((3-pyridinyl)methyl)-1-azabicyclo[2.2.2]oct-3-yl)urea,
N-(2-fluorophenyl)-N'-(2-((3-pyridinyl)methyl)-1-azabicyclo[2.2.2]oct-3-yl)urea,
N-(2-chlorophenyl)-N'-(2-((3-pyridinyl)methyl)-1-azabicyclo[2.2.2]oct-3-yl)urea,
N-(2-bromophenyl)-N'-(2-((3-pyridinyl)methyl)-1-azabicyclo[2.2.2]oct-3-yl)urea,
N-(3,4-dichlorophenyl)-N'-(2-((3-pyridinyl)methyl)-1-azabicyclo[2.2.2]oct-3-yl)urea,
N-(2-methylphenyl)-N'-(2-((3-pyridinyl)methyl)-1-azabicyclo[2.2.2]oct-3-yl)urea,
N-(2-biphenyl)-N'-(2-((3-pyridinyl)methyl)-1-azabicyclo[2.2.2]oct-3-yl)urea,
N-(3-methylphenyl)-N'-(2-((3-pyridinyl)methyl)-1-azabicyclo[2.2.2]oct-3-yl)urea,
N-(3-biphenyl)-N'-(2-((3-pyridinyl)methyl)-1-azabicyclo[2.2.2]oct-3-yl)urea,
N-(4-methylphenyl)-N'-(2-((3-pyridinyl)methyl)-1-azabicyclo[2.2.2]oct-3-yl)urea,
N-(4-biphenyl)-N'-(2-((3-pyridinyl)methyl)-1-azabicyclo[2.2.2]oct-3-yl)urea,
N-(2-cyanophenyl)-N'-(2-((3-pyridinyl)methyl)-1-azabicyclo[2.2.2]oct-3-yl)urea,
N-(3-cyanophenyl)-N'-(2-((3-pyridinyl)methyl)-1-azabicyclo(2.2.2]oct-3-yl)urea,
N-(4-cyanophenyl)-N'-(2-((3-pyridinyl)methyl)-1-azabicyclo[2.2.2]oct-3-yl)urea,
N-(3-trifluoromethylphenyl)-N'-(2-((3-pyridinyl)methyl)-1-azabicyclo[2.2.2]oct-3-yl)urea,
N-(4-dimethylaminophenyl)-N'-(2-((3-pyridinyl)methyl)-1-azabicyclo[2.2.2]oct-3-yl)urea,
N-(2-methoxyphenyl)-N'-(2-((3-pyridinyl)methyl)-1-azabicyclo[2.2.2]oct-3-yl)urea,
N-(2-phenoxyphenyl)-N'-(2-((3-pyridinyl)methyl)-1-azabicyclo[2.2.2]oct-3-yl)urea,
N-(2-methylthiophenyl)-N'-(2-((3-pyridinyl)methyl)-1-azabicyclo[2.2.2]oct-3-yl)urea,
N-(2-phenylthiophenyl)-N'-(2-((3-pyridinyl)methyl)-1-azabicyclo[2.2.2]oct-3-yl)urea,
N-(3-methoxyphenyl)-N'-(2-((3-pyridinyl)methyl)-1-azabicyclo[2.2.2]oct-3-yl)urea,
N-(3-phenoxyphenyl)-N'-(2-((3-pyridinyl)methyl)-1-azabicyclo[2.2.2]oct-3-yl)urea,
N-(3-methylthiophenyl)-N'-(2-((3-pyridinyl)methyl)-1-azabicyclo[2.2.2]oct-3-yl)urea,
N-(3-phenylthiophenyl)-N'-(2-((3-pyridinyl)methyl)-1-azabicyclo[2.2.2]oct-3-yl)urea,
N-(4-methoxyphenyl)-N'-(2-((3-pyridinyl)methyl)-1-azabicyclo[2.2.2]oct-3-yl)urea,
N-(4-phenoxyphenyl)-N'-(2-((3-pyridinyl)methyl)-1-azabicyclo[2.2.2]oct-3-yl)urea,
N-(4-methylthiophenyl)-N'-(2-((3-pyridinyl)methyl)-1-azabicyclo[2.2.2]oct-3-yl)urea,
N-(4-phenylthiophenyl)-N'-(2-((3-pyridinyl)methyl)-1-azabicyclo[2.2.2]oct-3-yl)urea,
N-(2,4-dimethoxyphenyl)-N'-(2-((3-pyridinyl)methyl)-1-azabicyclo[2.2.2]oct-3-yl)urea,
N-(2-thienyl)-N'-(2-((3-pyridinyl)methyl)-1-azabicyclo[2.2.2]oct-3-yl)urea,
N-(3-thienyl)-N'-(2-((3-pyridinyl)methyl)-1-azabicyclo[2.2.2]oct-3-yl)urea,
N-(3-benzothienyl)-N'-(2-((3-pyridinyl)methyl)-1-azabicyclo[2.2.2]oct-3-yl)urea,
N-(1-naphthyl)-N'-(2-((3-pyridinyl)methyl)-1-azabicyclo[2.2.2]oct-3-yl)urea, and
N-(2-naphthyl)-N'-(2-((3-pyridinyl)methyl)-1-azabicyclo[2.2.2]oct-3-yl)urea.

Other compounds representative of Formula 1 include:
N-(2-((3-pyridinyl)methyl)-1-azabicyclo[2.2.2]oct-3-yl)benzamide,
N-(2-((3-pyridinyl)methyl)-1-azabicyclo[2.2.2]oct-3-yl)-2-fluorobenzamide,
N-(2-((3-pyridinyl)methyl)-1-azabicyclo[2.2.2]oct-3-yl)-3-fluorobenzamide,
N-(2-((3-pyridinyl)methyl)-1-azabicyclo[2.2.2]oct-3-yl)-4-fluorobenzamide,
N-(2-((3-pyridinyl)methyl)-1-azabicyclo[2.2.2]oct-3-yl)-2-chlorobenzamide,
N-(2-((3-pyridinyl)methyl)-1-azabicyclo[2.2.2]oct-3-yl)-3-chlorobenzamide,
N-(2-((3-pyridinyl)methyl)-1-azabicyclo[2.2.2]oct-3-yl)-4-chlorobenzamide,
N-(2-((3-pyridinyl)methyl)-1-azabicyclo[2.2.2]oct-3-yl)-2-bromobenzamide,
N-(2-((3-pyridinyl)methyl)-1-azabicyclo[2.2.2]oct-3-yl)-3-bromobenzamide,
N-(2-((3-pyridinyl)methyl)-1-azabicyclo[2.2.2]oct-3-yl)-4-bromobenzamide,
N-(2-((3-pyridinyl)methyl)-1-azabicyclo[2.2.2]oct-3-yl)-3,4-dichlorobenzamide,
N-(2-((3-pyridinyl)methyl)-1-azabicyclo[2.2.2]oct-3-yl)-2-methylbenzamide,
N-(2-((3-pyridinyl)methyl)-1-azabicyclo[2.2.2]oct-3-yl)-3-methylbenzamide,
N-(2-((3-pyridinyl)methyl)-1-azabicyclo[2.2.2]oct-3-yl)-4-methylbenzamide,
N-(2-((3-pyridinyl)methyl)-1-azabicyclo[2.2.2]oct-3-yl)-2-phenylbenzamide,
N-(2-((3-pyridinyl)methyl)-1-azabicyclo[2.2.2]oct-3-yl)-3-phenylbenzamide,
N-(2-((3-pyridinyl)methyl)-1-azabicyclo[2.2.2]oct-3-yl)-4-phenylbenzamide,
N-(2-((3-pyridiny))methyl)-1-azabicyclo[2.2.2]oct-3-yl)-2-cyanobenzamide,
N-(2-((3-pyridinyl)methyl)-1-azabicyclo[2.2.2]oct-3-yl)-3-cyanobenzamide,
N-(2-((3-pyridinyl)methyl)-1-azabicyclo[2.2.2]oct-3-yl)-4-cyanobenzamide,
N-(2-((3-pyridinyl)methyl)-1-azabicyclo[2.2.2]oct-3-yl)-3-trifluoromethylbenzamide,
N-(2-((3-pyridinyl)methyl)-1-azabicyclo[2.2.2]oct-3-yl)-4-dimethylaminobenzamide,
N-(2-((3-pyridinyl)methyl)-1-azabicyclo[2.2.2]oct-3-yl)-2-methoxybenzamide,
N-(2-((3-pyridinyl)methyl)-1-azabicyclo[2.2.2]oct-3-yl)-3-methoxybenzamide,
N-(2-((3-pyridinyl)methyl)-1-azabicyclo[2.2.2]oct-3-yl)-4-methoxybenzamide,
N-(2-((3-pyridinyl)methyl)-1-azabicyclo[2.2.2]oct-3-yl)-2-phenoxybenzamide,
N-(2-((3-pyridinyl)methyl)-1-azabicyclo[2.2.2]oct-3-yl)-3-phenoxybenzamide,
N-(2-((3-pyridinyl)methyl)-1-azabicyclo[2.2.2]oct-3-yl)-4-phenoxybenzamide,
N-(2-((3-pyridinyl)methyl)-1-azabicyclo[2.2.2]oct-3-yl)-2-methylthiobenzamide,
N-(2-((3-pyridinyl)methyl)-1-azabicyclo[2.2.2]oct-3-yl)-3-methylthiobenzamide,
N-(2-((3-pyridinyl)methyl)-1-azabicyclo[2.2.2]oct-3-yl)-4-methylthiobenzamide,
N-(2-((3-pyridinyl)methyl)-1-azabicyclo[2.2.2]oct-3-yl)-2-phenylthiobenzamide,
N-(2-((3-pyridinyl)methyl)-1-azabicyclo[2.2.2]oct-3-yl)-3-phenylthiobenzamide,
N-(2-((3-pyridinyl)methyl)-1-azabicyclo[2.2.2]oct-3-yl)-4-phenylthiobenzamide,
N-(2-((3-pyridinyl)methyl)-1-azabicyclo[2.2.2]oct-3-yl)-2,4-dimethoxybenzamide,
N-(2-((3-pyridinyl)methyl)-1-azabicyclo[2.2.2]oct-3-yl)-5-bromonicotinamide,
N-(2-((3-pyridinyl)methyl)-1-azabicyclo[2.2.2]oct-3-yl)-6-chloronicotinamide,
N-(2-((3-pyridinyl)methyl)-1-azabicyclo[2.2.2]oct-3-yl)-5-phenylnicotinamide,
N-(2-((3-pyridinyl)methyl)-1-azabicyclo[2.2.2]oct-3-yl)furan-2-carboxamide,
N-(2-((3-pyridinyl)methyl)-1-azabicyclo[2.2.2]oct-3-yl)furan-3-carboxamide,
N-(2-((3-pyridinyl)methyl)-1-azabicyclo[2.2.2]oct-3-yl)thiophene-2-carboxamide,
N-(2-((3-pyridinyl)methyl)-1-azabicyclo[2.2.2]oct-3-yl)-5-bromothiophene-2-carboxamide,
N-(2-((3-pyridinyl)methyl)-1-azabicyclo[2.2.2]oct-3-yl)-5-methylthiothiophene-2-carboxamide,
N-(2-((3-pyridinyl)methyl)-1-azabicyclo[2.2.2]oct-3-yl)-5-phenylthiothiophene-2-carboxamide,
N-(2-((3-pyridinyl)methyl)-1-azabicyclo[2.2.2]oct-3-yl)-5-methylthiophene-2-carboxamide,
N-(2-((3-pyridinyl)methyl)-1-azabicyclo[2.2.2]oct-3-yl)-3-methylthiophene-2-carboxamide,
N-(2-((3-pyridinyl)methyl)-1-azabicyclo[2.2.2]oct-3-yl)-3-bromothiophene-2-carboxamide,
N-(2-((3-pyridinyl)methyl)-1-azabicyclo[2.2.2]oct-3-yl)-3-chlorothiophene-2-carboxamide,
N-(2-((3-pyridinyl)methyl)-1-azabicyclo[2.2.2]oct-3-yl)-5-(2-pyridinyl)thiophene-2-carboxamide,
N-(2-((3-pyridinyl)methyl)-1-azabicyclo[2.2.2]oct-3-yl)-5-acetylthiophene-2-carboxamide,
N-(2-((3-pyridinyl)methyl)-1-azabicyclo[2.2.2]oct-3-yl)-3-ethoxythiophene-2-carboxamide,
N-(2-((3-pyridinyl)methyl)-1-azabicyclo[2.2.2]oct-3-yl)-3-methoxythiophene-2-carboxamide,
N-(2-((3-pyridinyl)methyl)-1-azabicyclo[2.2.2]oct-3-yl)-4-acetyl-3-methyl-5-methylthiothiophene-2-carboxamide,
N-(2-((3-pyridinyl)methyl)-1-azabicyclo[2.2.2]oct-3-yl)thiophene-3-carboxamide,
N-(2-((3-pyridinyl)methyl)-1-azabicyclo[2.2.2]oct-3-yl)-1-methylpyrrole-2-carboxamide,
N-(2-((3-pyridinyl)methyl)-1-azabicyclo[2.2.2]oct-3-yl)pyrrole-3-carboxamide,
N-(2-((3-pyridinyl)methyl)-1-azabicyclo[2.2.2]oct-3-yl)indole-2-carboxamide,
N-(2-((3-pyridinyl)methyl)-1-azabicyclo[2.2.2]oct-3-yl)indole-3-carboxamide,
N-(2-((3-pyridinyl)methyl)-1-azabicyclo[2.2.2]oct-3-yl)-1-methylindole-3-carboxamide,
N-(2-((3-pyridinyl)methyl)-1-azabicyclo[2.2.2]oct-3-yl)-1-benzylindole-3-carboxamide,
N-(2-((3-pyridinyl)methyl)-1-azabicyclo[2.2.2]oct-3-yl)-1H-benzimidazole-2-carboxamide,
N-(2-((3-pyridinyl)methyl)-1-azabicyclo[2.2.2]oct-3-yl)-1-isopropyl-2-trifluoromethyl-1H-benzimidazole-5-carboxamide,
N-(2-((3-pyridinyl)methyl)-1-azabicyclo[2.2.2]oct-3-yl)-1-isopropyl-1H-benzotriazole-5-carboxamide,
N-(2-((3-pyridinyl)methyl)-1-azabicyclo[2.2.2]oct-3-yl)benzo[b]thiophene-2-carboxamide,
N-(2-((3-pyridinyl)methyl)-1-azabicyclo[2.2.2]oct-3-yl)benzo[b]thiophene-3-carboxamide,
N-(2-((3-pyridinyl)methyl)-1-azabicyclo[2.2.2]oct-3-yl)benzofuran-2-carboxamide,
N-(2-((3-pyridinyl)methyl)-1-azabicyclo[2.2.2]oct-3-yl)benzofuran-3-carboxamide,
N-(2-((3-pyridinyl)methyl-1-azabicyclo[2.2.2]oct-3-yl)-3-methylbenzofuran-2-carboxamide,
N-(2-((3-pyridinyl)methyl-1-azabicyclo[2.2.2]oct-3-yl)-5-nitrobenzofuran-2-carboxamide,
N-(2-((3-pyridinyl)methyl-1-azabicyclo[2.2.2]oct-3-yl)-5-methoxybenzofuran-2-carboxamide,
N-(2-((3-pyridinyl)methyl-1-azabicyclo[2.2.2]oct-3-yl)-7-methoxybenzofuran-2-carboxamide,
N-(2-((3-pyridinyl)methyl-1-azabicyclo[2.2.2]oct-3-yl)-7-ethoxybenzofuran-2-carboxamide,
N-(2-((3-pyridinyl)methyl-1-azabicyclo[2.2.2]oct-3-yl)-3-methyl-5-chlorobenzofuran-2-carboxamide,
N-(2-((3-pyridinyl)methyl-1-azabicyclo[2.2.2]oct-3-yl)-6-bromobenzofuran-2-carboxamide,
N-(2-((3-pyridinyl)methyl-1-azabicyclo[2.2.2]oct-3-yl)-4-acetyl-7-methoxybenzofuran-2-carboxamide,
N-(2-((3-pyridinyl)methyl-1-azabicyclo[2.2.2]oct-3-yl)-2-methylbenzofuran-4-carboxamide,
N-(2-((3-pyridinyl)methyl-1-azabicyclo[2.2.2]oct-3-yl)naphtho[2,1-b]furan-2-carboxamide,
N-(2-((3-pyridinyl)methyl)-1-azabicyclo[2.2.2]oct-3-yl)naphthalene-1-carboxamide,
N-(2-((3-pyridinyl)methyl)-1-azabicyclo[2.2.2]oct-3-yl)naphthalene-2-carboxamide,
N-(2-((3-pyridinyl)methyl)-1-azabicyclo[2.2.2]oct-3-yl)-6-aminonaphthalene-2-carboxamide,
N-(2-((3-pyridinyl)methyl-1-azabicyclo[2.2.2]oct-3-yl)-3-methoxynaphthalene-2-carboxamide,
N-(2-((3-pyridinyl)methyl-1-azabicyclo[2.2.2]oct-3-yl)-6-methoxynaphthalene-2-carboxamide,
N-(2-((3-pyridinyl)methyl-1-azabicyclo[2.2.2]oct-3-yl)-1-hydroxynaphthalene-2-carboxamide,
N-(2-((3-pyridinyl)methyl-1-azabicyclo[2.2.2]oct-3-yl)-6-hydroxynaphthalene-2-carboxamide,
N-(2-((3-pyridinyl)methyl-1-azabicyclo[2.2.2]oct-3-yl)-6-acetoxynaphthalene-2-carboxamide,
N-(2-((3-pyridinyl)methyl)-1-azabicyclo[2.2.2]oct-3-yl)3-phenylprop-2-enamide,
N-(2-((3-pyridinyl)methyl)-1-azabicyclo[2.2.2]oct-3-yl)-3-(3-fluorophenyl)prop-2-enamide,
N-(2-((3-pyridinyl)methyl)-1-azabicyclo[2.2.2]oct-3-yl)-3-(4-methoxyphenyl)prop-2-enamide,
N-(2-((3-pyridinyl)methyl)-1-azabicyclo[2.2.2]oct-3-yl)-2-methyl-3-phenylprop-2-enamide,
N-(2-((3-pyridinyl)methyl)-1-azabicyclo[2.2.2]oct-3-yl)-3-(2-fluorophenyl)prop-2-enamide,
N-(2-((3-pyridinyl)methyl)-1-azabicyclo[2.2.2]oct-3-yl)-3-(3-methylphenyl)prop-2-enamide,
N-(2-((3-pyridinyl)methyl)-1-azabicyclo[2.2.2]oct-3-yl)-3-(4-fluorophenyl)prop-2-enamide,
N-(2-((3-pyridinyl)methyl)-1-azabicyclo[2.2.2]oct-3-yl)-3-(4-methylphenyl)prop-2-enamide,
N-(2-((3-pyridinyl)methyl)-1-azabicyclo[2.2.2]oct-3-yl)-3-(2-furyl)prop-2-enamide,
N-(2-((3-pyridinyl)methyl)-1-azabicyclo[2.2.2]oct-3-yl)-3-(2-methoxyphenyl)prop-2-enamide,
N-(2-((3-pyridinyl)methyl)-1-azabicyclo[2.2.2]oct-3-yl)-3-(3-bromophenyl)prop-2-enamide,
N-(2-((3-pyridinyl)methyl)-1-azabicyclo[2.2.2]oct-3-yl)-3-(3-methoxyphenyl)prop-2-enamide,
N-(2-((3-pyridinyl)methyl)-1-azabicyclo[2.2.2]oct-3-yl)-3-(3-hydroxyphenyl)prop-2-enamide,
N-(2-((3-pyridinyl)methyl)-1-azabicyclo[2.2.2]oct-3-yl)-3-(4-bromophenyl)prop-2-enamide,
N-(2-((3-pyridinyl)methyl)-1-azabicyclo[2.2.2]oct-3-yl)-3-(4-chlorophenyl)prop-2-enamide,
N-(2-((3-pyridinyl)methyl)-1-azabicyclo[2.2.2]oct-3-yl)-3-(4-hydroxyphenyl)prop-2-enamide,
N-(2-((3-pyridinyl)methyl)-1-azabicyclo[2.2.2]oct-3-yl)-3-(4-hydroxy-3-methoxyphenyl)prop-2-enamide,
N-(2-((3-pyridinyl)methyl)-1-azabicyclo[2.2.2]oct-3-yl)-3-(2-thienyl)prop-2-enamide,
N-(2-((3-pyridinyl)methyl)-1-azabicyclo[2.2.2]oct-3-yl)-3-(3-pyridinyl)prop-2-enamide,
N-(2-((3-pyridinyl)methyl)-1-azabicyclo[2.2.2]oct-3-yl)-3-(4-biphenyl)prop-2-enamide,
N-(2-((3-pyridinyl)methyl)-1-azabicyclo[2.2.2]oct-3-yl)-3-(1-naphthyl)prop-2-enamide,
N-(2-((3-pyridinyl)methyl)-1-azabicyclo[2.2.2]oct-3-yl)-3-(3-thienyl)prop-2-enamide,
N-(2-((3-pyridinyl)methyl)-1-azabicyclo[2.2.2]oct-3-yl)-3-(4-isopropylphenyl)prop-2-enamide,
N-(2-((3-pyridinyl)methyl)-1-azabicyclo[2.2.2]oct-3-yl)-3-methyl-3-phenylprop-2-enamide,
N-(2-((3-pyridinyl)methyl)-1-azabicyclo[2.2.2]oct-3-yl)-3-(3-furyl)prop-2-enamide,
N-(2-((3-pyridinyl)methyl)-1-azabicyclo[2.2.2]oct-3-yl)-2-ethyl-3-phenylprop-2-enamide,
N-(2-((3-pyridinyl)methyl)-1-azabicyclo[2.2.2]oct-3-yl)-3-(2-pyridinyl)prop-2-enamide, N-(2-((3-pyridinyl)methyl)-1-azabicyclo[2.2.2]oct-3-yl)-3-(3,4-dimethylthieno[2,3-b]thiophen-2-yl)prop-2-enamide,
N-(2-((3-pyridinyl)methyl)-1-azabicyclo[2.2.2]oct-3-yl)-3-(3-methylthien-2-yl)prop-2-enamide,
N-(2-((3-pyridinyl)methyl)-1-azabicyclo[2.2.2]oct-3-yl)-3-(2-naphthyl)prop-2-enamide, and
N-(2-((3-pyridinyl)methyl)-1-azabicyclo[2.2.2]oct-3-yl)-3-(4-methylthiophenyl)prop-2-enamide.

A second genus of α7 NNR selective ligands (see US Appln. No. 11/465,914, Pub. No. 2007 00197579 A1; also see published international application WO 2007/024814 A1; each of which is incorporated herein by reference in its entirety), useful according to the present invention, is represented by Formula 2.

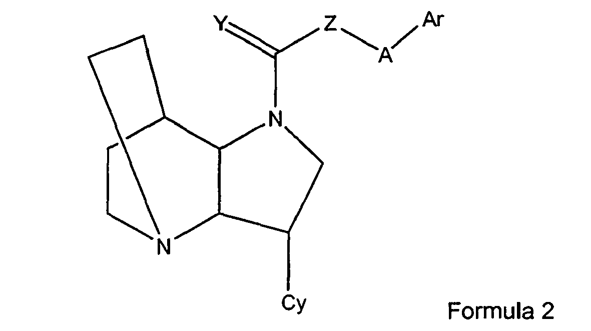

In Formula 2, Y is either oxygen or sulfur, and Z is either nitrogen (i.e., NR') or a covalent bond. A is either absent or a linker species selected from the group -CR' R"-, -CR' R"- CR' R"-, -CR'= CR'-, and -C₂-, wherein R' and R" are as hereinafter defined. Ar is an aryl group, either carbocyclic or heterocyclic, either monocyclic or fused polycyclic, unsubstituted or substituted; and Cy is a 5- or 6-membered heteroaromatic ring, unsubstituted or substituted. Thus, the invention includes compounds in which Ar is linked to the diazatricycle, at the nitrogen of the pyrrolidine ring, by a carbonyl group-containing functionality, forming an amide or a urea functionality. Ar may be bonded directly to the carbonyl group-containing functionality or may be linked to the carbonyl group-containing functionality through linker A. Furthermore, the invention includes compounds that contain a diazatricycle, containing a 1-azabicyclo[2.2.2]octane. As used in reference to Formula 2, a "carbonyl group-containing functionality" is a moiety of the formula -C(=Y)-Z-, where Y are Z are as defined herein.

In one embodiment, Cy is 3-pyridinyl or 5-pyrimidinyl, Y is oxygen, Z is a covalent bond and A is absent. In another embodiment, Cy is 3-pyridinyl or 5-pyrimidinyl, Y is oxygen, Z is nitrogen and A is absent. In a third embodiment, Cy is 3-pyridinyl or 5-pyrimidinyl, Y is oxygen, Z is a covalent bond, and A is a linker species. In a fourth embodiment, Cy is 3-pyridinyl or 5-pyrimidinyl, Y is oxygen, Z is nitrogen and A is a linker species.

The junction between the azacycle and the azabicycle can be characterized by any of the various relative and absolute stereochemical configurations at the junction sites (e.g., *cis* or *trans,* R or S). The compounds have one or more asymmetric carbons and can therefore exist in the form of racemic mixtures, enantiomers and diastereomers. In addition, some of the compounds exist as E and Z isomers about a carbon-carbon double bond. All these individual isomeric compounds and their mixtures are also intended to be within the scope of the present invention.

As used in Formula 2, Ar ("aryl") includes both carbocyclic and heterocyclic aromatic rings, both monocyclic and fused polycyclic, where the aromatic rings can be 5- or 6-membered rings. Representative monocyclic aryl groups include, but are not limited to, phenyl, furanyl, pyrrolyl, thienyl, pyridinyl, pyrimidinyl, oxazolyl, isoxazolyl, pyrazolyl, imidazolyl, thiazolyl, isothiazolyl and the like. Fused polycyclic aryl groups are those aromatic groups that include a 5- or 6-membered aromatic or heteroaromatic ring as one or more rings in a fused ring system. Representative fused polycyclic aryl groups include naphthalene, anthracene, indolizine, indole, isoindole, benzofuran, benzothiophene, indazole, benzimidazole, benzthiazole, purine, quinoline, isoquinoline, cinnoline, phthalazine, quinazoline, quinoxaline, 1,8-naphthyridine, pteridine, carbazole, acridine, phenazine, phenothiazine, phenoxazine, and azulene.

As used in Formula 2, "Cy" groups are 5- and 6-membered ring heteroaromatic groups. Representative Cy groups include pyridinyl, pyrimidinyl, furanyl, pyrrolyl, thienyl, oxazolyl, isoxazolyl, pyrazolyl, imidazolyl, thiazolyl, isothiazolyl and the like, where pyridinyl is preferred.
Individually, Ar and Cy can be unsubstituted or can be substituted with 1, 2 or 3 substituents, such as alkyl, alkenyl, heterocyclyl, cycloalkyl, aryl, substituted aryl, arylalkyl, substituted arylalkyl, halo (e.g., F, Cl, Br, or I), -OR', -NR'R", -CF₃, -CN, -NO₂, -C₂R', -SR', -N₃, - C(=O)NR'R", -NR'C(=O) R", -C(=O)R', -C(=O)OR', -OC(=O)R', -O(CR'R")ᵣC(=O)R', - O(CR'R")ᵣNR"C(=O)R', -O(CR'R")ᵣNR"SO₂R', -OC(=O)NR'R", -NR'C(=O)O R", -SO₂R', - SO₂NR'R", and -NR'SO₂R", where R' and R" are individually hydrogen, C₁-C₈ alkyl (e.g., straight chain or branched alkyl, preferably C₁-C₅, such as methyl, ethyl, or isopropyl), cycloalkyl (e.g., C₃₋₈ cyclic alkyl), heterocyclyl, aryl, or arylalkyl (such as benzyl), and r is an integer from 1 to 6. R' and R" can also combine to form a cyclic functionality.

Compounds of Formula 2 form acid addition salts which are useful according to the present invention. Examples of suitable pharmaceutically acceptable salts include inorganic acid addition salts such as chloride, bromide, sulfate, phosphate, and nitrate; organic acid addition salts such as acetate, galactarate, propionate, succinate, lactate, glycolate, malate, tartrate, citrate, maleate, fumarate, methanesulfonate, p-toluenesulfonate, and ascorbate; salts with acidic amino acid such as aspartate and glutamate. The salts may be in some cases hydrates or ethanol solvates.

Representative compounds of Formula 2 include:
5-benzoyl-3-pyridin-3-yl-1,5-diazatricyclo[5.2.2.0<2,6>]undecane,
5-(2-fluorobenzoyl)-3-pyridin-3-yl-1,5-diazatricyclo[5.2.2.0<2,6>]undecane,
5-(3-fluorobenzoyl)-3-pyridin-3-yl-1,5-diazatricyclo[5.2.2.0<2,6>]undecane,
5-(4-fluorobenzoyl)-3-pyridin-3-yl-1,5-diazatricyclo[5.2.2.0<2,6>]undecane,
5-(2-chlorobenzoyl)-3-pyridin-3-yl-1,5-diazatricyclo[5.2.2.0<2,6>]undecane,
5-(3-chlorobenzoyl)-3-pyridin-3-yl-1,5-diazatricyclo[5.2.2.0<2,6>]undecane,
5-(4-chlorobenzoyl)-3-pyridin-3-yl-1,5-diazatricyclo[5.2.2.0<2,6>]undecane,
5-(2-bromobenzoyl)-3-pyridin-3-yl-1,5-diazatricyclo[5.2.2.0<2,6>]undecane,
5-(3-bromobenzoyl)-3-pyridin-3-yl-1,5-diazatricyclo[5.2.2.0<2,6>]undecane,
5-(4-bromobenzoyl)-3-pyridin-3-yl-1,5-diazatricyclo[5.2.2.0<2,6>]undecane,
5-(2-iodobenzoyl)-3-pyridin-3-yl-1,5-diazatricyclo[5.2.2.0<2,6>]undecane,
5-(3-iodobenzoyl)-3-pyridin-3-yl-1,5-diazatricyclo[5.2.2.0<2,6>]undecane,
5-(4-iodobenzoyl)-3-pyridin-3-yl-1,5-diazatricyclo[5.2.2.0<2,6>]undecane,
5-(2-methylbenzoyl)-3-pyridin-3-yl-1,5-diazatricyclo[5.2.2.0<2,6>]undecane,
5-(3-methylbenzoyl)-3-pyridin-3-yl-1,5-diazatricyclo[5.2.2.0<2,6>]undecane,
5-(4-methylbenzoyl)-3-pyridin-3-yl-1,5-diazatricyclo[5.2.2.0<2,6>]undecane,
5-(2-methoxybenzoyl)-3-pyridin-3-yl-1,5-diazatricyclo[5.2.2.0<2,6>]undecane,
5-(3-methoxybenzoyl)-3-pyridin-3-yl-1,5-diazatricyclo[5.2.2.0<2,6>]undecane,
5-(4-methoxybenzoyl)-3-pyridin-3-yl-1,5-diazatricyclo[5.2.2.0<2,6>]undecane,
5-(2-methylthiobenzoyl)-3-pyridin-3-yl-1,5-diazatricyclo[5.2.2.0<2,6>]undecane,
5-(3-methylthiobenzoyl)-3-pyridin-3-yl-1,5-diazatricyclo[5.2.2.0<2,6>]undecane,
5-(4-methylthiobenzoyl)-3-pyridin-3-yl-1,5-diazatricyclo[5.2.2.0<2,6>]undecane,
5-(2-phenylbenzoyl)-3-pyridin-3-yl-1,5-diazatricyclo[5.2.2.0<2,6>]undecane,
5-(3-phenylbenzoyl)-3-pyridin-3-yl-1,5-diazatricyclo[5.2.2.0<2,6>]undecane,
5-(4-phenylbenzoyl)-3-pyridin-3-yl-1,5-diazatricyclo[5.2.2.0<2,6>]undecane,
5-(2-phenoxybenzoyl)-3-pyridin-3-yl-1,5-diazatricyclo[5.2.2.0<2,6>]undecane,
5-(3-phenoxybenzoyl)-3-pyridin-3-yl-1,5-diazatricyclo[5.2.2.0<2,6>]undecane,
5-(4-phenoxybenzoyl)-3-pyridin-3-yl-1,5-diazatricyclo[5.2.2.0<2,6>]undecane,
5-(2-phenylthiobenzoyl)-3-pyridin-3-yl-1,5-diazatricyclo[5.2.2.0<2,6>]undecane,
5-(3-phenylthiobenzoyl)-3-pyridin-3-yl-1,5-diazatricyclo[5.2.2.0<2,6>]undecane,
5-(4-phenylthiobenzoyl)-3-pyridin-3-yl-1,5-diazatricyclo[5.2.2.0<2,6>]undecane,
5-(2-cyanobenzoyl)-3-pyridin-3-yl-1,5-diazatricyclo[5.2.2.0<2,6>]undecane,
5-(3-cyanobenzoyl)-3-pyridin-3-yl-1,5-diazatricyclo[5.2.2.0<2,6>]undecane,
5-(4-cyanobenzoyl)-3-pyridin-3-yl-1,5-diazatricyclo[5.2.2.0<2,6>]undecane,
5-(2-trifluoromethylbenzoyl)-3-pyridin-3-yl-1,5-diazatricyclo[5.2.2.0<2,6>]undecane,
5-(3-trifluoromethylbenzoyl)-3-pyridin-3-yl-1,5-diazatricyclo[5.2.2.0<2,6>]undecane,
5-(4-trifluoromethylbenzoyl)-3-pyridin-3-yl-1,5-diazatricyclo[5.2.2.0<2,6>]undecane,
5-(2-dimethylaminobenzoyl)-3-pyridin-3-yl-1,5-diazatricyclo[5.2.2.0<2,6>]undecane,
5-(3-dimethylaminobenzoyl)-3-pyridin-3-yl-1,5-diazatricyclo[5.2.2.0<2,6>]undecane,
5-(4-dimethylaminobenzoyl)-3-pyridin-3-yl-1,5-diazatricyclo[5.2.2.0<2,6>]undecane,
5-(2-ethynylbenzoyl)-3-pyridin-3-yl-1, 5-d iazatricyclo[5.2.2.0<2,6>]undecane,
5-(3-ethynylbenzoyl)-3-pyridin-3-yl-1,5-diazatricyclo[5.2.2.0<2,6>]undecane,
5-(4-ethynylbenzoyl)-3-pyridin-3-yl-1,5-diazatricyclo[5.2.2.0<2,6>]undecane,
5-(3,4-dichlorobenzoyl)-3-pyridin-3-yl-1,5-diazatricyclo[5.2.2.0<2,6>]undecane,
5-(2,4-dimethoxybenzoyl)-3-pyridin-3-yl-1,5-diazatricyclo[5.2.2.0<2,6>]undecane,
5-(3,4,5-trimethoxybenzoyl)-3-pyridin-3-yl-1,5-diazatricyclo[5.2.2.0<2,6>]undecane,
5-(naphth-1-ylcarbonyl)-3-pyridin-3-yl-1,5-diazatricyclo[5.2.2.0<2,6>]undecane,
5-(naphth-2-ylcarbonyl)-3-pyridin-3-yl-1,5-diazatricyclo[5.2.2.0<2,6>]undecane,
5-(thien-2-ylcarbonyl)-3-pyridin-3-yl-1,5-diazatricyclo[5.2.2.0<2,6>]undecane,
5-(thien-3-ylcarbonyl)-3-pyridin-3-yl-1,5-diazatricyclo[5.2.2.0<2,6>]undecane,
5-(furan-2-ylcarbonyl)-3-pyridin-3-yl-1,5-diazatricyclo[5.2.2.0<2,6>]undecane, 5-(benzothien-2-ylcarbonyl)-3-pyridin-3-yl-1,5-diazatricyclo[5.2.2.0<2,6>]undecane,
5-(benzofuran-2-ylcarbonyl)-3-pyridin-3-yl-1,5-diazatricyclo[5.2.2.0<2,6>]undecane,
5-(7-methoxybenzofuran-2-ylcarbonyl)-3-pyridin-3-yl-1,5-diazatricyclo[5.2.2.0<2,6>]undecane, and
5-(1H-indol-3-ylcarbonyl)-3-pyridin-3-yl-1,5-diazatricyclo[5.2.2.0<2,6>]undecane.

Other compounds representative of Formula 2 include:
5-(phenylacetyl)-3-pyridin-3-yl-1,5-diazatricyclo[5.2.2.0<2,6>]undecane,
5-(diphenylacetyl)-3-pyridin-3-yl-1,5-diazatricyclo[5.2.2.0<2,6>]undecane,
5-(2-phenylpropanoyl)-3-pyridin-3-yl-1,5-diazatricyclo[5.2.2.0<2,6>]undecane, and
5-(3-phenylprop-2-enoyl)-3-pyridin-3-yl-1,5-diazatricyclo[5.2.2.0<2,6>]undecane.

Other compounds representative of Formula 2 include:
5-N-phenylcarbamoyl-3-pyridin-3-yl-1,5-diazatricyclo[5.2.2.0<2,6>]undecane, 5-(N-(2-fluorophenyl)carbamoyl)-3-pyridin-3-yl-1,5-diazatricyclo[5.2.2.0<2,6>]undecane,
5-(N-(3-fluorophenyl)carbamoyl)-3-pyridin-3-yl-1,5-diazatricyclo[5.2.2.0<2,6>]undecane,
5-(N-(4-fluorophenyl)carbamoyl)-3-pyridin-3-yl-1,5-diazatricyclo[5.2.2.0<2,6>]undecane,
5-(N-(2-chlorophenyl)carbamoyl)-3-pyridin-3-yl-1,5-diazatricyclo[5.2.2.0<2,6>]undecane,
5-(N-(3-chlorophenyl)carbamoyl)-3-pyridin-3-yl-1,5-diazatricyclo[5.2.2.0<2,6>]undecane,
5-(N-(4-chlorophenyl)carbamoyl)-3-pyridin-3-yl-1,5-diazatricyclo[5.2.2.0<2,6>]undecane,
5-(N-(2-bromophenyl)carbamoyl)-3-pyridin-3-yl-1,5-diazatricyclo[5.2.2.0<2,6>]undecane,
5-(N-(3-bromophenyl)carbamoyl)-3-pyridin-3-yl-1,5-diazatricyclo[5.2.2.0<2,6>]undecane,
5-(N-(4-bromophenyl)carbamoyl)-3-pyridin-3-yl-1 ,5-diazatricydo[5.2.2.0<2,6>]undecane,
5-(N-(2-iodophenyl)carbamoyl)-3-pyridin-3-yl-1,5-diazatricyclo[5.2.2.0<2,6>]undecane,
5-(N-(3-iodophenyl)carbamoyl)-3-pyridin-3-yl-1,5-diazatricyclo[5.2.2.0<2,6>]undecane,
5-(N-(4-iodophenyl)carbamoyl)-3-pyridin-3-yl-1,5-diazatricyclo[5.2.2.0<2,6>]undecane,
5-(N-(2-methylphenyl)carbamoyl)-3-pyridin-3-yl-1,5-diazatricyclo[5.2.2.0<2,6>]undecane,
5-(N-(3-methylphenyl)carbamoyl)-3-pyridin-3-yl-1,5-diazatricyclo[5.2.2.0<2,6>]undecane,
5-(N-(4-methylphenyl)carbamoyl)-3-pyridin-3-yl-1,5-diazatricyclo[5.2.2.0<2,6>]undecane,
5-(N-(2-methoxyphenyl)carbamoyl)-3-pyridin-3-yl-1,5-diazatricyclo[5.2.2.0<2,6>]undecane,
5-(N-(3-methoxyphenyl)carbamoyl)-3-pyridin-3-yl-1,5-diazatricyclo[5.2.2.0<2,6>]undecane,
5-(N-(4-methoxyphenyl)carbamoyl)-3-pyridin-3-yl-1,5-diazatricyclo[5.2.2.0<2,6>]undecane,
5-(N-(2-methylthiophenyl)carbamoyl)-3-pyridin-3-yl-1,5-diazatricyclo[5.2.2.0<2,6>] undecane,
5-(N-(3-methylthiophenyl)carbamoyl)-3-pyridin-3-yl-1,5-diazatricyclo[5.2.2.0<2,6>]undecane,
5-(N-(4-methylthiophenyl)carbamoyl)-3-pyridin-3-yl-1,5-diazatricyclo[5.2.2.0<2,6>]undecane,
5-(N-(2-phenylphenyl)carbamoyl)-3-pyridin-3-yl-1,5-diazatricyclo[5.2.2.0<2,6>]undecane,
5-(N-(3-phenylphenyl)carbamoyl)-3-pyridin-3-yl-1,5-diazatricyclo[5.2.2.0<2,6>]undecane,
5-(N-(4-phenylphenyl)carbamoyl)-3-pyridin-3-yl-1,5-diazatricyclo[5.2.2.0<2,6>]undecane,
5-(N-(2-phenoxyphenyl)carbamoyl)-3-pyridin-3-yl-1,5-diazatricyclo[5.2.2.0<2,6>]undecane,
5-(N-(3-phenoxyphenyl)carbamoyl)-3-pyridin-3-yl-1,5-diazatricyclo[5.2.2.0<2,6>]undecane,
5-(N-(4-phenoxyphenyl)carbamoyl)-3-pyridin-3-yl-1,5-diazatricyclo[5.2.2.0<2,6>]undecane,
5-(N-(2-phenylthiophenyl)carbamoyl)-3-pyridin-3-yl-1,5-diazatricyclo[5.2.2.0<2,6>]undecane,
5-(N-(3-phenylthiophenyl)carbamoyl)-3-pyridin-3-yl-1,5-diazatricyclo[5.2.2.0<2,6>]undecane,
5-(N-(4-phenylthiophenyl)carbamoyl)-3-pyridin-3-yl-1,5-diazatricyclo[5.2.2.0<2,6>]undecane,
5-(N-(2-cyanophenyl)carbamoyl)-3-pyridin-3-yl-1,5-diazatricyclo[5.2.2.0<2,6>]undecane,
5-(N-(3-cyanophenyl)carbamoyl)-3-pyridin-3-yl-1,5-diazatricyclo[5.2.2.0<2,6>]undecane,
5-(N-(4-cyanophenyl)carbamoyl)-3-pyridin-3-yl-1,5-diazatricyclo[5.2.2.0<2,6>]undecane,
5-(N-(2-trifluoromethylphenyl)carbamoyl)-3-pyridin-3-yl-1,5-diazatricyclo[5.2.2.0<2,6>]undecane,
5-(N-(3-trifluoromethylphenyl)carbamoyl)-3-pyridin-3-yl-1,5-diazatricyclo[5.2.2.0<2,6>]undecane,
5-(N-(4-trifluoromethylphenyl)carbamoyl)-3-pyridin-3-yl-1,5-diazatricyclo[5.2.2.0<2,6>]undecane,
5-(N-(2-dimethylaminophenyl)carbamoyl)-3-pyridin-3-yl-1,5-diazatricyclo[5.2.2.0<2,6>]undecane,
5-(N-(3-dimethylaminophenyl)carbamoyl)-3-pyridin-3-yl-1,5-diazatricyclo[5.2.2.0<2,6>]undecane,
5-(N-(4-dimethylaminophenyl)carbamoyl)-3-pyridin-3-yl-1,5-diazatricyclo[5.2.2.0<2,6>]undecane,
5-(N-(2-ethynylphenyl)carbamoyl)-3-pyridin-3-yl-1,5-diazatricyclo[5.2.2.0<2,6>]undecane,
5-(N-(3-ethynylphenyl)carbamoyl)-3-pyridin-3-yl-1,5-diazatricyclo[5.2.2.0<2,6>]undecane,
5-(N-(4-ethynylphenyl)carbamoyl)-3-pyridin-3-yl-1,5-diazatricyclo[5.2.2.0<2,6>]undecane,
5-(N-(3,4-dichlorophenyl)carbamoyl)-3-pyridin-3-yl-1,5-diazatricyclo[5.2.2.0<2,6>]undecane,
5-(N-(2,4-dimethoxyphenyl)carbamoyl)-3-pyridin-3-yl-1,5-diazatricyclo[5.2.2.0<2,6>]undecane,
5-(N-(3,4,5-trimethoxyphenyl)carbamoyl)-3-pyridin-3-yl-1,5-diazatricyclo[5.2.2.0<2,6>]undecane,
5-(N-(1-naphthyl)carbamoyl)-3-pyridin-3-yl-1,5-diazatricyclo[5.2.2.0<2,6>]undecane, and
5-(N-(2-naphthyl)carbamoyl)-3-pyridin-3-yl-1,5-diazatricyclo[5.2.2.0<2,6>]undecane.
   Other compounds representative of Formula 2 include:
5-(N-benzylcarbamoyl)-3-pyridin-3-yl-1,5-diazatricyclo[5.2.2.0<2,6>]undecane,
5-(N-(4-bromobenzyl)carbamoyl)-3-pyridin-3-yl-1,5-diazatricyclo[5.2.2.0<2,6>]undecane,
5-(N-(4-methoxybenzyl)carbamoyl)-3-pyridin-3-yl-1,5-diazatricyclo[5.2.2.0<2,6>]undecane,
5-(N-(1-phenylethyl)carbamoyl)-3-pyridin-3-yl-1,5-diazatricyclo[5.2.2.0<2,6>]undecane, and 5-(N-(diphenylmethyl)carbamoyl)-3-pyridin-3-yl-1,5-diazatricyclo[5.2.2.0<2,6>]undecane.

In each of these compounds, a 3-pyridin-3-yl-1,5-diazatricyclo[5.2.2.0<2,6>]undecane moiety has the structure, with a partial numbering scheme provided, shown below:

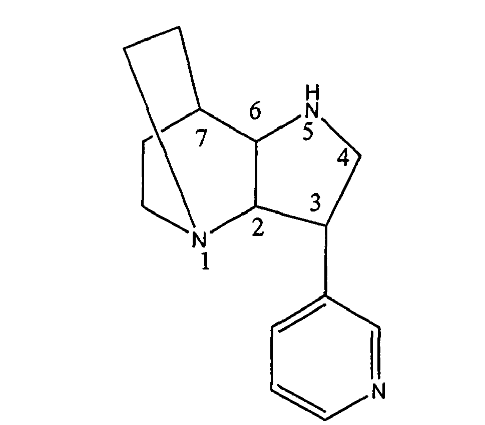

The nitrogen at the position indicated above as the 5-position is the nitrogen involved in the formation of the amides, thioamides, ureas and thioureas described herein.

Compounds useful according to the present invention also include compounds of Formula 3:

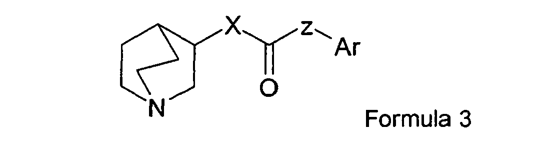

In Formula 3, X is either oxygen or nitrogen (i.e., NR'), and Z is either nitrogen (i.e., NR'), -CR'=CR'- or a covalent bond, provided that X must be nitrogen when Z is -CR'=CR'- or a covalent bond, and further provided that X and Z are not simultaneously nitrogen. Ar is an aryl group, either carbocyclic or heterocyclic, either monocyclic or fused polycyclic, unsubstituted or substituted; R' is hydrogen, C₁-C₈ alkyl (e.g., straight chain or branched alkyl, preferably C₁-C₅, such as methyl, ethyl, or isopropyl), aryl, or arylalkyl (such as benzyl).

Compounds in which X is oxygen and Z is nitrogen are disclosed as α7 selective ligands in, for instance, PCT WO 97/30998 and US Patent 6,054,464, each of which is incorporated herein in its entirety.

Compounds in which X is nitrogen and Z is covalent bond are disclosed as α7 selective ligands in, for instance, PCTs WO 02/16355, WO 02/16356, WO 02/16358, WO 04/029050, WO 04/039366, WO 04/052461, WO 07/038367, and in US Patent 6,486,172, US Patent 6,500,840, US Patent 6,599,916, US Patent 7,001,914, US Patent 7,067,515, and US Patent 7,176,198, each of which is herein incorporated herein in its entirety.

Compounds in which X is nitrogen and Z is -CR'=CR'- are disclosed as α7 selective ligands in, for instance, PCT WO 01/036417 and US Patent 6,683,090, each of which is incorporated herein in its entirety.

Particular embodiments according to the general Formula 3 include the following:
N-((3R)-1-azabicyclo[2.2.2]oct-3-yl)-5-phenylthiophene-2-carboxamide;
N-((3R)-1-azabicyclo[2.2.2]oct-3-yl)-2-phenyl-1,3-thiazole-5-carboxamide;
N-((3R)-1-azabicyclo[2.2.2]oct-3-yl)-5-phenyl-1,3-oxazole-2-carboxamide;
N-((3R)-1-azabicyclo[2.2.2]oct-3-yl)-5-phenyl-1,3,4-oxadiazole-2-carboxamide;
N-[(3R)-1-azabicyclo[2.2.2]oct-3-y-l]-4-(4-hydroxyphenoxy)benzamide;
N-[(3R)-1-azabicyclo[2.2.2]oct-3-yl]-4-(-4-acetamidophenoxy)benzamide;
N-[(3R)-1-azabicyclo[2.2.2]oct-3-yl]-4-phenoxybenzamide;
N-[(3R)-1-azabicyclo[2.2.2]oct-3-yl]-4-benzylbenzamide;
N-[(3R)-1-azabicyclo[2.2.2]oct-3-yl]-4-(phenylsulfanyl)benzamide;
N-[(3R)-1-azabicyclo[2.2.2]oct-3-yl]-3-phenoxybenzamide;
N-[(3R)-1-azabicyclo[2.2.2]oct-3-yl]-4-benzoylbenzamide;
N-[(3R)-1-azabicyclo[2.2.2]oct-3-yl]-4-(4-fluorophenoxy)benzamide;
N-[(3R)-1-azabicyclo[2.2.2]oct-3-yl]-4-(2-fluorophenoxy)benzamide;
N-[(3R)-1-azabicyclo[2.2.2]oct-3-yl]-4-(3-fluorophenoxy)benzamide;
N-[(3R)-1-azabicyclo[2.2.2]oct-3-yl]-4-(2-chlorophenoxy)benzamide;
N-[(3R)-1-azabicyclo[2.2.2]oct-3-yl]-4-(3-chlorophenoxy)benzamide;
N-[(3R)-1-azabicyclo[2.2.2]oct-3-yl]-4-(4-chlorophenoxy)benzamide;
N-[(3R)-1-azabicyclo[2.2.2]oct-3-yl]-4-(2-methoxyphenoxy)benzamide;
N-[(3R)-1-azabicyclo[2.2.2]oct-3-yl]-4-(3-methoxyphenoxy)benzamide;
N-[(3R)-1-azabicyclo[2.2.2]oct-3-yl]-4-(4-methoxyphenoxy)benzamide;
N-[(3R)-1-azabicyclo[2.2.2]oct-3-yl]-4-(3-chlorophenylsulfanyl)benzamide;
N-[(3R)-1-azabicyclo[2.2.2]oct-3-yl]-4-(4-methoxyphenoxy)benzamide;
N-[(3R)-1-azabicyclo[2.2.2]oct-3-yl]-4-(3-chlorophenylsulfanyl)benzamide;
N-[(3R)-1-azabicyclo[2.2.2]oct-3-yl]-4-(4-chlorophenylsulfanyl)benzamide;
N-[(3R)-1-azabicyclo[2.2.2]oct-3-yl]-4-(3-methoxyphenylsulfanyl)-benzamide;
N-[(3R)-1-azabicyclo[2.2.2]oct-3-yl]-4(2-methoxyphenylsulfanyl)-benzamide;
N-(2-methyl-1-azabicyclo[2.2.2]oct-3-yl)4-phenoxybenzamide;
N-((3R)-1-azabicyclo[2.2.2]oct-3-yl)-4-(pyridin-3-yloxy)benzamide;
N-phenylcarbamic acid 1-azabicyclo[2.2.2]octan-3-yl ester;
N-(4-bromophenyl)carbamic acid 1-azabicyclo[2.2.2]octan-3-yl ester;
N-(4-methylphenyl)carbamic acid 1-azabicyclo[2.2.2]octan-3-yl ester;
N-(4-methoxyphenyl)carbamic acid 1-azabicyclo[2.2.2]octan-3-yl ester;
N-(3,4-dichlorophenyl)carbamic acid 1-azabicyclo[2.2.2]octan-3-yl ester;
N-(4-cyanophenyl)carbamic acid 1-azabicyclo[2.2.2]octan-3-yl ester;
N-phenylcarbamic acid 1-azabicyclo[2.2.1]heptan-3-yl ester;
N-(3-methoxyphenyl)carbamic acid 1-azabicyclo[2.2.2]octan-3-yl ester;
N-phenylthiocarbamic acid 1-azabicyclo[2.2.2]octan-3-yl ester;
N-(2-pyridyl)carbamic acid 1-azabicyclo[2.2.2]octan-3-yl ester;
N-(1-naphthyl)carbamic acid 1-azabicyclo[2.2.2]octan-3-yl ester;
N-phenylcarbamic acid (3R)-1-azabicyclo[2.2.2]octan-3-yl ester;
N-phenylcarbamic acid (3S)-1-azabicyclo[2.2.2]octan-3-yl ester;
N-(4-pyridyl)carbamic acid 1-azabicyclo[2.2.2]octan-3-yl ester;
N-(m-biphenyl)carbamic acid 1-azabicyclo[2.2.2]octan-3-yl ester;
N-(3-quinolinyl)carbamic acid 1-azabicyclo[2.2.2]octan-3-yl ester;
N-(I-azabicyclo[2.2.2]oct-3-yl)(E-3-phenylpropenamide);
N-(I-azabicyclo[2.2.2]oct-3-yl)(3-phenylpropenamide);
   and pharmaceutically acceptable salts thereof.

Compounds useful according to the present invention also include compounds of Formula 4:

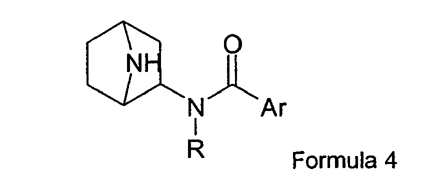

In Formula 4, Ar is an aryl group, either carbocyclic or heterocyclic, either monocyclic or fused polycyclic, unsubstituted or substituted; R is hydrogen, C₁-C₈ alkyl (e.g., straight chain or branched alkyl, preferably C₁-C₅, such as methyl, ethyl, or isopropyl), aryl, or arylalkyl (such as benzyl).

Such compounds are disclosed as α7 selective ligands in, for instance, PCTs WO 03/018585, WO 03/018586, WO 03/022856, WO 03/070732, WO 03/072578, WO 04/039815 and WO 04/052348, and US Patent 6,562,816, each of which is incorporated herein in its entirety.

Particular embodiments according to the general Formula 4 include the following:
N-(7-azabicyclo[2.2.1]hept-2-yl)-5-phenylthiophene-2-carbozamide;
N-(7-azabicyclo[2.2.1]hept-2-yl)-5-(2-pyridinyl)thiophene-2-carbozamide;
N-(7-azabicyclo[2.2.1]hept-2-yl)-5-phenylfuran-2-carbozamide;
   and pharmaceutically acceptable salts thereof.

Compounds useful according to the present invention also include compounds of Formula 5:

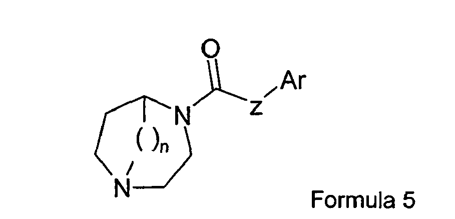

In Formula 5, n is 1 or 2; Ar is an aryl group, either carbocyclic or heterocyclic, either monocyclic or fused polycyclic, unsubstituted or substituted; and Z is oxygen, -CC-, - CH=CH- or a covalent bond.

Such compounds are disclosed as α7 selective ligands in, for instance, PCTs WO 00/058311, WO 04/016616, WO 04/016617, 04/061510, WO 04/061511 and WO 04/ 076453, each of which is incorporated herein in its entirety.

Particular embodiments according to the general Formula 5 include the following:
(1,4-diazabicyclo[3.2.2]non-4-yl)(4-methoxyphenyl)methanone;
(1,4-diazabicyclo[3.2.2]non-4-yl)(5-chlorofuran-2-yl)methanone;
(1,4-diazabicyclo[3.2.2]non-4-yl)(5-bromothiophen-2-yl)methanone;
(1,4-diazabicyclo[3.2.2]non-4-yl)(4-phenoxyphenyl)methanone;
(1,4-diazabicyclo[3.2.2]non-4-yl)(5-phenylfuran-2-yl)methanone;
(1,4-diazabicyclo[3.2.2]non-4-yl)(5-(3-pyridinyl)thiophen-2-yl)methanone;
1-(1,4-diazabicyclo[3.2.2]non-4-yl)-3-phenylpropenone;
   and pharmaceutically acceptable salts thereof.

Compounds useful according to the present invention also include compounds of Formula 6:

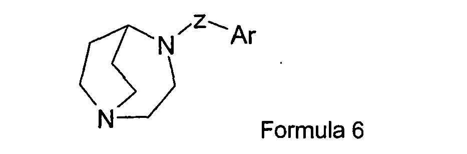

In Formula 6, Ar is a fused polycyclic, heterocyclic aryl group, unsubstituted or substituted; and Z is -CH₂- or a covalent bond.

Such compounds are disclosed as α7 selective ligands in, for instance, PCTs WO 03/119837 and WO 05/111038 and US Patent 6,881,734, each of which is herein incorporated by reference in its entirety.

Particular embodiments according to the general Formula 6 include the following:
4-benzoxazol-2-yl-1,4-diazabicyclo[3.2.2.]nonane;
4-benzothiazol-2-yl-1,4-diazabicyclo[3.2.2.]nonane;
4-benzoxazol-2-yl-1,4-diazabicyclo[3.2.2.]nonane;
4-oxazolo[5,4-b]pyridine-2-yl-1,4-diazabicyclo[3.2.2.]nonane;
4-(1 H-benzimidazol-2-yl-1,4-diazabicyclo[3.2.2.]nonane;
   and pharmaceutically acceptable salts thereof.

Compounds useful according to the present invention also include compounds of Formula 7:

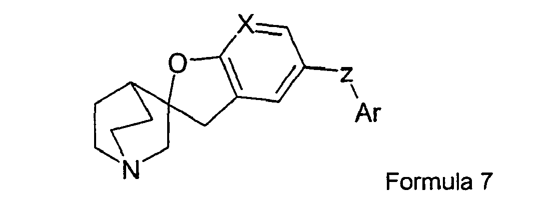

In Formula 7, Ar is an aryl group, either carbocyclic or heterocyclic, either monocyclic or fused polycyclic, unsubstituted or substituted; X is either CH or N; Z is either oxygen, nitrogen (NR) or a covalent bond; and R is H or alkyl. Optionally, "Z-Ar" is absent from Formula 7.

Such compounds are disclosed as α7 selective ligands in, for instance, PCTs WO 00/042044, WO 02/096912, WO 03/087102, WO 03/087103, WO 03/087104, WO 05/030778, WO 05/042538 and WO 05/066168, and US Patent 6,110,914, US Patent 6,369,224, US Patent 6,569,865, US Patent 6,703,502, US Patent 6,706,878, US Patent 6,995,167, US Patent 7,186,836 and US Patent 7,196,096, each of which is incorporated herein by reference in its entirety.

Particular embodiments according to the general Formula 7 include the following:
spiro[1-azabicyclo[2.2.2]octane-3,2'-(3'H)-furo[2,3-b]pyridine];
5'-phenylspiro[1-azabicyclo[2.2.2]octane-3,2'-(3'H)-furo[2,3-b]pyridine];
5'-(3-furanyl)spiro[1-azabicyclo[2.2.2]octane-3,2'-(3'H)-furo[2,3-b]pyridine];
5'-(2-thienyl)spiro[1-azabicyclo[2.2.2]octane-3,2'-(3'H)-furo[2,3-b]pyridine];
5'-(N-phenyl-N-methylamino)spiro[1-azabicyclo[2.2.2]octane-3,2'-(3'H)-furo[2,3-b]pyridine];
5'-(N-3-pyridinyl-N-methylamino)spiro[1-azabicyclo[2.2.2]octane-3,2'-(3'H)-furo[2,3-b]pyridine];
5'-(2-benzofuranyl)spiro[1-azabicyclo[2.2.2]octane-3,2'-(3'H)-furo[2,3- b]pyridine];
5'-(2-benzothiazolyl)spiro[1-azabicyclo[2.2.2]octane-3,2'-(3'H)-furo[2,3- b]pyridine];
5'-(3-pyridinyl)spiro[1-azabicyclo[2.2.2]octane-3,2'-(3'H)-furo[2,3-b]pyridine];
   and pharmaceutically acceptable salts thereof.

Compounds useful according to the present invention also include compounds of Formula 8:

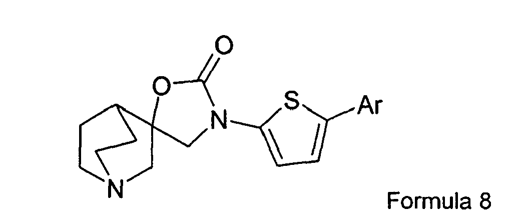

In Formula 8, Ar is an aryl group, either carbocyclic or heterocyclic, either unsubstituted or substituted.

Such compounds are disclosed as α7 selective ligands in, for instance, PCTs WO 05/005435 and WO 06/065209, each of which is herein incorporated by reference in its entirety. Particular embodiments according to the general Formula 8 include the following:
3'-(5-phenylthiophen-2-yl)spiro[1-azabicyclo[2.2.2]octan-3,5'-oxazolidin]-2'-one;
3'-(5-(3-pyridinyl)thiophen-2-yl)spiro[1-azabicyclo[2.2.2]octan-3,5'-oxazolidin]-2'-one;
   and pharmaceutically acceptable salts thereof.

Compounds useful according to the present invention also include compounds of Formula 9:

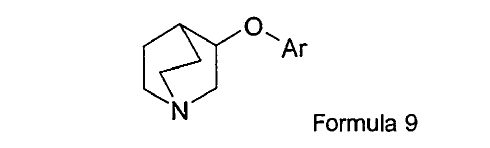

In Formula 9, Ar is an aryl group, either carbocyclic or heterocyclic, either monocyclic or fused polycyclic, unsubstituted or substituted (preferably by aryl or aryloxy substituents).

Such compounds are disclosed as α7 selective ligands in, for instance, PCTs WO 04/016608, WO 05/066166, WO 05/066167, WO 07/018738, and US Patent 7,160,876, each of which is herein incorporated by reference in its entirety.

Particular embodiments according to the general Formula 9 include the following: 2-[4-(1-azabicyclo[2.2.2]oct-3-yloxy)phenyl]-1 H-indole;
3-[4-(1-azabicyclo[2.2.2]oct-3-yloxy)phenyl]-1H-indole;
4-[4-(1-azabicyclo[2.2.2]oct-3-yloxy)phenyl]-1H-indole;
5-[4-(1-azabicydo[2.2.2]oct-3-yloxy)phenyl]-1H-indole;
6-[4-(1-azabicyclo[2.2.2]oct-3-yloxy)phenyl]-1H-indole;
5-[6-(1-azabicyclo[2.2.2]oct-3-yloxy)pyridazin-3-yl]-1H-indole;
4-[6-(1-azabicyclo[2.2.2]oct-3-yloxy)pyridazin-3-yl]-1H-indole;
5-[4-(1-azabicyclo[2.2.2]oct-3-yloxy)phenyl]-3-methyl-1H-indazole;
5-[2-(1-azabicyclo[2.2.2]oct-3-yloxy)pyrimidin-5-yl]-1H-indole;
6-[4-(1-azabicyclo[2.2.2]oct-3-yloxy)phenyl]-1,3-benzothiazo-3-amine;
   and pharmaceutically acceptable salts thereof.

Compounds useful according to the present invention also include compounds of Formula 10:

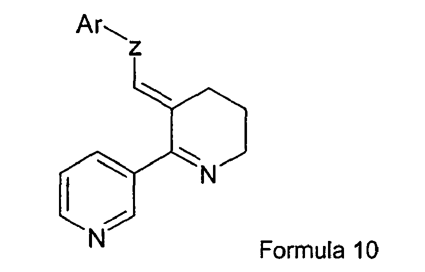

In Formula 10, Ar is an phenyl group, unsubstituted or substituted, and Z is either - CH=CH- or a covalent bond.

Such compounds are disclosed as α7 ligands in, for instance, PCTs WO 92/15306, WO 94/05288,WO 99/10338, WO04/019943, WO 04/052365 and WO 06/133303, and US

Patent 5,741,802 and US Patent 5,977,144, each of which is herein incorporated by reference in its entirety.

Particular embodiments according to the general Formula 10 include the following:
3-(2,4-dimethoxybenzylidene)anabaseine;
3-(4-hydroxybenzylidene)anabaseine;
3-(4-methoxybenzylidene)anabaseine;
3-(4-aminobenzylidene)anabaseine;
3-(4-hydroxy-2-methoxybenzylidene)anabaseine;
3-(2-hydroxy-4-methoxybenzylidene)anabaseine;
3-(4-isopropoxybenzylidene)anabaseine;
3-(4-acetylaminocinnamylidene)anabaseine;
3-(4-hydroxycinnamylidene)anabaseine;
3-(4-methoxycinnamylidene)anabaseine;
3-(4-hydroxy-2-methoxycinnamylidene)anabaseine;
3-(2,4-dimethoxycinnamylidene)anabaseine;
3-(4-acetoxycinnamylidene)anabaseine;
   and pharmaceutically acceptable salts thereof.

Compounds useful according to the present invention also include compounds of Formula 11:

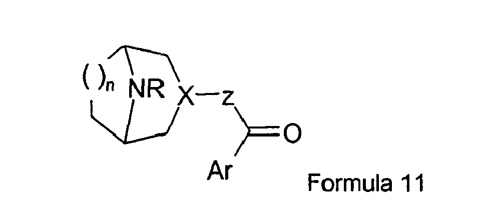

In Formula 11, n is 1 or 2; R is H or alkyl, but most preferably methyl; X is nitrogen or CH; Z is NH or a covalent bond, and when X is nitrogen, Z must be a covalent bond; and Ar is an indolyl, indazolyl, 1,2-benzisoxazolyl or 1,2-benzisothiazolyl moiety, attached in each case via the 3 position to the carbonyl.

Such compounds are disclosed as α7 ligands in, for instance, PCT WO 06/001894, herein incorporated by reference in its entirety.

Particular embodiments according to the general Formula 11 include the following:
(8-methyl-8-azabicyclo[3.2.1]oct-3-yl)-6-(2-thienyl)-7H-indazole-3-carboxamide;
3-((3-methyl-3,8-diazabicyclo[3.2.1]oct-8-yl)carbonyl)-7H-indazole;
3-((8-methyl-3,8-diazabicyclo[3.2.1]oct-3-yl)carbonyl)-7H-indazole;
5-methoxy-N-(9-methyl-9-azabicyclo[3.2.1]non-3-yl)-7H-indazole-3-carboxamide;
6-methoxy-N-(9-methyl-9-azabicyclo[3.2.1]non-3-yl)-1,2-benzisothiazole-3-carboxamide; and pharmaceutically acceptable salts thereof.

### Synthetic Examples

### (2S,3R)-3-amino-2-((3-pyridinyl)methyl)-1-azabicyclo[2.2.2]octane di-p-toluoyl-D-tartrate salt

The following large scale synthesis of (2S,3R)-3-amino-2-((3-pyridinyl)methyl)-1-azabicyclo[2.2.2]octane di-*p*-toluoyl-D-tartrate salt is representative.

### 2-((3-Pyridinyl)methylene)-1-azabicyclo[2.2.2]octan-3-one

3-Quinuclidinone hydrochloride (8.25 kg, 51.0 mol) and methanol (49.5 L) were added to a 100 L glass reaction flask, under an nitrogen atmosphere, equipped with a mechanical stirrer, temperature probe, and condenser. Potassium hydroxide (5.55 kg, 99.0 mol) was added via a powder funnel over an approximately 30 min period, resulting in a rise in reaction temperature from 50°C to 56°C. Over an approximately 2 h period, 3-pyridinecarboxaldehyde (4.80 kg, 44.9 mol) was added to the reaction mixture. The resulting mixture was stirred at 20°C ± 5°C for a minimum of 12 h, as the reaction was monitored by thin layer chromatography (TLC). Upon completion of the reaction, the reaction mixture was filtered through a sintered glass funnel and the filter cake was washed with methanol (74.2 L). The filtrate was concentrated, transferred to a reaction flask, and water (66.0 L) was added. The suspension was stirred for a minimum of 30 min, filtered, and the filter cake was washed with water (90.0 L) until the pH of the rinse was 7-9. The solid was dried under vacuum at 50°C ± 5°C for a minimum of 12 h to give 8.58 kg (89.3%) of 2-((3-pyridinyl)methylene)-1-azabicyclo[2.2.2]octan-3-one.

### (2S)-2-((3-pyridinyl)methyl)-1-azabicyclo[2.2.2]octan-3-one di-p-toluoyl-D-tartrate salt

2-((3-Pyridinyl)methylene)-1-azabicyclo[2.2.2]octan-3-one (5.40 kg, 25.2 mol) and methanol (40.5 L) were added to a 72 L reaction vessel under an inert atmosphere equipped with a mechanical stirrer, temperature probe, low-pressure gas regulator system, and pressure gauge. The headspace was filled with nitrogen, and the mixture was stirred to obtain a clear yellow solution. To the flask was added 10% palladium on carbon (50% wet) (270 g). The atmosphere of the reactor was evacuated using a vacuum pump, and the headspace was replaced with hydrogen to 10 to 20 inches water pressure. The evacuation and pressurization with hydrogen were repeated 2 more times, leaving the reactor under 20 inches water pressure of hydrogen gas after the third pressurization. The reaction mixture was stirred at 20°C ± 5°C for a minimum of 12 h, and the reaction was monitored via TLC. Upon completion of the reaction, the suspension was filtered through a bed of Celite^{®}545 (1.9 kg) on a sintered glass funnel, and the filter cake was washed with methanol (10.1 L). The filtrate was concentrated to obtain a semi-solid which was transferred, under an nitrogen atmosphere, to a 200 L reaction flask fitted with a mechanical stirrer, condenser, and temperature probe. The semi-solid was dissolved in ethanol (57.2 L), and di-p-toluoyl-D-tartaric acid (DTTA) (9.74 kg, 25.2 mol) was added. The stirring reaction mixture was heated at reflux for a minimum of 1 h, and for an additional minimum of 12 h while the reaction was cooled to between 15°C and 30°C. The suspension was filtered using a tabletop filter, and the filter cake was washed with ethanol (11.4 L). The product was dried under vacuum at ambient temperature to obtain 11.6 kg (76.2% yield, 59.5% factored for purity) of (2S)-2-((3-pyridinyl)methyl)-1-azabicyclo[2.2.2]octan-3-one di-*p*-toluoyl-D-tartrate salt.

### (2S,3R)-3-Amino-2-((3-pyridinyl)methyl)-1-azabicyclo[2.2.2]octane di-p-toluoyl-D-tartrate salt

Water (46.25 L) and sodium bicarbonate (4.35 kg, 51.8 mol) were added to a 200 L flask. Upon complete dissolution, dichloromethane (69.4 L) was added. (2S)-2-((3-Pyridinyl)methyl)-1-azabicyclo[2.2.2]octan-3-one di-*p*-toluoyl-D-tartrate salt (11.56 kg, 19.19 mol) was added, and the reaction mixture was stirred for between 2 min and 10 min. The layers were allowed to separate for a minimum of 2 min (additional water (20 L) was added when necessary to partition the layers). The organic phase was removed and dried over anhydrous sodium sulfate. Dichloromethane (34.7 L) was added to the remaining aqueous phase, and the suspension was stirred for between 2 min and 10 min. The layers were allowed to separate for between 2 min and 10 min. Again, the organic phase was removed and dried over anhydrous sodium sulfate. The extraction of the aqueous phase with dichloromethane (34.7 L) was repeated one more time, as above. Samples of each extraction were submitted for chiral HPLC analysis. The sodium sulfate was removed by filtration, and the filtrates were concentrated to obtain (2S)-2-((3-pyridinyl)methyl)-1-azabicyclo[2.2.2]octan-3-one (4.0 kg) as a solid.

The (2S)-2-((3-pyridinyl)methyl)-1-azabicyclo[2.2.2]octan-3-one (3.8 kg) was transferred to a clean 100 L glass reaction flask, under a nitrogen atmosphere, fitted with a mechanical stirrer and temperature probe. Anhydrous tetrahydrofuran (7.24 L) and (+)-(R)-α-methylbenzylamine (2.55 L, 20.1 mol) were added. Titanium(IV) isopropoxide (6.47 L, 21.8 mol) was added to the stirred reaction mixture over a 1 h period. The reaction was stirred under a nitrogen atmosphere for a minimum of 12 h. Ethanol (36.17 L) was added to the reaction mixture. The reaction mixture was cooled to below -5°C, and sodium borohydride (1.53 kg, 40.5 mol) was added in portions, keeping the reaction temperature below 15°C (this addition took several hours). The reaction mixture was then stirred at 15°C ± 10°C for a minimum of 1 h. The reaction was monitored by HPLC, and upon completion of the reaction (as indicated by less than 0.5% of (2S)-2-((3-pyridinyl)methyl)-1-azabicyclo[2.2.2]octan-3-one remaining), 2 M sodium hydroxide (15.99 L) was added and the mixture was stirred for a minimum of 10 min. The reaction mixture was filtered through a bed of Celite^{®}545 in a tabletop funnel. The filter cake was washed with ethanol (15.23 L), and the filtrate was concentrated to obtain an oil.

The concentrate was transferred to a clean 100 L glass reaction flask equipped with a mechanical stirrer and temperature probe under an inert atmosphere. Water (1 L) was added, and the mixture was cooled to 0°C ± 5°C. 2 M Hydrochloric acid (24 L) was added to the mixture to adjust the pH of the mixture to pH 1. The mixture was then stirred for a minimum of 10 min, and 2 M sodium hydroxide (24 L) was slowly added to adjust the pH of the mixture to pH 14. The mixture was stirred for a minimum of 10 min, and the aqueous phase was extracted with dichloromethane (3 x 15.23 L). The organic phases were dried over anhydrous sodium sulfate (2.0 kg), filtered, and concentrated to give (2S,3R)-N-((1R)-phenylethyl)-3-amino-2-((3-pyridinyl)methyl))-1-azabicyclo[2.2.2]octane (4.80 kg, 84.7% yield).

The (2S,3R)-N-((1R)-phenylethyl)-3-amino-2-((3-pyridinyl)methyl)-1-azabicyclo[2.2.2]octane was transferred to a 22 L glass flask equipped with a mechanical stirrer and temperature probe under an inert atmosphere. Water (4.8 L) was added, and the stirring mixture was cooled to 5°C ± 5°C. Concentrated hydrochloric acid (2.97 L) was slowly added to the reaction flask, keeping the temperature of the mixture below 25°C. The resulting solution was transferred to a 72 L reaction flask containing ethanol (18 L), equipped with a mechanical stirrer, temperature probe, and condenser under an inert atmosphere. To the flask was added 10% palladium on carbon (50% wet) (311.1 g) and cyclohexene (14.36 L). The reaction mixture was heated at near-reflux for a minimum of 12 h, and the reaction was monitored by TLC. Upon completion of the reaction, the reaction mixture was cooled to below 45°C, and it was filtered through a bed of Celite^{®}545 (1.2 kg) on a sintered glass funnel. The filter cake was rinsed with ethanol (3 L) and the filtrate was concentrated to obtain an aqueous phase. Water (500 mL) was added to the concentrated filtrate, and this combined aqueous layer was washed with methyl tert-butyl ether (MTBE) (2 x 4.79 L). 2 M Sodium hydroxide (19.5 L) was added to the aqueous phase to adjust the pH of the mixture to pH 14. The mixture was then stirred for a minimum of 10 min. The aqueous phase was extracted with chloroform (4 × 11.96 L), and the combined organic phases were dried over anhydrous sodium sulfate (2.34 kg). The filtrate was filtered and concentrated to obtain (2S,3R)-3-amino-2-((3-pyridinyl)methyl)-1-azabicyclo[2.2.2]octane (3.49 kg, > quantitative yield) as an oil.

The (2S,3R)-3-amino-2-((3-pyridinyl)methyl)-1-azabicyclo[2.2.2]octane was transferred to a clean 100 L reaction flask equipped with a mechanical stirrer, condenser, and temperature probe under an inert atmosphere. Ethanol (38.4 L) and di-p-toluoyl-D-tartaric acid (3.58 kg, 9.27 mol) were added. The reaction mixture was heated at gentle reflux for a minimum of 1 h. The reaction mixture was then stirred for a minimum of 12 h while it was cooled to between 15 °C and 30°C. The resulting suspension was filtered, and the filter cake was washed with ethanol (5.76 L). The filter cake was transferred to a clean 100 L glass reaction flask equipped with a mechanical stirrer, temperature probe, and condenser under an inert atmosphere. A 9:1 ethanol/water solution (30.7 L) was added, and the resulting slurry was heated at gentle reflux for a minimum of 1 h. The reaction mixture was then stirred for a minimum of 12 h while cooling to between 15 °C and 30°C. The mixture was filtered and the filter cake was washed with ethanol (5.76 L). The product was collected and dried under vacuum at 50°C ± 5°C for a minimum of 12 h to give 5.63 kg (58.1 % yield) of (2S,3R)-3-amino-2-((3-pyridinyl)methyl)-1-azabicyclo[2.2.2]octane di-*p*-toluoyl-D-tartrate salt.

### Compound A: (2S,3R)-N-(2-((3-Pyridinyl)methyl)-1-azabicyclo[2.2.2]octan-3-yl)-5-methylthiophene-2-carboxamide

(2S,3R)-3-Amino-2-((3-pyridinyl)methyl)-1-azabicyclo[2.2.2]octane di-*p*-toluoyl-D-tartrate salt (51.0 g, 84.5 mmol), water (125 mL), 2 M sodium hydroxide (150 mL) and chloroform (400 mL) were shaken together in a separatory funnel, and the chloroform layer was drawn off. The aqueous layer was extracted three more times with chloroform (2 × 200 mL, then 100 mL). The combined chloroform layers were washed with saturated aqueous sodium chloride, dried over anhydrous sodium sulfate and concentrated by rotary evaporation. High vacuum treatment left (2S,3R)-3-amino-2-((3-pyridinyl)methyl)-1-azabicyclo[2.2.2]octane (18.0 g) as an oil.

The (2S,3R)-3-amino-2-((3-pyridinyl)methyl)-1-azabicyclo[2.2.2]octane was transferred to a 1 L glass reaction flask under an inert atmosphere. Dichloromethane (500 mL), triethylamine (40 mL, 0.30 mol), 5-methylthiophene-2-carboxylic acid (13.5 g, 94.9 mmol) and O-(benzotriazol-1-yl)-N,N,N,1-tetramethyluronium hexafluorophosphate (HBTU) (36.0 g, 94.9 mmol) were added to the reaction mixture. The mixture was stirred overnight at ambient temperature, and at which time the reaction was complete by HPLC. Water (200 mL), 2 M sodium hydroxide (200 mL) were added to the reaction, and the resulting mixture was shaken. The dichloromethane layer and a 200 mL dichloromethane extract of the aqueous layer were combined and washed with saturated aqueous sodium chloride (200 mL), dried over anhydrous sodium sulfate and concentrated, by rotary evaporation, to give an oil (quantitative yield). Column chromatographic purification on silica gel, eluting with a methanol in ethyl acetate gradient, gave (2S,3R)-N-(2-((3-pyridinyl)methyl)-1-azabicyclo[2.2.2]octan-3-yl)-5-methylthiophene-2-carboxamide (22.6 g, 78.5% yield) as a powder.

### Compound B: (2S,3R)-N-(2-((3-Pyridinyl)methyl)-1-azabicyclo[2.2.2]octan-3-yl)-5-(2 pyridinyl)thiophene-2-carboxamide

A sample of (2S,3R)-3-amino-2-((3-pyridinyl)methyl)-1-azabicyclo[2.2.2]octane (5.5 g, 25 mmol), generated as described above from (2S,3R)-3-Amino-2-((3-pyridinyl)methyl)-1-azabicyclo[2.2.2]octane di-*p*-toluoyl-D-tartrate salt, was transferred to a 500 mL glass reaction flask under an inert atmosphere. Dichloromethane (200 mL), triethylamine (10 mL, 72 mmol), 5-(2-pyridinyl)thiophene-2-carboxylic acid (6.0 g, 29 mmol) and O-(benzotriazol-1-yl)-N,N,N,1-tetramethyluronium hexafluorophosphate (HBTU) (11.1 g, 29.2 mmol) were added to the reaction mixture. The mixture was stirred overnight at ambient temperature, and at which time the reaction was complete by HPLC. Water (100 mL), 2 M sodium hydroxide (100 mL) were added to the reaction, and the resulting mixture was shaken. The dichloromethane layer and two 250 mL dichloromethane extracts of the aqueous layer were combined and washed with saturated aqueous sodium chloride (200 mL), dried over anhydrous sodium sulfate and concentrated, by rotary evaporation, to give an oil (quantitative yield). Column chromatographic purification on silica gel, eluting with a methanol in ethyl acetate gradient, gave (2S,3R)-N-(2-((3-pyridinyl)methyl)-1-azabicyclo[2.2.2]octan-3-yl)-5-(2-pyridinyl)thiophene-2-carboxamide (8.0 g, 80% yield) as a powder.

### Compound C

### (2S, 3R)-N-(2-((3-pyridinyl)methyl)-1-azabicyclo[2.2.2]octan-3-yl)benzofuran-2-carboxamide

Racemic N-(2-((3-pyridinyl)methyl-1-azabicyclo[2.2.2]oct-3-yl)benzofuran-2-carboxamide, a synthesis, and utility in medical treatment, is described in US Patent No. 6,953,855 to Mazurov et al, herein incorporated by reference.

Particular synthetic steps vary in their amenability to scale-up. Reactions are found lacking in their ability to be scaled-up for a variety of reasons, including safety concerns, reagent expense, difficult work-up or purification, reaction energetics (thermodynamics or kinetics), and reaction yield. Both small scale and large scale synthetic methods are herein described. The scalable synthesis utilizes both the dynamic resolution of a racemizable ketone (2-((3-pyridinyl)methyl)-1-azabicyclo[2.2.2]octan-3-one) and the stereoselective reduction of the (R)-α-methylbenzylamine imine derivative (reductive amination) of the resolved ketone.

### Small scale

### 2-((3-Pyridinyl)methylene)-1-azabicyclo[2.2.2]octan-3-one

Potassium hydroxide (56 g, 0.54 mole) was dissolved in methanol (420 mL). 3-Quinuclidinone hydrochloride (75 g, 0.49 mole) was added and the mixture was stirred for 30 min at ambient temperature. 3-Pyridinecarboxaldehyde (58 g, 0.54 mole) was added and the mixture stirred for 16 h at ambient temperature. The reaction mixture became yellow during this period, with solids caking on the walls of the flask. The solids were scraped from the walls and the chunks broken up. With rapid stirring, water (390 mL) was added. When the solids dissolved, the mixture was cooled at 4°C overnight. The crystals were collected by filtration, washed with water, and air dried to obtain 80 g of yellow solid. A second crop (8 g) was obtained by concentration of the filtrate to ~10% of its former volume and cooling at 4°C overnight. Both crops were sufficiently pure for further transformation (88 g, 82% yield).

### 2-((3-Pyridinyl)methyl)-1-azabicyclo[2.2.2]octan-3-one

2-((3-Pyridinyl)methylene)-1-azabicyclo[2.2.2]octan-3-one (20 g, 93 mmol) was suspended in methanol (200 mL) and treated with 46 mL of 6 M hydrochloric acid. 10% Palladium on carbon (1.6 g) was added and the mixture was shaken under 25 psi hydrogen for 16 h. The mixture was filtered through diatomaceous earth, and the solvent was removed from the filtrate by rotary evaporation. This provided crude 2-((3-pyridinyl)methyl)-1-azabicyclo[2.2.2]octan-3-one hydrochloride, as a white gum (20 g), which was subsequently treated with 2 M sodium hydroxide (50 mL) and chloroform (50 mL) and stirred for an hour. The chloroform layer was separated, and the aqueous phase was treated with 2 M sodium hydroxide (-5 mL, enough to raise the pH to 10) and saturated aqueous Sodium chloride (25 mL). This aqueous mixture was extracted with chloroform (3 × 10 mL), and the combined chloroform extracts were dried (anhydrous magnesium sulfate) and concentrated by rotary evaporation. The residue (18 g) was dissolved in warm ether (320 mL) and cooled to 4°C. The white solid was filtered off, washed with a small portion of cold ether and air dried. Concentration of the filtrate to ~10% of its former volume and cooling at 4°C produced a second crop. A combined yield 16 g (79%) was obtained.

### 3-Amino-2-((3-pyridinyl)methyl)-1-azabicyclo[2.2.2]octane

To a stirred solution of 2-((3-pyridinyl)methyl)-1-azabicyclo[2.2.2]octan-3-one (3.00 g, 13.9 mmol) in dry methanol (20 mL), under nitrogen, was added a 1 M solution of zinc chloride in ether (2.78 mL, 2.78 mmol). After stirring at ambient temperature for 30 min, this mixture was treated with solid ammonium formate (10.4 g, 167 mmol). After stirring another hour at ambient temperature, solid sodium cyanoborohydride (1.75 g, 27.8 mmol) was added in portions. The reaction was then stirred at ambient temperature overnight and terminated by addition of water (- 5 mL). The quenched reaction was partitioned between 5 M sodium hydroxide (10 mL) and chloroform (20 mL). The aqueous layer was extracted with chloroform (20 mL), and combined organic layers were dried (sodium sulfate), filtered and concentrated. This left 2.97 g of yellow gum. GCMS analysis indicated that the product was a 1:9 mixture of the *cis* and *trans* amines, along with a trace of the corresponding alcohol (98% total mass recovery).

### (2R,3S) and (2S,3R)-3-amino-2-((3-pyridinyl)methyl)-1-azabicyclo[2.2.2]octane

Di-p-toluoyl-D-tartaric acid (5.33 g, 13.8 mmol) was added to a stirred solution of crude 3-amino-2-((3-pyridinyl)methyl)-1-azabicyclo[2.2.2]octane (6.00 g, 27.6 mmol of 1:9 *cis*/*trans*) in methanol (20 mL). After complete dissolution, the clear solution was then concentrated to a solid mass by rotary evaporation. The solid was dissolved in a minimum amount of boiling methanol (-5 mL). The solution was cooled slowly, first to ambient temperature (1 h), then for - 4 h at 5°C and finally at -5°C overnight. The precipitated salt was collected by suction filtration and recrystallized from 5 mL of methanol. Air drying left 1.4 g of white solid, which was partitioned between chloroform (5 mL) and 2 M sodium hydroxide (5 mL). The chloroform layer and a 5 mL chloroform extract of the aqueous layer were combined, dried (anhydrous sodium sulfate) and concentrated to give a colorless oil (0.434 g). The enantiomeric purity of this free base was determined by conversion of a portion into its N-(tert-butoxycarbonyl)-L-prolinamide, which was then analyzed for diastereomeric purity (98%) using LCMS.

The mother liquor from the initial crystallization was made basic (- pH 11) with 2 M sodium hydroxide and extracted twice with chloroform (10 mL). The chloroform extracts were dried (anhydrous sodium sulfate) and concentrated to give an oil. This amine (3.00 g, 13.8 mmol) was dissolved in methanol (10 mL) and treated with di-p-toluoyl-L-tartaric acid (2.76 g, 6.90 mmol). The mixture was warmed to aid dissolution and then cooled slowly to -5°C, where it remained overnight. The precipitate was collected by suction filtration, recrystallized from methanol and dried. This left 1.05 g of white solid. The salt was converted into the free base (yield = 0.364 g), and the enantiomeric purity (97%) was assessed using the prolinamide method, as described above for the other enantiomer.

### Trans isomer 1 of N-(2-((3-pyridinyl)methyl)-1-azabicyclo[2.2.2]octan-3-yl)benzofuran-2-carboxamide

Diphenylchlorophosphate (0.35 mL, 0.46 g, 1.7 mmol) was added drop-wise to a solution of benzofuran-2-carboxylic acid (0.28 g, 1.7 mmol) and triethylamine (0.24 mL, 0.17 g, 1.7 mmol) in dry dichloromethane (5 mL). After stirring at ambient temperature for 30 min, a solution of (2S,3R)-3-amino-2-((3-pyridinyl)methyl)-1-azabicyclo[2.2.2]octane (0.337 g, 1.55 mmol) (that derived from the di-p-toluoyl-D-tartaric acid salt) and triethylamine (0.24 mL, 0.17 g, 1.7 mmol) in dry dichloromethane (5 mL) was added. The reaction mixture was stirred overnight at ambient temperature, and then treated with 10% sodium hydroxide (1 mL). The biphasic mixture was separated, and the organic layer was concentrated on a Genevac centrifugal evaporator. The residue was dissolved in methanol (6 mL) and purified by HPLC on a C18 silica gel column, using an acetonitrile/water gradient, containing 0.05% trifluoroacetic acid, as eluent. Concentration of selected fractions, partitioning of the resulting residue between chloroform and saturated aqueous sodium bicarbonate, and evaporation of the chloroform gave 0.310 g (42% yield) of white powder (95% pure by GCMS). ¹H NMR (300 MHz, CDCl₃) δ 8.51 (d, 1H), 8.34 (dd, 1H), 7.66 (d, 1H), 7.58 (dt, 1 H), 7.49 (d, 1 H), 7.44 (s, 1H), 7.40 (dd, 1 H), 7.29 (t, 1 H), 7.13 (dd, 1H), 6.63 (d, 1 H), 3.95 (t, 1 H), 3.08 (m, 1 H), 2.95 (m, 4H), 2.78 (m, 2H), 2.03 (m, 1 H), 1.72 (m, 3H), 1.52 (m, 1 H). This material (trans enantiomer 1) was later determined to be identical, by chiral chromatogrphic analysis, to material whose absolute configuration is 2S,3R (established by x-ray crystallographic analysis).

### Trans isomer 2 of N-(2-((3-pyridinyl)methyl)-1-azabicyclo[2.2.2]octan-3-yl)benzofuran-2-carboxamide

Diphenylchlorophosphate (96 µL, 124 mg, 0.46 mmol) was added drop-wise to a solution of the benzofuran-2-carboxylic acid (75 mg, 0.46 mmol) (that derived from the di-p-toluoyl-L-tartaric acid salt) and triethylamine (64 µL, 46 mg, 0.46 mmol) in dry dichloromethane (1 mL). After stirring at ambient temperature for 45 min, a solution of (2R,3S)-3-amino-2-((3-pyridinyl)methyl)-1-azabicyclo[2.2.2]octane (0.10 g, 0.46 mmol) and triethylamine (64 µL, 46 mg, 0.46 mmol) in dry dichloromethane (1 mL) was added. The reaction mixture was stirred overnight at ambient temperature, and then treated with 10% sodium hydroxide (1 mL). The biphasic mixture was separated, and the organic layer and a chloroform extract (2 mL) of the aqueous layer was concentrated by rotary evaporation. The residue was dissolved in methanol and purified by HPLC on a C18 silica gel column, using an acetonitrile/water gradient, containing 0.05% trifluoroacetic acid, as eluent. Concentration of selected fractions, partitioning of the resulting residue between chloroform and saturated aqueous sodium bicarbonate, and evaporation of the chloroform gave 82.5 mg (50% yield) of a white powder. The NMR spectrum was identical to that obtained for the (2S,3R) isomer. Since the immediate precursor of this material (trans enantiomer 2) is enantiomeric to the immediate precursor of 2S,3R compound (trans enantiomer 1), the absolute configuration of trans enantiomer 2 is presumed to be 2R,3S.

### Large scale

### 2-((3-Pyridinyl)methylene)-1-azabicyclo[2.2.2]octan-3-one

3-Quinuclidinone hydrochloride (8.25 kg, 51.0 mol) and methanol (49.5 L) were added to a 100 L glass reaction flask, under an nitrogen atmosphere, equipped with a mechanical stirrer, temperature probe, and condenser. Potassium hydroxide (5.55 kg, 99.0 mol) was added via a powder funnel over an approximately 30 min period, resulting in a rise in reaction temperature from 50°C to 56°C. Over an approximately 2 h period, 3-pyridinecarboxaldehyde (4.80 kg, 44.9 mol) was added to the reaction mixture. The resulting mixture was stirred at 20°C ± 5°C for a minimum of 12 h, as the reaction was monitored by thin layer chromatography (TLC). Upon completion of the reaction, the reaction mixture was filtered through a sintered glass funnel and the filter cake was washed with methanol (74.2 L). The filtrate was concentrated, transferred to a reaction flask, and water (66.0 L) was added. The suspension was stirred for a minimum of 30 min, filtered, and the filter cake was washed with water (90.0 L) until the pH of the rinse was 7-9. The solid was dried under vacuum at 50°C ± 5°C for a minimum of 12 h to give 8.58 kg (89.3%) of 2-((3-pyridinyl)methylene)-1-azabicyclo[2.2.2]octan-3-one.

### (2S)-2-((3-pyridinyl)methyl)-1-azabicyclo[2.2.2]octan-3-one di-p-toluoyl-D-tartrate salt

2-((3-Pyridinyl)methylene)-1-azabicyclo[2.2.2]octan-3-one (5.40 kg, 25.2 mol) and methanol (40.5 L) were added to a 72 L reaction vessel under an inert atmosphere equipped with a mechanical stirrer, temperature probe, low-pressure gas regulator system, and pressure gauge. The headspace was filled with nitrogen, and the mixture was stirred to obtain a clear yellow solution. To the flask was added 10% palladium on carbon (50% wet) (270 g). The atmosphere of the reactor was evacuated using a vacuum pump, and the headspace was replaced with hydrogen to 10 to 20 inches water pressure. The evacuation and pressurization with hydrogen were repeated 2 more times, leaving the reactor under 20 inches water pressure of hydrogen gas after the third pressurization. The reaction mixture was stirred at 20°C ± 5°C for a minimum of 12 h, and the reaction was monitored via TLC. Upon completion of the reaction, the suspension was filtered through a bed of Celite^{®}545 (1.9 kg) on a sintered glass funnel, and the filter cake was washed with methanol (10.1 L). The filtrate was concentrated to obtain a semi-solid which was transferred, under an nitrogen atmosphere, to a 200 L reaction flask fitted with a mechanical stirrer, condenser, and temperature probe. The semi-solid was dissolved in ethanol (57.2 L), and di-p-toluoyl-D-tartaric acid (DTTA) (9.74 kg, 25.2 mol) was added. The stirring reaction mixture was heated at reflux for a minimum of 1 h, and for an additional minimum of 12 h while the reaction was cooled to between 15°C and 30°C. The suspension was filtered using a tabletop filter, and the filter cake was washed with ethanol (11.4 L). The product was dried under vacuum at ambient temperature to obtain 11.6 kg (76.2% yield, 59.5% factored for purity) of (2S)-2-((3-pyridinyl)methyl)-1-azabicyclo[2.2.2]octan-3-one di-*p*-toluoyl-D-tartrate salt.

### (2S,3R)-3-Amino-2-((3-pyridinyl)methyl)-1-azabicyclo[2.2.2]octane di-p-toluoyl-D-tartrate salt

Water (46.25 L) and sodium bicarbonate (4.35 kg, 51.8 mol) were added to a 200 L flask. Upon complete dissolution, dichloromethane (69.4 L) was added. (2S)-2-((3-Pyridinyl)methyl)-1-azabicyclo[2.2.2]octan-3-one di-*p*-toluoyl-D-tartrate salt (11.56 kg, 19.19 mol) was added, and the reaction mixture was stirred for between 2 min and 10 min. The layers were allowed to separate for a minimum of 2 min (additional water (20 L) was added when necessary to partition the layers). The organic phase was removed and dried over anhydrous sodium sulfate. Dichloromethane (34.7 L) was added to the remaining aqueous phase, and the suspension was stirred for between 2 min and 10 min. The layers were allowed to separate for between 2 min and 10 min. Again, the organic phase was removed and dried over anhydrous sodium sulfate. The extraction of the aqueous phase with dichloromethane (34.7 L) was repeated one more time, as above. Samples of each extraction were submitted for chiral HPLC analysis. The sodium sulfate was removed by filtration, and the filtrates were concentrated to obtain (2S)-2-((3-pyridinyl)methyl)-1-azabicyclo[2.2.2]octan-3-one (4.0 kg) as a solid.

The (2S)-2-((3-pyridinyl)methyl)-1-azabicyclo[2.2.2]octan-3-one (3.8 kg) was transferred to a clean 100 L glass reaction flask, under a nitrogen atmosphere, fitted with a mechanical stirrer and temperature probe. Anhydrous tetrahydrofuran (7.24 L) and (+)-(R)-α-methylbenzylamine (2.55 L, 20.1 mol) were added. Titanium(IV) isopropoxide (6.47 L, 21.8 mol) was added to the stirred reaction mixture over a 1 h period. The reaction was stirred under a nitrogen atmosphere for a minimum of 12 h. Ethanol (36.17 L) was added to the reaction mixture. The reaction mixture was cooled to below -5°C, and sodium borohydride (1.53 kg, 40.5 mol) was added in portions, keeping the reaction temperature below 15°C (this addition took several hours). The reaction mixture was then stirred at 15°C ± 10°C for a minimum of 1 h. The reaction was monitored by HPLC, and upon completion of the reaction (as indicated by less than 0.5% of -(2S)-2-((3-pyridinyl)methyl)-1-azabicyclo[2.2.2]octan-3 one remaining), 2 M sodium hydroxide (15.99 L) was added and the mixture was stirred for a minimum of 10 min. The reaction mixture was filtered through a bed of Celite^{®}545 in a tabletop funnel. The filter cake was washed with ethanol (15.23 L), and the filtrate was concentrated to obtain an oil.

The concentrate was transferred to a clean 100 L glass reaction flask equipped with a mechanical stirrer and temperature probe under an inert atmosphere. Water (1 L) was added, and the mixture was cooled to 0°C ± 5°C. 2 M Hydrochloric acid (24 L) was added to the mixture to adjust the pH of the mixture to pH 1. The mixture was then stirred for a minimum of 10 min, and 2 M sodium hydroxide (24 L) was slowly added to adjust the pH of the mixture to pH 14. The mixture was stirred for a minimum of 10 min, and the aqueous phase was extracted with dichloromethane (3 × 15.23 L). The organic phases were dried over anhydrous sodium sulfate (2.0 kg), filtered, and concentrated to give (2S,3R)-N-((1R)-phenylethyl)-3-amino-2-((3-pyridinyl)methyl))-1-azabicyclo[2.2.2]octane (4.80 kg, 84.7% yield).

The (2S,3R)-N-((1R)-phenylethyl)-3-amino-2-((3-pyridinyl)methyl)-1-azabicyclo[2.2.2]octane was transferred to a 22 L glass flask equipped with a mechanical stirrer and temperature probe under an inert atmosphere. Water (4.8 L) was added, and the stirring mixture was cooled to 5°C ± 5°C. Concentrated hydrochloric acid (2.97 L) was slowly added to the reaction flask, keeping the temperature of the mixture below 25°C. The resulting solution was transferred to a 72 L reaction flask containing ethanol (18 L), equipped with a mechanical stirrer, temperature probe, and condenser under an inert atmosphere. To the flask was added 10% palladium on carbon (50% wet) (311.1 g) and cyclohexene (14.36 L). The reaction mixture was heated at near-reflux for a minimum of 12 h, and the reaction was monitored by TLC. Upon completion of the reaction, the reaction mixture was cooled to below 45°C, and it was filtered through a bed of Celite^{®}545 (1.2 kg) on a sintered glass funnel. The filter cake was rinsed with ethanol (3 L) and the filtrate was concentrated to obtain an aqueous phase. Water (500 mL) was added to the concentrated filtrate, and this combined aqueous layer was washed with methyl tert-butyl ether (MTBE) (2 ×4.79 L). 2 M Sodium hydroxide (19.5 L) was added to the aqueous phase to adjust the pH of the mixture to pH 14. The mixture was then stirred for a minimum of 10 min. The aqueous phase was extracted with chloroform (4 × 11.96 L), and the combined organic phases were dried over anhydrous sodium sulfate (2.34 kg). The filtrate was filtered and concentrated to obtain (2S,3R)-3-amino-2-((3-pyridinyl)methyl)-1-azabicyclo[2.2.2]octane (3.49 kg, > quantitative yield) as an oil.

The (2S,3R)-3-amino-2-((3-pyridinyl)methyl)-1-azabicyclo[2.2.2]octane was transferred to a clean 100 L reaction flask equipped with a mechanical stirrer, condenser, and temperature probe under an inert atmosphere. Ethanol (38.4 L) and di-p-toluoyl-D-tartaric acid (3.58 kg, 9.27 mol) were added. The reaction mixture was heated at gentle reflux for a minimum of 1 h. The reaction mixture was then stirred for a minimum of 12 h while it was cooled to between 15°C and 30°C. The resulting suspension was filtered, and the filter cake was washed with ethanol (5.76 L). The filter cake was transferred to a clean 100 L glass reaction flask equipped with a mechanical stirrer, temperature probe, and condenser under an inert atmosphere. A 9:1 ethanol/water solution (30.7 L) was added, and the resulting slurry was heated at gentle reflux for a minimum of 1 h. The reaction mixture was then stirred for a minimum of 12 h while cooling to between 15°C and 30°C. The mixture was filtered and the filter cake was washed with ethanol (5.76 L). The product was collected and dried under vacuum at 50°C ± 5°C for a minimum of 12 h to give 5.63 kg (58.1% yield) of (2S,3R)-3-amino-2-((3-pyridinyl)methyl)-1-azabicyclo[2.2.2]octane di-*p*-toluoyl-D-tartrate salt.

### (2S,3R)-N-(2-((3-Pyridinyl)methyl)-1-azabicyclo[2.2.2]octan-3-yl)benzofuran-2-carboxamide

(2S,3R)-3-Amino-2-((3-pyridinyl)methyl)-1-azabicyclo[2.2.2]octane di-*p*-toluoyl-D-tartrate salt (3.64 kg, 5.96 mol) and 10% aqueous sodium chloride solution (14.4 L, 46.4 mol) were added to a 72 L glass reaction flask equipped with a mechanical stirrer under an inert atmosphere. 5 M Sodium hydroxide (5.09 L) was added to the stirring mixture to adjust the pH of the mixture to pH 14. The mixture was then stirred for a minimum of 10 min. The aqueous solution was extracted with chloroform (4 × 12.0 L), and the combined organic layers were dried over anhydrous sodium sulfate (1.72 kg). The combined organic layers were filtered, and the filtrate was concentrated to obtain (2S,3R)-3-amino-2-((3-pyridinyl)methyl)-1-azabicyclo[2.2.2]octane (1.27 kg) as an oil.

The (2S,3R)-3-amino-2-((3-pyridinyl)methyl)-1-azabicyclo[2.2.2]octane was transferred to a 50 L glass reaction flask equipped with a mechanical stirrer under an inert atmosphere. Dichloromethane (16.5 L), triethylamine (847 mL, 6.08 mol), benzofuran-2-carboxylic acid (948 g, 5.85 mol) and O-(benzotriazol-1-yl)-N,N,N,1-tetramethyluronium hexafluorophosphate (HBTU) (2.17 kg, 5.85 mol) were added to the reaction mixture. The mixture was stirred for a minimum of 4 h at ambient temperature, and the reaction was monitored by HPLC. Upon completion of the reaction, 10% aqueous potassium carbonate (12.7 L, 17.1 mol) was added to the reaction mixture and the mixture was stirred for a minimum of 5 min. The layers were separated and the organic phase was washed with 10% brine (12.7 L). The layers were separated and the organic phase was cooled to 15°C ± 10 °C. 3 M Hydrochloric acid (8.0 L) was slowly added to the reaction mixture to adjust the pH of the mixture to pH 1. The mixture was then stirred for a minimum of 5 min, and the layers were allowed to partition for a minimum of 5 min. The solids were filtered using a table top filter. The layers of the filtrate were separated, and the aqueous phase and the solids from the funnel were transferred to the reaction flask. 3 M Sodium hydroxide (9.0 L) was slowly added to the flask in portions to adjust the pH of the mixture to pH 14. The aqueous phase was extracted with dichloromethane (2 x 16.5 L). The combined organic phases were dried over anhydrous sodium sulfate (1.71 kg). The mixture was filtered, and the filtrate was concentrated to give (2S,3R)-N-(2-((3-pyridinyl)methyl)-1-azabicyclo[2.2.2]octan-3-yl)benzofuran-2-carboxamide (1.63 kg, 77.0% yield) as a yellow solid.

### (2S,3R)-N-(2-((3-pyridinyl)methyl)-1-azabicyclo[2.2.2]oct-3-yl]benzofuran-2-carboxamide p-toluenesulfonate

(2S,3R)-N-(2-((3-Pyridinyl)methyl)-1-azabicyclo[2.2.2]octan-3-yl)benzofuran-2-carboxamide (1.62 kg, 4.48 mol) and dichloromethane (8.60 kg) were added into a carboy. The weight/weight percent of the material in solution was determined through HPLC analysis. The solution was concentrated to an oil, acetone (4 L) was added, and the mixture was concentrated to an oily solid. Additional acetone (12 L) was added to the oily solid in the rotary evaporator bulb, and the resulting slurry was transferred to a 50 L glass reaction flask with a mechanical stirrer, condenser, temperature probe, and condenser under an inert atmosphere. The reaction mixture was heated to 50°C ± 5°C. Water (80.7 g) was added to the solution, and it was stirred for a minimum of 10 min. *p*-Toluenesulfonic acid (853 g, 4.44 mol) was added to the reaction mixture in portions over approximately 15 min. The reaction mixture was heated to reflux and held at that temperature for a minimum of 30 min to obtain a solution. The reaction was cooled to 40°C ± 5°C over approximately 2 h. Isopropyl acetate (14.1 L) was added over approximately 1.5 h. The reaction mixture was slowly cooled to ambient temperature over a minimum of 10 h. The mixture was filtered and the filter cake was washed with isopropyl acetate (3.5 L). The isolated product was dried under vacuum at 105°C ± 5°C for between 2 h and 9 h to give 2.19 kg (88.5% yield) of (2S,3R)-N-(2-((3-pyridinyl)methyl)-1-azabicyclo[2.2.2]oct-3-yl)benzofuran-2-carboxamide p-toluenesulfonate, mp 226-228°C. ¹H NMR (500 MHz, D₂O) δ 8.29 (s, 1 H), 7.78 (m, J = 5.1, 1 H), 7.63 (d, J = 7.9, 1 H), 7.54 (d, J = 7.8, 1 H), 7.49 (d, *J* = 8.1, 2H), 7.37 (m, *J* = 8.3, 1H), 7.33 (m, J = 8.3, 6.9, 1.0, 1 H), 7.18 (m, J = 7.8, 6.9, 1.0, 1H), 7.14 (d, J = 8.1, 2H), 7.09 (s, 1 H), 6.99 (dd, J = 7.9, 5.1, 1 H), 4.05 (m, J = 7.7, 1H), 3.74 (m, 1 H), 3.47 (m, 2H), 3.28 (m, 1H), 3.22 (m, 1H), 3.15 (dd, J = 13.2, 4.7, 1H), 3.02 (dd, J = 13.2, 11.5, 1 H), 2.19 (s, 3H), 2.02 (m, 2H), 1.93 (m, 2H), 1.79 (m, 1H). ¹³C NMR (126 MHz, D₂O) δ 157.2, 154.1, 150.1, 148.2, 146.4, 145.2, 138.0, 137.0, 130.9, 128.2 (2), 126.9, 126.8, 125.5 (2), 123.7, 123.3, 122.7, 111.7, 100.7, 61.3, 50.2, 48.0, 40.9, 33.1, 26.9, 21.5, 20.8, 17.0.

Samples of this material were converted into Compound C free base (for use in salt selection studies) by treatment with aqueous sodium hydroxide and extraction with chloroform. Thorough evaporation of the chloroform left an off-white powder, mp 167-170°C, with the following spectral characteristics: Positive ion electrospray MS [M+H]⁺ ion m/z = 362. ¹H NMR (500 MHz, DMSO-d₆) δ 8.53 (d, J = 7.6 Hz, 1 H), 8.43 (d, J = 1.7 Hz, 1 H), 8.28 (dd, *J* = 1.6, 4.7 Hz, 1H), 7.77 (d, *J* = 7.7 Hz, 1H), 7.66 (d, *J* = 8.5 Hz, 1H), 7.63 (dt, *J* = 1.7, 7.7 Hz, 1 H), 7.52 (s, 1 H), 7.46 (m, *J* = 8.5, 7.5 Hz, 1 H), 7.33 (m, J = 7.7, 7.5 Hz, 1 H), 7.21 (dd, J = 4.7, 7.7 Hz, 1 H), 3.71 (m, *J* = 7.6 Hz, 1 H), 3.11 (m, 1 H), 3.02 (m, 1H), 2.80 (m, 2H), 2.69 (m, 2H), 2.55 (m, 1 H), 1.80 (m, 1 H), 1.77 (m, 1H), 1.62 (m, 1 H), 1.56 (m, 1H), 1.26 (m, 1 H). ¹³C NMR (126 MHz, DMSO-d₆) δ 158.1, 154.1, 150.1, 149.1, 146.8, 136.4, 135.4, 127.1, 126.7, 123.6, 122.9, 122.6, 111.8, 109.3, 61.9, 53.4, 49.9, 40.3, 35.0, 28.1, 26.1, 19.6.

The monohydrochloride salt of Compound C (see Example 5) was submitted for x-ray crystallographic analysis. The resulting crystal structure established the 2S,3R absolute configuration of Compound C.

### Example 5: Synthesis of (2S,3R)-N-(2-((3-pyridinyl)methyl)-1-azabicyclo[2.2.2]oct-3-yl)benzofuran-2-carboxamide hydrochloride salt

A hydrochloric acid/THF solution was prepared by adding of concentrated hydrochloric acid (1.93 mL of 12M, 23.2 mmol) drop-wise to 8.5 mL of chilled THF. The solution was warmed to ambient temperature. To a round bottom flask was added (2S,3R)-N-(2-((3-pyridinyl)methyl)-1-azabicyclo[2.2.2]oct-3-yl)benzofuran-2-carboxamide (8.49 g, 23.5 mmol) and acetone (85 mL). The mixture was stirred and heated at 45-50°C until a complete solution was obtained. The hydrochloric acid/THF solution prepared above was added drop-wise over a 5 min period, with additional THF (1.5 mL) used in the transfer. Granular, white solids began to form during the addition of the acid solution. The mixture was cooled to ambient temperature, and stirred overnight (16 h). The solids were collected by suction filtration, the filter cake was washed with acetone (10 mL), and the solid was airdried with suction for 30 min. The solid was further dried in a vacuum oven at 75°C for 2 h to give 8.79 g of the fine white crystals (94% yield), mp 255-262°C. Chiral LC analysis gave a purity of 98.8% (270 nm). ¹H-NMR (DMSO-d₆) shows no residual solvents and confirms mono stoichiometry. ¹H NMR (300 MHz, DMSO-d₆) δ 10.7 (broad s, 1H - quaternary ammonium), 8.80 (broad s, 1H - amide H), 8.54 (s, 1 H), 8.23 (d, 1 H), 7.78 (d, 1 H), 7.74 (d, 1H), 7.60 (d, 1 H), 7.47 (m, 2H), 7.33 (m, 1 H), 7.19 (m, 1 H), 4.19 (m, 1H), 4.08 (m, 1 H), 3.05-3.55 (m, 6H), 2.00-2.10 (m, 3H), 1.90 (m, 1 H), 1.70 (m, 1 H). An x-ray crystallographic analysis of this salt established stereochemical assignment and stoichiometry.

### Biological Examples

Compounds A, B, and C are α7-selective ligands. For example, Compounds A, B and C are α7 agonists with Ki values = 1-2 nM in displacement studies using ³H-MLA in rat hippocampal tissues. These compounds exhibit very poor affinity at other nicotinic receptors, namely > 1000 nM, including α4β2. In functional studies, Compounds A, B and C exhibited Eₘₐₓ values > 50% in an electrophysiology functional assay in *Xenopus laevis* oocytes transiently expressing human α7 nicotinic receptor. The IC50s for Compound A are >10 micromolar at more than 60 targets in a receptor profile screen.

### Physiological effects of Selective alpha7 nAChR Agonist

*Body Weight Gain and Food Intake.* Several protein tyrosine phosphatases (PTPases) such as PTP1 B, LAR, SHP-2 and PTEN have been implicated in the development of insulin resistance. In the present studies we measured body mass and weight gain in lean (db+) and obese (db-) mice that were either wild type (PTP1B+) or knockout (PTP1 B-) for the protein phosphatase 1 B gene, following treatment with an α7 nAChR agonist, Compound A, B, or C.

At the end of seven weeks of treatment we found that weight gain was reduced in the alpha7 agonist-treated ten week old db- mice. The db+ lean mice were unaffected by treatment, confirming that the compound was not producing toxic effects that limited food intake. The weight gain in control obese groups (db-) was significantly lower (p< 0.01) than that of controls in both the PTP1B wild type and PTP1B knockout mice. In addition, the daily food intake was significantly lower (p< 0.01) in the treated mice than in the controls, in both PTP1B wild type and knockout mice.

When the alpha7 antagonist, MLA, was given concurrently, the obese mice showed no significant differences in body weight gain or food intake. As such, dietary supplementation with treatment compound effectively lowered food intake and weight gain in obese mice. α7 nAChRs play a central role in regulating food intake through a mechanism that is not dependent on PTP1 B. Further, AG-490 significantly inhibited (p< 0.01) both the weight loss and decreased food intake induced by the α7 agonist.

*Glucose Metabolism.* Similar to the results obtained for food intake and body weight gain, at the end of seven weeks of treatment, plasma glucose levels in the obese (db-) mice treated with the α7 agonist were significantly lower (p< 0.01) than those in the untreated PTP1 B+ and PTP1 B- mice. The α7 nAChR antagonist MLA was given concurrently with treatment compound and the mice showed no significant decrease in plasma glucose. Dietary supplementation with the α7 agonist, therefore, effectively lowers weight gain and food intake in obese mice and lowers the increased levels of glucose due to obesity. This mechanism is not dependent on PTP1B, but rather on JAK2 activation. The JAK2 inhibitor significantly prevented (p< 0.01) the treatment-induced decrease in plasma glucose.

Lean mice displayed rapid glucose disposal and the injected bolus was cleared within 30 minutes. Clearance of glucose in the lean mice treated was similar to that of shamtreated mice. Consistent with obesity-induced insulin resistance, the obese mice showed markedly blunted glucose clearance, removing only 50% of the injected bolus within 30 minutes. By contrast, the treated mice showed normalization of glucose clearance despite obesity (90% of the injected bolus within 30 minutes. Thus, insulin resistance is improved by the alpha7 agonist because in the presence of the α7 antagonist MLA the increased insulin sensitivity is abrogated.

*Glycosylation of Hemoglobin.* Total glycemic load reflects both fasting and post-prandial glucose levels in the blood. A time averaged index of glycemic load is accumulation of advanced glycation end products (AGEs), which can be estimated from the glycosylation of hemoglobin, HbA1 c. Lean treated and non-treated PTP1B+ and PTP1B- mice all showed HbA1C levels lower than 5%, consistent with normal glycemic control. In contrast, obese mice showed markedly elevated HbA1 c levels, consistent with the observed glucose intolerance fasting hyperglycemia, which were significantly lowered (p< 0.01) by the α7 agonist. In the PTP1B knockout mice, HbA1c levels were markedly reduced and further reduced by treatment. α7 nAChR plays a central role in regulating both the fasting and post-prandial glucose levels in the blood and that this effect is not dependent on PTP1 B. In the presence of the α7 antagonist, MLA, the increased insulin sensitivity induced by the α7 agonist is suppressed.

*Lipid Metabolism.* Treated and non-treated lean mice show normal levels of triglycerides. However, obese mice display elevated fasting triglyceride levels, consistent with the loss of insulin sensitivity in fat cells. Nevertheless, when the obese mice were treated they displayed largely normal levels of triglycerides, an effect which was blocked by the α7 antagonist MLA, suggesting a normalization of adipocyte insulin resistance via an α7 nAChR-mediated pathway.

*Plasma TNF-α Levels.* Plasma concentration of inflammatory mediators such as TNF-α is increased in the insulin resistant states of obesity and type 2 diabetes. Reduction of the levels of TNFα in diabetic mice correlates with increased insulin sensitivity and decreased plasma insulin and blood glucose levels. Treated and non-treated lean mice showed no change in the plasma levels of TNF-α, but obese mice had elevated fasting plasma TNF-α levels. However, when the obese mice were treated, they displayed significantly decreased plasma TNF-α levels and this was blocked by the α7 antagonist MLA, confirming that α7 nAChRs are directly involved in blocking the obesity-induced increase of TNF-α and hence the decreased insulin resistance.

*α7 agonist on Obesity. Animal Models:* Parental strains of mice used in these studies were the leptin receptor deficient db/db mice on a C57BL6 background obtained from Jackson Laboratories and PTP1 B-null mice on a mixed C57BL6/Balb C background from Dr. Michel Tremblay at the Cancer Institute at McGill University in Montreal, Canada. Because obese *db*/*db* mice are infertile, mice were generated as dual heterozygotes, heterozygous for both the mutant leptin receptor and the deleted PTP1 B. Dual heterozygotes were interbred, producing 1:4 obese mice and 1:4 PTP-1 B null mice. In this breeding configuration 1:16 were dual KO mice. In the fourth generation, mice heterozygous for both genes were bred to PTP-1 B null mice heterozygous for the mutant *db* allele. In this breeding configuration 1:4 mice were obese and 1:8 were dual KO mice. For reasons of parsimony, heterozygotes were preferred to wild-types over controls. Dual heterozygous littermates were used as lean controls and littermates heterozygous for db were used as lean PTP1 B KO controls.

*Mouse genotyping*: At 3 weeks of age, DNA was obtained by tail clip. The genomic DNA from tail clip was used to screen for the presence of the mutant leptin receptor and deletion cassette of PTP-1 B using the Polymerase Chain Reaction. Specific genotypes were determining by resolving PCR products with agarose gel electrophoresis. Deletion of PTP-1 B was verified by Western analysis using an anti-PTP-1B antibody from Upstate Biotechnology.

*Metabolic Phenotyping*: The effects of the tested compound (for example, Compound A at 1 mg/kg/day via oral gavage) on growth rates and food intake of mice were generated by measuring body weight and food intake bi-weekly for from ages 3 to 10 weeks. In selected cohorts, the α7 antagonist MLA was also given via gavage, concurrently, at 3mg/kg daily. The JAK2 kinase inhibitor (AG-490) was administered intraperitoneally (lP) at 1 mg/kg daily. Fasting glucose was measured once a week after food withdrawal, with a Precision XL glucometer using tail vein bleeding. HbA1c levels were also measured from these samples with the A1 C kit from Metrika, Inc. To assess glucose tolerance, the mice were anesthetized with 2% isoflurane and the left carotid artery and jugular vein cannulated after an overnight fast. A 10 mg bolus of glucose was injected intravenously (iv) via the jugular vein and blood glucose measured every 5 minutes for 40 minutes in a drop of blood from the carotid line. For measurements of blood plasma analytes, a separate group of fasted mice were anesthetized by isoflurane in a rapid induction chamber and swiftly decapitated. Blood was collected in heparin and rapidly centrifuged at 4°C to remove cells and to obtain plasma, and the samples were frozen for later analyses. Plasma TNF-α concentrations were determined using ELISA assay kits from eBioscience and plasma triglyceride levels were determined using the L-Type TG H test (Wako Diagnostics), an *in vitro* assay for the quantitative determination of triglycerides in serum or plasma. All data are expressed as mean and SEM. Differences among all groups were compared by One Way ANOVA.

*Statistics:* All data are expressed as mean and SEM. Differences among all four genotypes were compared by One Way ANOVA.

### Effects of an α7 selective ligand on STZ-induced diabetes.

*The effects of an α7 selective ligand on the STZ-induced diabetes and the influence of JAK2:* In order to induce diabetes in the mice five multiple doses of STZ (50 mg/kg ip) or vehicle (citrate buffer) were administered daily for ten days as suggested for such investigations. Mice were weighed and blood glucose levels were determined at baseline and every four days thereafter. The mice reached stable hyperglycemia within two weeks. Now in order to determine the influence of JAK2, conditional floxed JAK2 KO mice provided by Dr. Wagner were used. These mice were crossed with inducible non-tissue specific mER-Cre mice from Jackson Labs. These mER-Cre mutant mice have an inducible element which is a mERT2-Cre fusion cDNA, which encodes the mutated murine estrogen receptor ligand-binding domain (amino acids 281 to 599, G525R) and which is insensitive to estrogen but sensitive to tamoxifen. This inducible transgenic mouse line facilitates gene targeting and would be beneficial in investigating the role of JAK2 in the adult mouse. The time point of Cre activity can be regulated by injections with tamoxifen and using these inducible Cre transgenic mice, we will be able to generate JAK2 KO mutants in a conditional and inducible manner. Homozygous JAK2flox mice carrying the mCre/mERT (i.e., mER-Cre/JAK2flox) were generated by breeding double heterozygous mice containing JAK2flox and Cre/mERT, and have assess the efficiency of induced Cre-mediated deletion of the IoxP flanked JAK2 gene segment via Southern assay before and after intraperitoneal injections of tamoxifen. Western blot analysis demonstrates that after 7 days of intraperitoneal injections of tamoxifen at a concentration of 20mg/kg there was a total ablation of JAK2 expression in the pancreas while there is no effect on the expression of Actin. In addition, analysis of mouse growth by body weight (age 1 to 16 weeks) prior to or after tamoxifen administration showed no differences among the genotypes. Furthermore, the mER-Cre/JAK2flox mice had grossly normal appearance, activity and behavior. Two separate groups of mER-Cre/JAK2+/+ and mER-Cre-JAK2flox adult mice (age, 7 weeks), post tamoxifen treatment, were then made diabetic as described above with five multiple low doses of STZ every other day with or without the compound, for example Compound A (1 mg/kg/day via oral gavage). All the groups of mice (i.e., mER-Cre/JAK2+/+ and mER-Cre-JAK2flox) reached stable hyperglycemia within two weeks.

The effects of tested compound on STZ-induced diabetes and cytokines levels were measured via the following ways. For example, fasting glucose levels were measured at least twice a week via tail vein bleeding with a Precision XL glucometer while HbA1 c levels were measured using the A1C kit from Metrika Inc. In a second group of mice, fasted mice were anesthetized by isoflurance in a rapid induction chamber and swiftly decapitated. Trunk blood was collected in heparin and rapidly centrifuged at 4C to remove blood cells and obtain plasma. Samples were frozen for later analyses. Plasma insulin, TNFα and IL-6 concentration were determined using ELISA assay kits.

*Statistics:* All data are expressed as mean and SEM. Differences among all four genotypes were compared by One Way ANOVA.

*Animal Models and Metabolic Phenotyping:* Parental strains of mice used in these studies were the leptin receptor deficient db/db fat mice or leptin receptor wild type DB/DB lean mice on a C57BL6 background obtained from Jackson Laboratories. Animal were treated with simvastatin and tested compound, such as Compound A at 1 mg/kg/day via gavage. Growth rates of mice were generated by measuring body weight twice weekly for 10 weeks. Daily food intake was measured in mice metabolic cages obtained from Fisher. To assess glucose tolerance, mice were anesthetized with 2% isoflurane and the left carotid artery and jugular vein cannulated after an ovemite fast. Fasting blood glucose was assessed as the initial two measurements in the anesthetized mice. A second measurement was used to determine HbA1 c levels using the A1CNow kit from Metrika Inc.. A 10 mg bolus of glucose was injected into each mouse and blood glucose measure by a drop of blood from the carotid line every 5 minutes for 40 minutes. Glucose was measured with a Precision XL glucometer. At the end of the experiment mice were euthanized by isoflurane overdose. In a second group of mice, fasted mice were anesthetized by isoflurance in a rapid induction chamber and swiftly decapitated. Trunk blood was collected in heparin and rapidly centrifuged to remove blood cells and obtain plasma. Samples were then collected for analyses. Plasma cholesterol, free fatty acids and triglycerides were determined using colorimetric assays from Wako Chemical while plasma TNFα concentration was determined using ELISA assay kits from eBioscience.

These data indicate that Compounds A, B, and C ameliorate glycemic state in type II diabetes.

The compounds, Compounds A, B, and C, are demonstrated to reduce weight gain, normalize glucose levels, decrease glycated hemoglobin, reduce pro-inflammatory cytokines, reduce triglycerides, and normalize insulin resistance glucose tolerance test. These data indicate that α7 ligands, including Compounds A, B, and C, ameliorate the glycemic state in metabolic disorders such as diabetes type II and biological parameters associated with the metabolic syndrome.

Selective α7 nAChR agonists can reduce weight gain, normalize glucose levels, decrease glycated hemoglobin, reduce the pro-inflammatory cytokine TNF-α, reduce triglycerides, and normalize insulin sensitivity in transgenic models of type 2 diabetes. These effects were not prevented in obese mice lacking the phosphotyrosine phosphatase PTP1 B but were fully reversed by the α7 antagonist MLA. Furthermore, the JAK2 kinase specific inhibitor AG-490 also inhibited the α7 agonist-induced weight loss, decreased food intake and normalization of glucose levels. α7 nAChRs play a central role in regulating the biological parameters associated with type 2 diabetes and the metabolic syndrome.

Insulin resistance, diabetes, obesity and dyslipidemia are components of the metabolic syndrome, and although pro-inflammatory cytokines have been suggested to contribute to the development of these disorders, the molecular mechanism of the development of this syndrome is poorly understood. The CNS modulates the immune system through the reticuloendothelial system. This CNS modulation is mediated through the vagus nerve, utilizing the major vagal neurotransmitter acetylcholine which acts upon α7 nAChRs on macrophages. Neuroprotective effects elicited by α7-selective ligands can be traced to α7 nAChR activation and transduction of signals to PI-3-K (phosphatidylinositol 3-kinase) and AKT (protein kinase B) through the protein tyrosine kinase Janus kinase 2 (JAK2), all of which compose a key cell survival pathway. The results of coimmunoprecipitation experiments indicate that there is a direct interaction between the alpha7 nicotinic receptor and JAK2. Other studies that examined the effects of nicotine on LPS-treated and control peritoneal macrophages have shown that nicotine treatment leads to phosphorylation of STAT3, a member of the STAT (Signal transducers and activators of transcription) family of proteins and a component of the cellular anti-apoptotic cascade. This nicotine-mediated phosphorylation is inhibited by the α7-selective antagonists α-bungarotoxin and MLA, and by AG-490, a selective inhibitor of JAK2 phosphorylation. These data support the interaction of JAK2 and α7 nAChRs and reveal the critical role played by STAT3 in the cholinergic anti-inflammatory pathway. The present results extend these findings and underline the importance of α7 nAChR interactions with JAK2 in modulating the biological parameters associated with weight gain and food intake. Interestingly, regulatory feedback on adiposity, mediated by the leptin receptor in hypothalamus, also involves receptor-JAK2 interactions. In obese mice which lack an active leptin receptor (i.e., db/db mice) the α7 nAChR may substitute for the leptin receptor in the activation of JAK2, which in turn leads to decreased food intake and weight gain.

Another pathway that appears to intersect with the cholinergic ant-inflammatory pathway, and one that is directly relevant to a key component of the metabolic syndrome, involves the protein tyrosine phosphatase (PTP) PTP1 B. Specifically, PTP1 B has been shown to act as a negative regulator of insulin signaling. Overexpression of PTP1 B impairs insulin signals, whereas loss of PTP1 B is associated with increased sensitivity to insulin. PTP1 B binds to and catalyzes the dephosphorylation of the insulin receptor and many of the effects of PTP1B on insulin signaling can be explained on the basis of this interaction. Studies have shown that deletion of the phosphotyrosine phosphatase PTP1 B improves insulin signaling in mouse models of obesity and PTP1 B antagonists have been used pharmacologically to improve glucose tolerance. More importantly, PTP1 B has also been reported to dephosphorylate JAK2, suggesting that there is cross-talk between the α7 nAChR-linked anti-inflammatory pathway and insulin regulation. Since PTP1 B regulates body weight, adiposity and leptin action, α7 nAChRs may play a critical role in regulating numerous aspects of the metabolic syndrome.

The specific pharmacological responses observed may vary according to and depending on the particular active compound selected or whether there are present pharmaceutical carriers, as well as the type of formulation and mode of administration employed, and such expected variations or differences in the results are contemplated in accordance with practice of the present invention.

The following numbered paragraphs summarise certain aspects of the invention.
1. A method for treating or preventing metabolic disorders comprising the administration of a selective α7 nAChR agonist.
2. A method for treating or preventing drug-induced central nervous system disorders comprising the administration of a selective α7 nAChR agonist.
3. The method of paragraphs 1 or 2, wherein the α7 nAChR agonist is Compound A, Compound B, Compound C, or a pharmaceutically acceptable salt thereof.
4. The method of paragraphs 1 to 3, wherein the α7 nAChR agonist is Compound C or a pharmaceutically acceptable salt thereof.
5. The method of paragraphs 1, 3, or 4, wherein the metabolic disorder is one or more of type I diabetes mellitus, type II diabetes mellitus, metabolic syndrome, atherosclerosis, obesity, and hyperglycemia.
6. The method of paragraph 5, wherein the hyperglycemia is a result of statin therapy.
7. The method of paragraph 2, wherein the drug-induced central nervous system disorder is a result of statin therapy.
8. A method for treating or preventing a metabolic disorder comprising the administration of 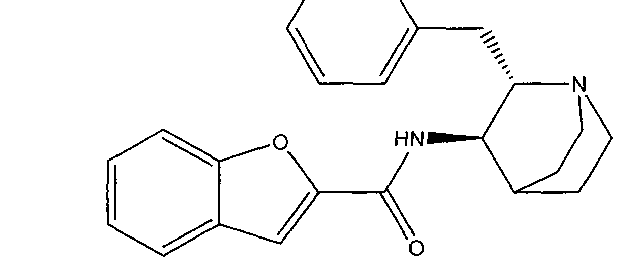 or a pharmaceutically acceptable salt thereof.
9. The method of paragraph 8 wherein the metabolic disorder is one or more of type I diabetes mellitus, type II diabetes mellitus, metabolic syndrome, atherosclerosis, obesity, and hyperglycemia.
10. The method of paragraph 8 or 9, wherein a daily dose is from about 0.001 mg/kg to about 3.0 mg/kg.
11. Use of a selective α7 nAChR agonist in the manufacture of a medicament for treating or preventing metabolic disorders.
12. Use of a selective α7 nAChR agonist in the manufacture of a medicament for treating or preventing drug-induced central nervous system disorders.
13. The use of paragraph 11 or 12, wherein the α7 nAChR agonist is Compound A, Compound B, Compound C, or a pharmaceutically acceptable salt thereof.
14. The use of paragraphs 11 to 13, wherein the α7 nAChR agonist is Compound C or a pharmaceutically acceptable salt thereof.
15. The use of paragraphs 11, 13, or 14, wherein the metabolic disorder is one or more of type I diabetes mellitus, type II diabetes mellitus, metabolic syndrome, atherosclerosis, obesity, and hyperglycemia.
16. The use of paragraph 15, wherein the hyperglycemia is a result of statin therapy.
17. The use of paragraph 12, wherein the drug-induced central nervous system disorder is a result of statin therapy.
18. Use of Compound C: 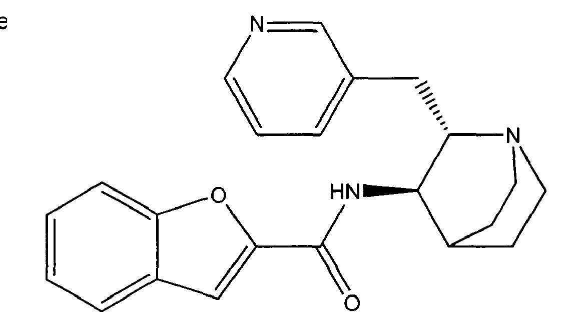 or a pharmaceutically acceptable salt thereof in the manufacture of a medicament for treating or preventing a metabolic disorder.
19. The use of paragraph 18 wherein the metabolic disorder is one or more of type I diabetes mellitus, type II diabetes mellitus, metabolic syndrome, atherosclerosis, obesity, and hyperglycemia.
20. The use of paragraphs 18 or 19, wherein a daily dose is from about 0.001 mg/kg to about 3.0 mg/kg.
21. A selective α7 nAChR agonist compound for use in treating or preventing metabolic disorders.
22. A selective α7 nAChR agonist compound for use in treating or preventing drug-induced central nervous system disorders.
23. The compound of paragraphs 21 or 22, wherein the α7 nAChR agonist is Compound A, Compound B, Compound C, or a pharmaceutically acceptable salt thereof.
24. The compound of paragraphs 21 to 23, wherein the α7 nAChR agonist is Compound C or a pharmaceutically acceptable salt thereof.
25. The compound of paragraphs 21, 23, or 24, wherein the metabolic disorder is one or more of type I diabetes mellitus, type II diabetes mellitus, metabolic syndrome, atherosclerosis, obesity, and hyperglycemia.
26. The compound of paragraph 25, wherein the hyperglycemia is a result of statin therapy.
27. The compound of paragraph 22, wherein the drug-induced central nervous system disorder is a result of statin therapy.
28. A selective α7 nAChR agonist compound 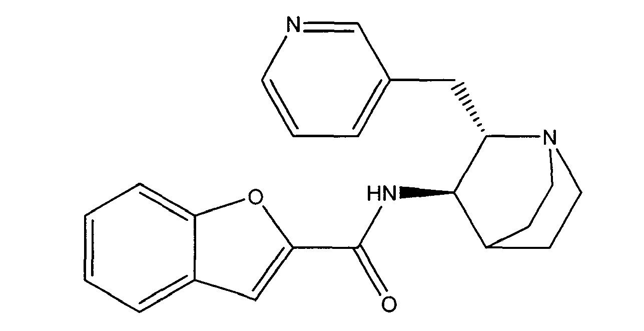 or a pharmaceutically acceptable salt thereof for use in treating or preventing a metabolic disorder.
29. The compound of paragraph 28 wherein the metabolic disorder is one or more of type I diabetes mellitus, type II diabetes mellitus, metabolic syndrome, atherosclerosis, obesity, and hyperglycemia.
30. The compound of paragraphs 28 or 29, wherein a daily dose is from about 0.001 mg/kg to about 3.0 mg/kg.

Although specific embodiments of the present invention are herein illustrated and described in detail, the invention is not limited thereto. The above detailed descriptions are provided as exemplary of the present invention and should be construed as constituting any limitation of the invention. Modifications will be obvious to those skilled in the art, and all modifications that do not depart from the spirit of the invention are intended to be included with the scope of the appended claims.

## Claims

1. Use of a selective α7 nAChR agonist in the manufacture of a medicament for treating or preventing metabolic syndrome.

2. A selective α7 nAChR agonist compound for use in treating or preventing metabolic syndrome.

3. Use of a selective α7 nAChR agonist in the manufacture of a medicament for treating or preventing drug-induced metabolic syndrome.

4. A selective α7 nAChR agonist compound for use in treating or preventing drug-induced metabolic syndrome.

5. The use of claim 3 or the compound of claim 4, wherein the drug induced metabolic syndrome is a result of statin therapy.

6. The use of claim 1 or 3 or 5 or a compound of claim 2 or 4 or 5, wherein the α7 nAChR agonist is a compound of formula I, 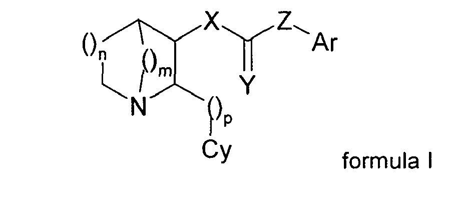 wherein m and n are individually 1 or 2, and p is 1, 2, 3 or 4, X is oxygen or -NR', Y is oxygen or sulfur, and Z is -NR', a covalent bond, or a linker species A; wherein A is selected from -CR'R"-, -CR'R"-CR'R"-, -CR'=CR'-, and -C≡C-, wherein when Z is a covalent bond or A, X must be -NR',
Ar is a substituted or unsubsituted aryl, carbocyclic, heterocyclic, monocyclic or fused polycyclic, and Cy is a substituted or unsubstituted 5 or 6-membered heteroaromatic ring;
wherein individually, Ar and Cy are unsubstituted or substituted with 1, 2 or 3 substituents, selected from alkyl, alkenyl, heterocyclyl, cycloalkyl, aryl, substituted aryl, arylalkyl, substituted arylalkyl, halo, OR', NR'R", -CF₃, -CN, -NO₂, -C₂R', -SR', -N₃, -C(=O)NR'R", -NR'C(=O)R", -C(=O)R', -C(=O)OR', -OC(=O)R', -O(CR'R")ᵣC(=O)R', -O(CR'R")ᵣNR"C(=O)R', -O(CR'R")ᵣNR"SO₂R', -OC(=O)NR'R", NR'C(=O)OR", -SO₂R', SO₂NR'R" and -NR'SO₂R",
wherein R' and R" are individually hydrogen, C₁-C₈ alkyl, cycloalkyl, heterocycyl, aryl, or arylalkyl; and r is an integer from 1 to 6, or R' and R" together form a cyclic functionality, or pharmaceutically acceptable salts thereof.

7. The use of claim 6 or the compound of claim 6, wherein a daily dose is from about 0.001 mg/kg to about 3.0 mg/kg.
